(19) Europäisches Patentamt
European Patent Office
Office européen des brevets

(11) EP 4 537 813 A1

(12) EUROPEAN PATENT APPLICATION
published in accordance with Art. 153(4) EPC

(43) Date of publication:
**16.04.2025 Bulletin 2025/16**

(21) Application number: **23820135.4**

(22) Date of filing: **09.06.2023**

(51) International Patent Classification (IPC):
***A61K 9/06*** *(2006.01)* ***A61K 47/34*** *(2017.01)*
***A61K 9/00*** *(2006.01)* ***A61P 29/00*** *(2006.01)*
***A61P 19/02*** *(2006.01)*

(52) Cooperative Patent Classification (CPC):
**A61K 9/00; A61K 9/06; A61K 47/34; A61P 19/02; A61P 29/00**

(86) International application number:
**PCT/KR2023/007948**

(87) International publication number:
**WO 2023/239200 (14.12.2023 Gazette 2023/50)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.06.2022 KR 20220070182**

(71) Applicant: **Korea Institute of Science and Technology**
**Seoul 02792 (KR)**

(72) Inventors:
- **SONG, Soo Chang**
**Seoul 02792 (KR)**
- **KIM, Jun**
**Seoul 02792 (KR)**

(74) Representative: **advotec.**
**Patent- und Rechtsanwaltspartnerschaft**
**Tappe mbB**
**Widenmayerstraße 4**
**80538 München (DE)**

# (54) COMPOSITION FOR PREVENTING OR TREATING INFLAMMATORY DISEASES, CONTAINING POLY(ORGANOPHOSPHAZENE) POLYMER

(57) The present invention relates to: a thermo-sensitive poly(organophosphazene) polymer loaded with a drug; a pharmaceutical composition for preventing or treating inflammatory diseases, comprising same; and the like. An injectable polymer nanoparticle hydrogel system comprising the hydrogel has a long-term anti-inflammatory effect and can slowly release a drug at a therapeutically-effective concentration, and thus can be used in the prevention and treatment of various inflammatory diseases such as osteoarthritis.

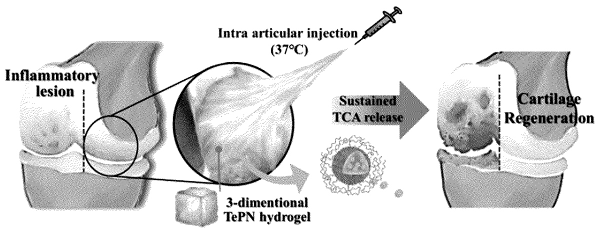


FIG. 1



Processed by Luminess, 75001 PARIS (FR)

## Description

### TECHNICAL FIELD

The present disclosure relates to a composition for preventing or treating inflammatory diseases containing a poly(organophosphazene) polymer, and the like.

### BACKGROUND ART

Osteoarthritis (OA) is the most common type of degenerative joint disease. Degeneration and inflammation of the joints lead to chronic pain, stiffness, and progressive loss of articular cartilage that may not be regenerated later. Degenerative joints may be replaced with artificial prostheses in the late stage of osteoarthritis, but the artificial prostheses have limitations and may require additional surgery for artificial joint replacement. Intra articular (IA) administration of autologous chondrocytes, other therapeutic strategies such as platelet-rich plasma, or supplementation of viscous substances such as hyaluronic acid (HA) to eroded cartilage may be other solutions for regenerating damaged articular cartilage. However, the resulting anti-OA effects of these methods are not satisfactory due to poor target-specific action and retention time in the body after IA administration. Drug therapy may include oral administration of analgesics, nonsteroidal anti-inflammatory drugs (NSAIDs), specific cyclooxygenase (COX)-2 inhibitors, and opioids, but these treatments are limited in symptomatic pain treatment and have little effect on local treatment of inflammation [Buchman A.L. Side effects of corticosteroid therapy. J. Clin. Gastroenterol. 2001;33(4):289-294.]. In addition, NSAIDs, COX-2 inhibitors, and opioids are known to cause serious adverse effects on the gastrointestinal (GI) system, heart, and brain. Therefore, these oral drugs should be prescribed cautiously to elderly patients with underlying gastrointestinal, cardiac, or brain diseases. Direct IA administration of corticosteroids such as methotrexate, diclofenac, and triamcinolone acetonide (TCA) is possible to increase the bioavailability of the administered drug at a target site and reduce possible systemic side effects. However, the half-lives of these drugs are known to be 0.56 to 2.9, 5.2, and 1.47 hours, respectively, and reported to disappear rapidly from the body [Larsen C., Ostergaard J., Larsen S.W., Jensen H., Jacobsen S., Lindegaard C., Andersen P.H. Intra-articular depot formulation principles: role in the management of postoperative pain and arthritic disorders. J. Pharmacol. Sci. 2008;97(11):4622-4654. etc.]. These short retention times are not sufficient to reduce the sustained inflammatory response.

Therefore, for these reasons, research has been conducted on new methods for the ultimate treatment of osteoarthritis and the prevention in the early stages. Potential new OA treatment methods using anti-OA drug delivery systems with long-term in vivo activity have been applied to liposomes, nanoparticles, microparticles, hydrogels, and the like, and the duration of anti-OA drug release is significantly extended from several weeks to several months [Pradal J., Zuluaga M.F., Maudens P., Waldburger J.M., Seemayer C.A., Doelker E., Gabay C., Jordan O., Allemann E. Intra-articular bioactivity of a p38 MAPK inhibitor and development of an extended-release system. EUR. J. Pharm. Biopharm. 2015;93:110-117. etc.]. However, these systems have been shown to be unsuitable for precise drug delivery to the target site and have low release efficiency [Kopecek J. Hydrogel biomaterials: a smart future? Biomaterials. 2007;28(34):5185-5192.].

Meanwhile, a hydrogel-based drug delivery system may provide an advantage of local drug storage, which may extend a drug release period and provide a physically supportive 3D space, such as a synovial area. These properties may further reduce damage to synovial joints by reducing the physical friction of inflammatory arthritis. In addition, thermosensitive sol-gel transition properties may provide noninvasive administration advantages. However, a hydrogel-based anti-osteoarthritis drug delivery system has been reported to have a limitation in exhibiting drug release characteristics for only several days [Park C.W., Ma K.W., Jang S.W., Son M., Kang M.J. Comparison of piroxicam pharmacokinetics and anti-inflammatory effect in rats after intra-articular and intramuscular administration. Biomol. Ther. (Seoul) 2014;22(3):260-266.].

A hybrid system of a nano-drug delivery system and a 3D hydrogel drug delivery system may be a powerful tool for successful OA treatment. To this end, poly(organophosphazene) substituted with hydrophobic and hydrophilic side chains may function as a biodegradable drug delivery system having injectable in situ hydrogel formation and drug loading processes and long-term drug release properties [Hong K.H., Kim Y.M., Song S.C. Fine-tunable and injectable 3D hydrogel for on-demand stem cell niche. Adv. Sci. 2019;6(17):1900597. doi: 10.1002/advs.201900597.]. In an aqueous solution, the polymer exists in the form of extended chains or nano-sized spherical particles, depending on a change in hydrophobicity according to a temperature change. Based on these properties, various drugs with hydrophobic moieties may be encapsulated into polymeric nanoparticles (PNs) by participating in the self-assembly process of the polymeric nanoparticles (PNs). In addition, the polymeric nanoparticles may form a 3D hydrogel network at a specific concentration or higher.

The present inventors conducted research to enable local and sustained TCA delivery using the poly(organophosphazene)-based PN hydrogel system and then completed the present disclosure.

## DISCLOSURE OF THE INVENTION

## TECHNICAL GOALS

An aspect to be achieved by the present disclosure is to provide a composition for preventing or treating inflammatory diseases including a poly(organophosphazene) polymer.

However, technical goals to be achieved are not limited to those described above, and other goals not mentioned above are clearly understood by one of ordinary skill in the art from the following description.

## TECHNICAL SOLUTIONS

In order to solve the aspect, embodiments provide a composition for preventing or treating inflammatory diseases including: a polyphosphazene polymer including, on the phosphorus atom of a polyphosphazene backbone represented by the following Chemical Formula 1,

a first moiety of amino acid ester represented by the following Chemical Formula 2;
a second moiety of polyethylene glycol represented by the following Chemical Formula 3; and
a third moiety including aminoethanol and an organic acid, each in a molar ratio of a : b : c; and
a therapeutically effective amount of a drug for controlling and treating inflammation.

[Chemical Formula 1]

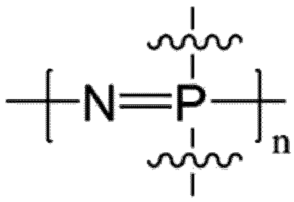

[Chemical Formula 2]

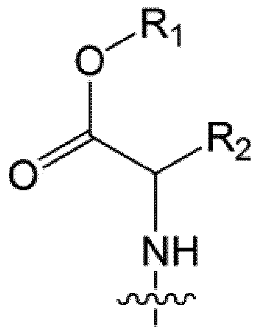

[Chemical Formula 3]

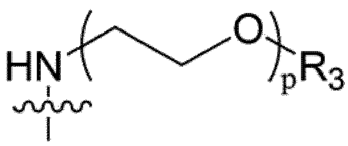

in which,

$R_1$ may be $C_{1-6}$ alkyl, $C_{1-6}$ alkenyl, or $C_{6-10}$ aryl-$C_{1-6}$ alkyl;
$R_2$ may be hydrogen, methyl, isopropyl, 1-methylpropyl, 2-methylpropyl, thiomethyl, methylthioethyl, benzyl, hydroxybenzyl or 2-indolylmethyl;
$R_3$ may be $C_{1-6}$ alkyl;
n may be an integer of 3 to 100,000,

p may be an integer of 1 to 20,
a molar ratio of a : b may be 5.5 : 1 to 7.5 : 1, and
a may be 70 to 80 mol%.

According to one aspect, the organic acid in the third moiety may include glutaric acid.

According to one aspect, the $R_1$ may be methyl, ethyl, propyl, butyl, benzyl or 2-propenyl.

According to one aspect, the $R_3$ may be methyl.

According to one aspect, the drug for treating the inflammatory diseases may be hydrophobic.

According to one aspect, the hydrophobic drug for treating the inflammatory diseases may be at least one selected from the group consisting of triamcinolone acetonide, methylprednisolone, dexamethasone, celecoxib, ibuprofen, naproxen, indomethacin, ketoprofen, etodolac, meloxicam, rofecoxib, etoricoxib, valdecoxib, lumiracoxib, and diclofenac.

According to one aspect, the polymer may slowly release the drug for treating the inflammatory diseases.

According to one aspect, the polyphosphazene polymer may be dissolved and included in a solvent at a concentration of 1 to 50% by weight (wt%). The solvent may be at least one selected from the group consisting of water, a buffer solution, an acid solution, a basic solution, a salt solution, a saline solution, water for injection, a cell culture solution, and a glucose saline solution, but is not limited thereto.

According to one aspect, the polymer may exhibit sol-gel transition behavior in the range of 5 to 70°C and form a hydrogel at a predetermined temperature.

According to one aspect, the polymer may gel when applied to a living body or in an in vitro environment, but may lose thermo-sensitivity at a predetermined temperature and maintain a gel state regardless of a change in temperature.

According to one aspect, the inflammatory diseases may be any one selected from the group consisting of osteoarthritis, degenerative arthritis, rheumatoid arthritis, osteoporosis, and Achilles tendonitis.

## EFFECTS OF THE INVENTION

The present disclosure relates to a thermo-sensitive poly(organophosphazene) polymer loaded with a drug, a hydrogel including the same, a composition for preventing or treating inflammatory diseases, including the same and the like. An injectable polymeric nanoparticle hydrogel system including the hydrogel has an anti-inflammatory effect that is maintained in the body for a long period of time, and has an effect of regenerating tendons including Achilles tendon, ligaments, muscles, cells, and the like, and therefore can be utilized for preventing and treating various inflammatory diseases, such as osteoarthritis.

In addition, the present inventors optimize a ratio of hydrophobic side chains and hydrophilic side chains forming the poly(organophosphazene) polymer so as to exhibit ideal drug release characteristics in the body, and to administer drugs into the body at a constant concentration for a long period of time.

The effects of the present disclosure are not limited to the aforementioned effects, and other effects, which are not mentioned above, can be clearly appreciated by one of ordinary skill in the art from the following description.

## BRIEF DESCRIPTION OF THE DRAWINGS

FIG. 1 shows a functional schematic diagram of TCA-loaded poly(organophosphazene) nanoparticles.

FIG. 2 shows 1H NMR data of poly(organophosphazene).

FIG. 3 illustrates results of a biocompatibility experiment of poly(organophosphazene).

FIG. 4 shows cytotoxicity data of carboxyl-terminated poly(organophosphazene).

FIG. 5 shows the characterization of poly(organophosphazene) nanoparticles. FIG. 5A illustrates a structure and self-assembly behavior of a poly(organophosphazene) nanoparticle. FIGS. 5B, 5C, and 5D show TEM images of a self-assembled PN, TCA aggregates, and TePN, respectively, and FIG. 5E illustrates hydrodynamic diameters of the self-assembled PN, TCA aggregate, and TePN in a PBS solution (n = 6).

FIG. 6 is a TEM image of micro-sized TCA aggregates.

FIG. 7 shows hydrodynamic diameters of self-assembled PN, TCA, and TePN in a PBS solution.

FIG. 8 shows the characterization of a TePN hydrogel system. FIG. 8A shows changes in temperature-dependent sol-gel transition and viscosity, FIG. 8B shows temperature-dependent changes in storage and loss modulus of a PN solution, and FIG. 8C shows changes in temperature and TCA content for the storage and loss modulus of a TePN solution.

FIG. 9 shows biodegradability of a PN hydrogel. FIG. 9A is a photograph of a mouse experiment, and FIG. 9B is a time-dependent hydrogel degradation graph.

FIG. 10 shows hydrogel photographs of injection sites by date.
FIG. 11 shows predicted hydrogel volumes from in vitro mass loss experiments.
FIG. 12 shows sustained release profiles of TCA from TePN. FIG. 12A shows time-dependent hydrogel forms of TePN, and FIG. 12B shows cumulative TCA release amounts.
FIG. 13 shows endochondral photographs 8 weeks after treatment.
FIG. 14 shows in vivo anti-inflammatory efficacy characteristics of TePN after IA injection as shown in X-ray and CT images (FIG. 14A) and interarticular cartilage distances (FIG. 14B).
FIG. 15 shows in vivo anti-inflammatory efficacy of a TePN hydrogel system by histochemical staining.
FIG. 16 shows real-time PCR analysis of anti-inflammatory cytokine expression.
FIG. 17 shows changes in body weight of rats in each group.
FIG. 18 shows NMR data of prepared PPZ.
FIG. 19 shows changes in physical properties according to a change in IleOEt/AMPEG molar ratio.
FIG. 20 shows expression levels of inflammation-related factors according to a TCA concentration.
FIG. 21 shows a process of confirming an optimal IleOEt/AMPEG molar ratio.
FIG. 22 is a schematic diagram of the process of administering a CXB-PPZ complex to AT.
FIG. 23 shows $^1$H NMR and GPC results of a synthesized PNP structure.
FIG. 24 shows FT-IR results of PCNP, CXB, and PNP.
FIG. 25 shows CXB encapsulation and thermo-sensitive gelation.
FIG. 26 shows the gelation of a PNP hydrogel.
FIG. 27 shows rheological properties of PNP and PCNP hydrogels.
FIG. 28 shows characterization of PNP and PCNP.
FIG. 29 shows that CXB release is sustained from a PCNP hydrogel, and a hydrogel network is maintained for about a month.
FIG. 30 shows in vitro degradation of a PNP hydrogel.
FIG. 31 shows a stable modulus of a PNP hydrogel at 37°C.
FIG. 32 shows in vivo biodegradation of a PNP hydrogel.
FIG. 33 shows results of a cytotoxicity experiment using MTT assay of a PNP hydrogel.
FIG. 34 shows expression levels of inflammation-related factors as a result of real-time PCR analysis of blood samples.
FIG. 35 shows regeneration of damaged Achilles tendon as an anti-inflammatory effect by long-term release of a PCNP hydrogel system.
FIG. 36 shows hydroxyproline contents, stiffness, and tensile strength in a control group, a non-treated group, a CXB solution treated group, and a PCNP treated group.

## BEST MODE FOR CARRYING OUT THE INVENTION

In order to solve the problems, the present inventors provide the following poly(organophosphazene) polymer and a composition for preventing or treating inflammatory diseases including the same.

The composition for preventing or treating inflammatory diseases includes a polyphosphazene polymer including, on a phosphorus atom of a polyphosphazene backbone represented by the following Chemical Formula 1,

a first moiety of amino acid ester represented by the following Chemical Formula 2;
a second moiety of polyethylene glycol represented by the following Chemical Formula 3; and
a third moiety including aminoethanol and an organic acid, each in a molar ratio of a : b : c; and
a therapeutically effective amount of a drug for controlling and treating inflammation.

[Chemical Formula 1]

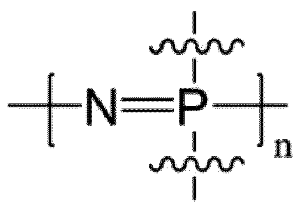

[Chemical Formula 2]

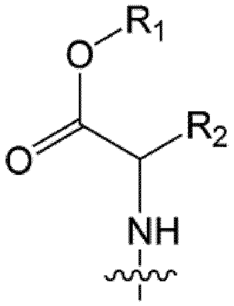

[Chemical Formula 3]

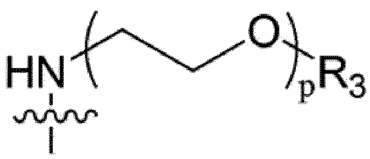

in which,

$R_1$ is $C_{1-6}$ alkyl, $C_{1-6}$ alkenyl, or $C_{6-10}$ aryl-$C_{1-6}$ alkyl;
$R_2$ is hydrogen, methyl, isopropyl, 1-methylpropyl, 2-methylpropyl, thiomethyl, methylthioethyl, benzyl, hydroxybenzyl or 2-indolylmethyl;
$R_3$ is $C_{1-6}$ alkyl;
n is an integer of 3 to 100,000,
p is an integer of 1 to 20,
a molar ratio of a : b is 5.5 : 1 to 7.5 : 1, and
a may be 70 to 80 mol%.

In general, a thermo-sensitive polymer is a polymer that exhibits abrupt changes in solubility depending on a temperature change. As the temperature increases, hydrogen bonds between the polymer and a solvent are weaken to cause dehydration and the hydrophobic force between the polymers is strengthened to have a more hydrophobic structure. At a low critical solution temperature (LCST), polymer-polymer and water-water interactions are desirable to hydrogen bonds between polymers and water, so that rapid dehydration occurs from the polymer to have a more hydrophobic structure. The LCST of the thermo-sensitive polymer changes depending on the balance of hydrophobic and hydrophilic groups bonded to the polymer backbone. In general, as the content of hydrophilic groups increases, the phase transition temperature increases, and conversely, as the content of hydrophobic groups increases, the phase transition temperature decreases. These characteristics may also be confirmed in a $T_{max}$ change according to a change in a ratio of hydrophobic side chains and hydrophilic side chains disclosed in Example 9 below.

When the essential constituent moieties constituting the poly(organophosphazene) polymer have a predetermined ratio, the sol-gel transition of the polymer solution occurs reversibly by a temperature change below a predetermined temperature, but after exposure to a specific temperature or higher, the rapid reversible transition characteristic is lost and a gel form may be maintained for a long period of time. In particular, the characteristic may be changed by a ratio of substituents introduced during the initial polymer synthesis.

In particular, in the present disclosure, it was confirmed that when the first moiety and the second moiety are within a specific molar ratio range, the drug may be slowly released while maintaining an optimal drug concentration required in a living body (see Example 9 below). This is based on the discovery of the present inventors that the hydrophobic property and phase transition temperature of the polymer change depending on a composition ratio of the first moiety having hydrophobicity and the second moiety having hydrophilicity. As the molar ratio of hydrophobic side chains increases, the polymer has the hydrophobic property, and the interactions with hydrophobic drugs become stronger, so that the drug may be less released into the body. Therefore, a rate at which the drug is released into the body may be controlled by adjusting the molar ratio of the hydrophobic side chains and the hydrophilic side chains.

Both drugs for treating steroid-based inflammatory disease including TCA and non-steroidal drugs including celecoxib have concentration ranges that are most suitable for acting in the body, and in the case of TCA, as shown in FIG. 20, it may be confirmed that when a predetermined concentration range is exceeded, the expression of inflammatory factors actually increases.

As shown in Example 9 and FIG. 21 below by the present inventors, the molar ratio of the hydrophobic side chains

to the hydrophilic side chains may be in the range of desirably 5.5 : 1 to 7.5 : 1, more desirably 6 : 1 to 7 : 1, and most desirably 6.13 : 1. Within the molar ratio range, the drug, desirably TCA, may be slowly released within the suitable concentration range. When the molar ratio range is exceeded, a pharmacological effect of TCA may be reduced.

According to one aspect, the organic acid in the third moiety may include glutaric acid. The glutaric acid refers to (Acid) disclosed in Example 10-2 below. The glutaric acid is prepared and produced according to a preparation method of Example 10-2, and may be glutaric acid linked to a hydroxyl functional group by an ester bond in a final product.

The composition may further include a fourth moiety in which a partial or entire functional group of the third moiety is bonded directly or via a linker with at least one functional moiety selected from the group consisting of a substance capable of controlling the degradation rate of the polymer, a substituent including an ionic group capable of controlling the degradation rate, a cross-linkable substituent, an additional compound capable of inducing tissue adhesion, a physiologically active substance, and a composite substance formed by linearly linking two or more of these functional substances.

At this time, the fourth moiety may include at least one selected from the group consisting of folic acid, hyaluronic acid, cyclodextrin, imidazole-based compounds, anticancer agents, histidine, lysine, arginine, cysteine, thiol arylamine, spermine, spermidine, polyethyleneimine, polyhistidine, polylysine, polyarginine, protamine, heparin, chitosan, and peptides consisting of 1 to 20 amino acids.

According to one aspect, the $R_1$ may be methyl, ethyl, propyl, butyl, benzyl or 2-propenyl.

According to one aspect, the $R_3$ may be methyl.

According to one aspect, the drug for treating the inflammatory diseases may be hydrophobic, and may further include a functional substance.

The functional substance may be preosteoblast, chondrocyte, osteoblast, adult stem cell, schwann cell, oligodendrocyte, insulin, oxytocin, vasopressin, adrenocorticotropic hormone, fibroblast growth factor, epidermal growth factor, platelet-derived growth factor, insulin-like growth factor, vascular endothelial growth factor, transforming growth factor, neuroblastoma growth factor, antibiotics, and angiogenic inhibitor such as integrin alpha-5-beta-1 antagonist, or combinations thereof.

The hydrophobic drug for treating osteoarthritis may be at least one selected from the group consisting of triamcinolone, methylprednisolone, dexamethasone, ibuprofen, naproxen, and diclofenac, and most desirably triamcinolone.

According to one aspect, the polymer may slowly release the drug for treating osteoarthritis. As used herein, "slowly releasing" means releasing a drug while maintained in the body for at least 21 days, and desirably, maintaining a release concentration of the drug at a therapeutically-effective concentration of the drug. **In** addition, the slowly releasing may be releasing the drug at a drug concentration in a therapeutically effective range, and the drug concentration in the therapeutically effective range may be desirably 9.375 to 75 ug/ml, and more desirably 10 to 50 ug/ml.

According to one aspect, the polyphosphazene polymer may be dissolved and included in a solvent at a concentration of 1 to 50% by weight (wt%). The solvent may be at least one selected from the group consisting of water, a buffer solution, an acid solution, a basic solution, a salt solution, a saline solution, water for injection, a cell culture solution, and a glucose saline solution, but is not limited thereto. **In** the presence of the solvent, the polyphosphazene polymer of the present disclosure may be hydrogelized and may exhibit sol-gel transition behavior as described below.

According to one aspect, the polymer may exhibit sol-gel transition behavior in the range of 5 to 70°C and form a hydrogel at a predetermined temperature.

According to one aspect, the polymer may gel when applied to a living body or in an in vitro environment, but may lose thermo-sensitivity at a predetermined temperature and maintain a gel state regardless of a change in temperature.

The inflammatory disease may desirably be any one selected from the group consisting of osteoarthritis, degenerative arthritis, rheumatoid arthritis, osteoporosis, and Achilles tendonitis, and most desirably osteoarthritis.

The composition of the present disclosure has the use of "prevention" and/or "treatment" of inflammatory diseases, desirably osteoarthritis. For preventive use, the composition of the present disclosure may be administered to a subject who has diseases or symptoms described herein or is suspected of having a risk for developing the diseases. For therapeutic use, the pharmaceutical composition of the present disclosure is administered to a subject such as patients already suffering from the diseases described herein in an amount enough to treat or at least partially stop the diseases or symptoms described herein. The effective amount for the use will vary according to the severity and course of the diseases or symptoms, previous treatment, the health condition of a subject and responsiveness to a drug, and the judgment of physicians or veterinarians.

The composition may have any one formulation selected from the group consisting of sterile aqueous solutions, non-aqueous solvents, suspensions, tablets, pills, powders, granules, capsules, solutions, emulsions, syrups, emulsions, lyophilized agents and suppositories, and may be various parenteral or oral formulations. When the pharmaceutical composition is formulated, the formulation may be prepared by using diluents or excipients, such as a filler, an extender, a binder, a wetting agent, a disintegrating agent, and a surfactant, which are generally used. Solid formulations for oral administration include tablets, pills, powders, granules, capsules, etc., and these solid formulations may be prepared by

mixing at least one excipient, for example, starch, calcium carbonate, sucrose or lactose, gelatin, and the like with at least one compound. Further, lubricants such as magnesium stearate, talc, and the like may also be used in addition to simple excipients. Liquid formulations for oral administration may correspond to a include suspensions, solutions, emulsions, syrups, and the like, and may include various excipients, such as wetting agents, sweeteners, flavoring agents, preservatives, and the like, in addition to water and liquid paraffin which are commonly used as simple diluents. Formulations for parenteral administration include sterile aqueous solutions, non-aqueous solutions, suspensions, emulsions, lyophilized preparations, and suppositories. As the non-aqueous solution and the suspension, propylene glycol, polyethylene glycol, vegetable oil such as olive oil, injectable ester such as ethyl oleate, and the like may be used. As a base of the suppository, witepsol, macrogol, tween 61, cacao butter, laurinum, glycerogelatin, and the like may be used.

In the present disclosure, the composition of the present disclosure may be formulated as a sterile aqueous solution, a non-aqueous solvent, or a suspension for parenteral administration, and specifically, may be formulated as an injection. The pharmaceutical composition of the present disclosure may additionally contain suitable carriers, excipients or diluents commonly used. At this time, the content of the injection composition of the present disclosure, which is an active ingredient included in the pharmaceutical composition, is not particularly limited.

The composition of the present disclosure may be administered to a subject in a pharmaceutically effective amount.

As used herein, the term "pharmaceutically effective amount" refers to an amount enough to treat diseases at a reasonable benefit/risk ratio applicable to medical treatment, and an effective dose level may be determined according to factors including a kind of subject, the severity, age, sex, a type of disease, the activity of a drug, sensitivity to a drug, a time of administration, a route of administration, an excretion rate, duration of treatment, and agents to be simultaneously used, and other factors well-known in the medical field.

The composition of the present disclosure may be administered as an individual therapeutic agent or in combination with other therapeutic agents, and may be administered sequentially or simultaneously with conventional therapeutic agents, and administered singly or multiply. It is important to administer an amount capable of obtaining a maximum effect with a minimal amount without side effects in consideration with all the factors, and the amount thereof may be easily determined by those skilled in the art. A preferred dose of the composition of the present disclosure varies depending on the condition and weight of a patient, the severity of a disease, the form of a drug, the route and duration of administration, and the dose may be administered once a day or administered several times.

In the present disclosure, the composition of the present disclosure may be administered in a mixture with the functional substance including stem cells, which is as described above.

The composition of the present disclosure is able to be administered to any individual for the prevention or treatment of osteoarthritis without particular limitation. The administration method includes any conventional method in the art without limitation. The composition may be administered intra-articularly (e.g., intrachondral), subcutaneously, intraperitoneally, intrapulmonary and intranasally, and may be administered by any suitable method, including administration of the lesion, that is, intra articular (e.g., intrachondral) administration, if necessary, for local treatment. For example, the composition may be administered (injected) by intra articular injection, but is not limited thereto.

As used herein, the term "subject" means all animals that have developed or are likely to develop osteoarthritis, and the subject may be effectively treated by administering the composition of the present disclosure to a subject suspected of having osteoarthritis. The subject is not particularly limited and may include animals such as monkeys, dogs, cats, rabbits, guinea pigs, rats, mice, cows, sheep, pigs, and goats, birds, etc., but is not limited thereto.

As used herein, the term "administration" means introducing the composition of the present disclosure to a subject suspected of osteoarthritis by any suitable method, and the composition of the present disclosure may be administered through various parenteral routes as long as the composition may reach a target tissue. The composition of the present disclosure may be administered in a pharmaceutically effective amount, and the pharmaceutically effective amount is as described above.

The terms used in the examples are used for the purpose of description only, and should not be construed to be limited. A singular expression includes a plural expression unless otherwise defined differently in a context. In the present disclosure, it should be understood that term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present disclosure.

The present disclosure may have various modifications and various examples, and specific examples will be

hereinafter illustrated in the drawings and described in detail in the detailed description. However, the present disclosure is not limited to specific embodiments, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

## MODE FOR CARRYING OUT THE INVENTION

The following Examples 1 to 9 disclose poly(organophosphazenes) loaded with TCA. A schematic diagram of a mechanism of action of TCA-loaded poly(organophosphazene) was shown in FIG. 1.

### Example 1. Experimental method A

Example 1-1. Materials

Hexachlorocyclotriphosphazene was purchased from Sigma-Aldrich (USA) and purified by sublimation at 55°C under vacuum (approximately 0.1 mmHg). Poly(dichlorophosphazene) was prepared as described by [Bromberg L., Temchenko M. Self-assembly in aqueous solutions of poly(ethylene oxide)-b-poly(propylene oxide)-b-poly(ethylene oxide)-b-poly(vinyl alcohol) Langmuir. 1999;15:8633-8639.]. Methoxy poly(ethylene glycol) (Sigma-Aldrich, USA) with a molecular weight of 750 Da was replaced with $\alpha$-amino-$\omega$-methoxy poly(ethylene glycol) (AMPEG) to be substituted into a poly(organophosphazene) backbone. l-Isoleucine ethyl ester hydrochloride (IleOEt · HCl) (A&Z food additives, Hang-Zhou, china), aminoethanol (Sigma-Aldrich, USA), glutaric anhydride (Alfa Aesar, USA), and 4-(dimethylamino) pyridine (DMAP) (Alfa Aesar, USA) were purchased. Tetrahydrofuran (THF) and trimethylamine (TEA) were reflux-dried over sodium metal and barium oxide under dry nitrogen, respectively. Triamcinolone acetonide (TCA) was purchased from TCI (Japan). All other reagents were purchased from commercial suppliers.

Example 1-2. Synthesis of poly(organophosphazene)

All reactions were performed in a dry nitrogen atmosphere using standard Schlenk-line techniques.

1-2-1. Synthesis of precursor polymer (PP, aminoethanol poly(organophosphazene))

A precursor polymer (PP) was prepared as follows. First, IleOEt · HCl (10.8 g, 55.22 mmol) was suspended in anhydrous THF (200 mL) and a reaction flask was cooled. Second, poly(dichlorophosphazene) (10.00 g, 8.63 mmol) was dissolved in anhydrous THF (200 mL), and then slowly added to a reaction flask containing IleOEt.HCl (23.64 g, 12.08 mmol) dissolved in anhydrous THA and TEA. The reaction mixture was stirred in a dry ice bath for 12 hours and then at 45°C for 24 hours. Third, AEtOH (1.42 g, 2.33 mmol) and AMPEG750 (13.59 g, 18.12 mmol) were dissolved in anhydrous THF and added to the reaction mixture. The reaction mixture was stirred at room temperature for 24 hours and then further stirred at 45°C for 24 hours. The reaction mixture was filtered and poured into n-hexane to obtain a precipitate, and then re-precipitated twice in the same solvent system. The polymer product was further purified by dialysis against methanol at room temperature for 4 days and against distilled water at 4°C for 4 days through a dialysis membrane (Spectra/Por, MWCO: 10-12 kDa). PP was obtained by freeze-drying a dialysate. The yield was 73.7% and the NMR data were as follows.

$^1$H NMR($CDCl_3$), $\delta$ (ppm): 0.8-1.0 (s, 6H), 1.1-1.3(b. 3H), 1.3-1.6(b, 2H), 1.6-1.9(b, 1H), 2.8-3.3(b, 2H), 3.4-3.8(b, 73H), 3.9 (s, 1H), 4.0-4.3(b, 3H).

1-2-2. Synthesis of carboxylic acid-terminal functionalized poly(organophosphazene) (CP)

To substitute a hydroxyl terminal with a carboxylic acid terminal, PP (10 g, 12.25 mmol) was dissolved in dry THF. The reaction mixture was stirred at room temperature for 12 hours and then heated to 40 to 45°C for another 24 hours. A final product, CP, was dialyzed against methanol at room temperature for 3 days and against water at 4°C for 3 days through a dialysis membrane. Purified CP was obtained by freeze-drying. A peak newly shown at 2.1 to 2.32 ppm (b, 4H, $CH_2$) represented a generated glutaric acid terminal that was not present in CP $^1$H NMR. The yield was 96% and the NMR data were as follows.

$^1$H NMR($CDCl_3$), $\delta$(ppm): $^1$H NMR (300 MHz, $CDCl_3$, $\delta$), d (ppm): 0.8-1.0 (s, 6H, $CH_3$), 1.1-1.3 (b, 3H, $CH_3$), 1.3-1.6 (b, 2H, $CH_2$), 1.6-1.9 (b, 1H, CH), 2.1-2.32 (b, 4H, $CH_2$), 2.8-3.3 (b, 2H, $CH_2$), 3.4-3.8 (b, 62H, $CH_2$), 3.9 (s, 1H, CH), 4.0-4.3 (b, 3H, $CH_3$).

Example 1-3. Identification of characteristics of CP

A structure of synthesized CP was estimated by measuring $^1H$ NMR (Varian Gem-ini-300 spectrometer operating at 300 MHz in a Fourier transform mode using $CDCl_3$). The surface charge of CP in an aqueous solution was measured using a Zetasizer Nano ZS (Malvern Instruments Ltd., Malvern, UK). The molecular weight (MW) of CP was calculated by a gel permeation chromatography system (Tosoh, EcoSEC HLC-8320 GPC) with two Styragel columns (TSKgel SupermultiporeHZ-M and TSKgel SuperHZ-2500) connected in series and a refractive index detector at a flow rate of 0.35 mL/min at 25°C. THF containing 0.1 wt% tetrabutylammonium bromide was used as a mobile phase. Polystyrene (MW: 162; 580; 1920; 3090; 9590; 27,810; 70,500; 133,500; 290,300; 729,500; 1,074,000; 2,703,000) was used as a standard.

Example 1-4. Biocompatibility experiment

1-4-1. Cytotoxicity test of CP

A cytotoxicity test of CP was performed using a mouse fibroblast cell line (NIH3T3). Cells were dispensed at a density of $2 \times 10^4$/well in a 96-well plate (SPL, Korea) and attached overnight in a Dulbecco's modified Eagle's medium (DMEM) supplemented with 10% fetal bovine serum and 1% penicillin/streptomycin in a humidified atmosphere of 5% $CO_2$ at 37°C. The culture medium was replaced with fresh DMEM after 24 hours and treated with various concentrations of CP (0 mg/mL to 10 mg/mL in DMEM, n = 6). After 24/48/72 hours, an MTT solution (3-(4,5-dimethylthiazol-2-yl)-2,5 diphenyltetrazolium bromide) was treated, and the medium was suctioned and then cultured for 3 hours. The precipitate was dissolved in 100 μl of DMSO, and the absorbance of a produced purple solution was measured at an excitation wavelength of 570 nm using a Spectra MAX 340 (BIO-RAD, Hercules, CA). Cell viability (%) was calculated as [ab]test/[ab] control × 100%.

1-4-2. Confirmation of mutagenicity of CP

The mutagenicity of CP was evaluated with several auxotrophic strains of the bacterium *Salmonella typhimurium.* Mutagenicity was determined by evaluating the production of revertant colonies (Ames test). For testing T98 and T100, a sterile liquid medium (2.5% Oxoid Nutrient Broth No. 2) was used. After culturing for 10 hours in a shaking bath (37°C, 200 rpm), 0.1 mL of the cultured bacteria was mixed with 2 mL of top agar, 0.1 mL of a CP solution, and S9 fraction. Thereafter, the mixture was carefully poured into a previously prepared petri dish containing 20 mL of a minimal glucose agar plate and cultured until solidified. After incubation for 48 hours at 37°C, the colony number was counted. Phosphate buffered saline (PBS) was used as a negative control, and 2-nitrofluorene, benzo(a)pyrene, and sodium azide were used as positive control groups specific for bacteria T98 and T100, respectively. All negative and positive control groups were performed in the same manner. A minimal glucose agar plate was prepared using Bacto agar (Difco), Vogel-Bonner medium E and 2% glucose.

Example 1-5. Preparation of self-assembled polymeric nanoparticles (PNs) and TCA-encapsulated polymeric nanoparticles (TePNs)

The synthesized CP was dissolved in a PBS solution at 4°C using a magnetic stirrer (12 wt% CP solution was prepared and 10-fold diluted). The prepared CP solution was observed by dynamic light scattering (DLS) using a Zetasizer Nano ZS (Malvern Instruments Ltd., Malvern, UK) and transmission electron microscopy (TEM, CM30 electron microscope, Philips, CA). The observed sizes and shapes of the self-assembled PNs were confirmed. TCA-encapsulated polymeric nanoparticles (TePNs) were prepared with three concentrations of TCA (0.3, 1.0, and 1.5 mg/mL). TCA was added directly to the PNs solution and mixed gently. The mixed solution was incubated at room temperature for 30 minutes to induce TCA encapsulation. The properties of TePNs were verified by DLS and TEM.

Example 1-6. Thermo-sensitive sol-gel transition behavior of TePN solution

The viscosity of a PN aqueous solution (12 wt%) was measured between 5 to 75°C at a constant shear rate of 0.1 $s^{-1}$ using a Brookfield RVDV-III + viscometer. The measurements were processed at a set spindle speed of 0.2 rpm and a heating rate of 0.33°C/min. Rheological measurement (MSC 102, Anton Paar, DE) was performed with a PN solution (12 wt% PN in PBS) and a TePN solution (0.3, 1.0, and 1.5 mg/mL of TCA were added to the prepared 12 wt% PN solution). Storage modulus (G') and loss modulus (G") were measured at a vibration frequency of 1 Hz, an oscillation strain of 5%, and temperatures of 4°C and 60°C with a gap length of 0.3 mm.

Example 1-7. In vivo degradation study of TePN hydrogel

A TePN solution (1.5 mg/mL of TCA, 200 μL) was injected subcutaneously into the backs of mice (Balb/c nude mice, 6-week-old, male, Orient Bio, Korea) using a 31-gauge needle. Locally generated TePN hydrogels were identified immediately after injection. The remaining TePN hydrogel was weighed every predetermined day.

Example 1-8. In vitro release of TCA from TePN

TePN solutions were prepared as 12 wt% PN solutions containing 0.3, 1.0, and 1.5 mg/mL of TCA, respectively. 0.3 mL of the prepared TePN solution was added to a tube and the temperature was raised to 37°C to confirm hydrogel formation. 6 mL of a PBS solution was gently added to the hydrogel, and the tube was incubated in a water bath (KMC-12055W1, Vision, Korea) at 37°C with gentle shaking motion (50 rpm). The PBS solution was replaced with a fresh PBS solution at each time point. The amount of released TCA was measured by high-performance liquid chromatography (HPLC, Agilent) using water/acetonitrile (50/50, v/v%) as an eluent and calculated with a standard sample established using UV detection at 240 nm.

Example 1-9. In vivo anti-OA effect of TePN hydrogel system

1-9-1. Intra articular injection of TePN in rats with osteoarthritis (OA)

OA was induced by injecting monosodium iodoacetate (MIA, Sigma-Aldrich, USA) (0.5 mg/50 μl) into the intra-articular area of Sprague Dawley (SD) rats (6 weeks old, male, orient Bio, Korea) (n = 6 per group). One week later, OA-induced rats were treated with 0.3 mL of a TePN solution by intra articular injection. A non-treated MIA-induced OA rat model was used as a control group. Rats were sacrificed 8 weeks after TePN injection for further analysis.

1-9-2. Intra-articular structure and X-ray, micro-computed tomography (μ-CT) scanning

Eight weeks after injection, the rats were sacrificed, and the isolated knee joints were excised from the femur and tibia. The isolated knee samples were scanned using X-ray (In-Vivo Series, DXS PRO, Carestream, USA) and μ-CT (Aibira CT system, Carestream, USA). The distance of destroyed cartilage was measured using μ-CT images and Image J software.

1-9-3. Histological analysis

All collected tissues were embedded in paraffin and sectioned using a microtome (8 μm-thick). For histological evaluation, the tissue sections were deparaffinized, rehydrated, and stained. After staining with hematoxylin and eosin (H&E) and safranin-O, the histological aspects of individual knee joints were observed under a light microscope (Nikkon; E400, Japan).

1-9-4. RNA isolation and real-time polymerase chain reaction

Blood samples were collected at each time point (1 week, 4 weeks, and 8 weeks after OA induction and TePN hydrogel treatment). RNA was isolated using an RNA blood kit (QIAamp, QIAGEN) according to the manufacturer's instructions. cDNA was synthesized using Accupower CycleScriptRT Pre Mix (dT20) (BIO-RAD) according to the manufacturer's protocol. Real-time polymerase chain reaction (PCR) amplification and detection were performed using an ABI7300 Real-Time Thermal cycler (Applied Biosystems, Foster City, CA, USA). Gene expression of pro-inflammatory cytokines such as metalloproteinase-3 (MMP-3), MMP-13, interleukin-6 (IL-6), and tumor necrosis factor-α (TNF-α) and anti-inflammatory cytokines such as IL-4, IL-10, and IL-13 was examined, respectively. The sequences of each genetic marker were listed in Table 1 below.

[Table 1]

| Gene | Forward sequence | Reverse sequence |
|---|---|---|
| MMP-3 | GAACAATGGACAAAGGTACAACA | GGTCCTTGGAGTGGTCAAGA |
| MMP-13 | TAAGGAGCATGGCGACTTCT | GGTCCTTGGAGTGGTCAAGA |

(continued)

| Gene | Forward sequence | Reverse sequence |
|---|---|---|
| IL-6 | AACGATGATGCACTTGCAGA | GAGCATTGGAAATTGGGGTA |
| TNF-α | GGCAGGTCTACTTTGGAGTCATTGC | ACATTCGACGCTCCATGTAATTCGG |
| IL-4 | ACAGGAGAAGGGACGCCAT | GAAGCCCTACAGACGAGCTCA |
| IL-10 | GGTTGCCAAGCCTTATCGGA | ACCTGCTCCACTGCCTTGCT |
| IL-13 | AGACCAGACTCCCCTGTGCA | TGGGTCCTGTAGATGGCATTG |

**Example 2. Synthesis and properties of poly(organophosphazene)**

Poly(organophosphazene)s containing mainly hydrophobic l-isoleucine ethyl ester (IleOEt) and hydrophilic α-amino-ω-methoxy poly(ethylene glycol) (AMPEG) in the side chains were synthesized and used as a thermoresponsive and in situ gelling drug storage system. In addition, the extracellular matrix of natural cartilage, which was mainly composed of chondroitin sulfate and hyaluronic acid, was mimicked by introducing a carboxylic acid terminal, and interacted with various cytokines related to cartilage homeostasis and regeneration, which was as shown in Chemical Formula below.

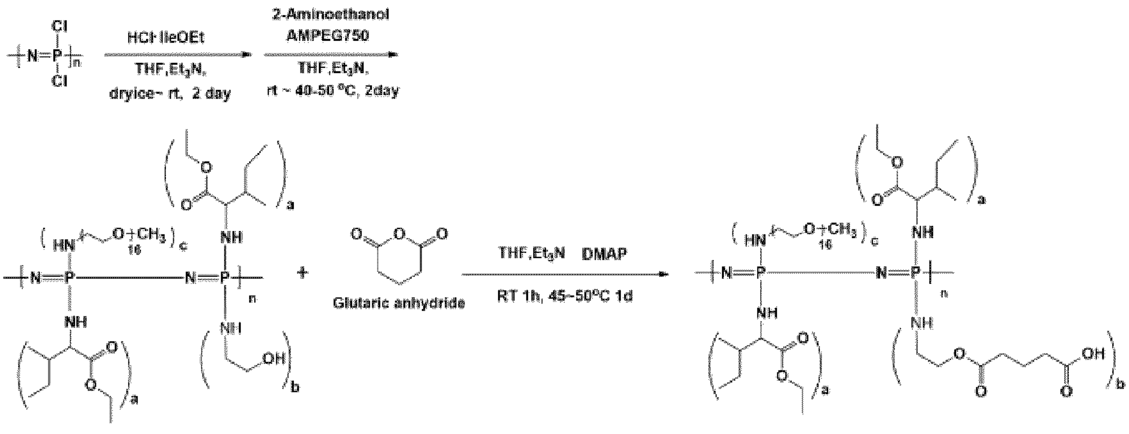


PP CP

The molar ratios of substituted side chains were calculated using NMR spectra. The concentrations of IleOEt, AMPEG and carboxylic acid were 70%, 10.5% and 13.5% in the final polymer, respectively. The substitution degree of each complex in the polymer was evaluated by integration of specific peaks at 0.8 to 1.0 ppm for 6 protons of IleOEt/2.1 to 2.32 ppm for 4 protons of carboxylic acid/3.4 to 3.8 ppm for 62 protons of AMPEG as $^{1}H$ NMR data (FIG. 2). The charge of the final polymer was characterized by zeta potential measurement as - 4.0 ± 1.6, as a negative charge mimicking the extracellular matrix of cartilage, and GPC analysis using a polystyrene standard represented a molecular weight of 50,085 Da.

In addition, the biocompatibility of poly(organophosphazene) was evaluated as follows. The Ames test was a test to evaluate the potential mutagenicity of test chemicals, which were generally associated with carcinogenic potency. For the mutagenicity test, Salmonella typhimurium TA98 or TA100 was treated with 200 μg of a polymer. The polymer-treated group showed revertant colonies almost identical to the negative control group (FIG. 3A). These non-toxic and mutagenic reactions suggest the safety of the synthesized polymer, and these results support the possibility that the polymer may be used as a bioavailable substance. Particularly, for anti-OA treatment, TCA carriers should not promote immune responses at the lesion site to require highly biocompatible properties. For these reasons, it was confirmed that the examined biocompatibility of the synthesized poly(organophosphazene) was suitable as an anti-OA drug delivery system.

Cytotoxicity and mutagenicity tests were conducted to generate toxicity data for novel drugs or bioavailable substances. The toxicity of the synthesized poly(organophosphazene) was examined using the MTT assay and Ames test. The cytotoxicity of the polymer was tested on an NIH 3T3 cell line (polymer concentrations in the medium ranged of 0 mg/mL to 30 mg/mL). After 24 hours of treatment, cell viability of 90 to 95% was observed at all polymer concentrations (FIG. 3B). There was no cytotoxic effect after treating the polymer of the same composition with CP for 48 and 72 hours, as

shown in FIG. 4. It was shown that CP was a cytocompatible substance.

**Example 3. Characteristics of self-assembled PN and TCA encapsulation**

The side chains of a polymer are filled with hydrophobic and hydrophilic portions to have balanced amphiphilicity. The polymer exists as spherical nanoparticles in an aqueous environment to minimize the exposure region of hydrophobic IleOEt units of the polymer to water molecules. In addition, a hydrophobic drug such as TCA interacts with the hydrophobic core portion of PN (FIG. 5A). Self-assembled PN and TePN were examined by TEM and size distribution studies. The round-shaped PNs were identified by a TEM image (FIG. 5B) and had a size of ~140 nm at 25°C (120 mg/mL of PNs in a phosphate-buffered saline (PBS) solution) with a margin of error of ≤ 4 nm. TCA, a low water-soluble drug, exhibited an irregular nano- and micro-sized crystallin in the range of 232.9 nm to 3343 nm (error range ≥1450 nm in DLS and TEM data) in a PBS solution (1.5 mg/mL TCA in PBS) (FIGS. 5C and 6). However, the fine-sized TCA aggregates disappeared from the size distribution after mixing and incubation with the PN-dispersed PBS solution. Only particles with the size of ~140 nm were measured with an error range of ≤ 4 nm in the mixed solution of TCA and PN (120 mg/mL of PN and 1.5 mg/mL of TCA in PBS solution) (FIG. 5D). These results indicate successful encapsulation of TCA into PN nanoparticles. The average size and polydispersity index (PDI) values of these particles are shown in FIGS. 5E and 7. The size change and significant reduction demonstrate that TCA may be well encapsulated into the hydrophobic core of PN by hydrophobic interactions.

**Example 4. Confirmation of thermo-sensitive sol-gel transition behavior of TePN solution**

In an aqueous solution of PNs of a certain concentration or higher, clear viscosity change and phase transition occur according to a temperature change. This phase transition occurred because the hydrogen bonds between the hydrophilic PEG chains of PNs and water molecules became weaker as the temperature increased, whereas the hydrophobic interaction between PNs increased. In this study, a 12 wt% PN solution was prepared to have good injectability, sufficient viscosity, and maximum storage modulus to form a gel at a body temperature. As shown in FIG. 8A, the PN solution exhibited the viscosity close to 0 Pa·s up to 32°C. The viscosity value increased continuously at 32°C and reached 518.75 Pa·s at 37°C. Similarly, both the storage and loss modulus values increased at 32°C, and the storage modulus value G' was greater than the loss modulus values G" between 32°C and 44°C in the rheological study. The G' and G" values at 37°C were 1284.3 and 765 Pa, respectively, indicating a gel state (FIG. 8B).

In addition, the sol-gel transition behavior after TCA loading was monitored because the physical properties of the drug delivery hydrogel may significantly affect a drug release pattern or duration, as shown in FIG. 8C. The storage and loss modulus of the TePN solution were measured after TCA loading at three different volumes. All groups showed similar changes in storage and loss modulus values according to a temperature change. These results may demonstrate that the loaded TCA was homogeneously encapsulated inside the PN and had little effect on the sol-gel transition behavior, and these properties are expectable in that the hydrogel will exhibit similar behavior even at various drug doses.

**Example 5. In vivo degradation of TePN hydrogel**

Since biodegradation was a prerequisite for biomaterials and drug delivery systems, the hydrogel degradation rate and general symptoms were monitored around the tissue where the PN hydrogel was locally placed. The prepared PN solution was injected under the mouse skin using a 31-gauge needle. The formation of PN hydrogel was directly confirmed after injection, and the remaining PN hydrogel was measured through a simple separation process from the skin at an intended time (FIG. 9A). The amount of the remaining PN hydrogel decreased over time up to day 42 (FIGS. 9B and 10). These biodegradable properties, mild conditions for PN hydrogel formation, and long-term retention at the injection site indicate that the developed PN hydrogel system is a long-term drug storage and suitable for use as a drug delivery carrier.

**Example 6. Confirmation of sustained release profile of TCA from TePN in vitro**

Steroid anti-inflammatory drugs such as TCA have a short retention time after Intra articular administration and are rapidly eliminated from the body. Therefore, long-term anti-inflammatory effects are limited during general administration. Since the TePN hydrogel system has a tendency that strongly interacting drugs are highly dependent on the degradation properties of the material, the TCA release pattern and duration may be controlled by controlling the dissociation and degradation rates of the PN hydrogel. The TCA release behavior was experimented using freshly prepared TePN solutions containing three different concentrations of TCA. After forming TePN hydrogels at 37°C, each TePN hydrogel was immersed in a PBS buffer. In contrast to the PN group, which showed rapid swelling in less than 1 day, most of the hydrogels with TCA showed swelling after 1 week, which was considered because the hydrophobic interaction between TCA and the hydrogel improved the stability of the polymer structure in the aqueous solution (FIG. 11). In addition, in all of the gels, the mass loss started after 4 weeks (FIG. 12A). The mass loss was closely correlated with the TCA release

pattern of the hydrogel for 42 days (FIG. 12B). The inhibited initial release of TCA was observed in three TePN hydrogel groups with TCA concentrations of 0.3, 1.0, and 1.5 mg/mL for first 24 hours, and the observed values were read as 14.45%, 9.55%, and 9.72%, compared to the total loaded TCA amount, respectively. These results were in contrast to a result that the half-life of TCA without a delivery carrier was only 1.27 hours in the human body when administered directly. In each TePN hydrogel group, approximately half of the loaded TCA amount was released with values of 0.3, 1.0, and 1.5 mg/mL at days 7, 14, and 16, respectively. The sustained release process continued until the TePN hydrogel was completely degraded. Meanwhile, the TePN hydrogel group with the lowest TCA concentration showed a faster release pattern than other two groups. The low TCA concentration may affect loss of a hydrophobic network, rapid swelling, and rapid dissolution. The TCA release pattern was linear over time in the TePN hydrogel groups containing 1.0 mg/mL and 1.5 mg/mL TCA. The degradation and TCA release periods of the TePN hydrogels were almost similar to each other. The inhibited initial release and continuous TCA release show that TCA interacts strongly with the polymer and is well encapsulated by PN.

**Example 7.** Confirmation of long-term anti-inflammatory and cartilage degeneration prevention effects by single injection of TePN hydrogel in OA rat model

Long-term anti-inflammation and cartilage degeneration inhibition effects were verified with single injection of a TePN hydrogel storage system. A rat model with monosodium iodoacetate (MIA)-induced OA was prepared for treatment with the TePN hydrogel system. TePN injection was performed 1 week after OA generation to mimic the early stage of OA. In addition to the TePN hydrogel group, TCA solution direct injection and saline groups were added as control groups. 300 μl each of saline, TePN, and TCA solutions were prepared and injected into the injured area of the rat.

Eight weeks after treatment, the knee joints of the sacrificed rat were dissected, and as a result, a clearly rare inflammatory response was shown only in the TePN-treated group compared to other groups (FIG. 13). X-ray and μ-CT images were also taken for morphological studies as shown in FIG. 14A. The saline-treated group showed advanced osteoarthritis with severe cartilage loss and cartilage destruction. However, each TePN-treated group showed a well-maintained morphological characteristic, which indicated that inflammation in the early stage of OA was well inhibited by sustained-release TCA. In addition, all of the TePN hydrogel groups showed significant improvement in the anti-OA effect compared to the TCA solution group. The destroyed cartilage condition in the TCA solution direct injection group reflects the failure of long-term inflammation treatment and prevention of cartilage degeneration. The pathological condition of cartilage in the TCA solution group was similar to or worse than that in the saline treated group. This was expected because the possible adverse effects of direct IA injection of TCA on chondrocytecytotoxicity and the local treatment with a high dose of TCA may decrease hydroxyproline production to inhibit fibroblast growth and collagen synthesis, so that the structural stability of the connective tissue around the knee may be reduced. For statistical evaluation, an interarticular cartilage (DIC) distance was measured (FIG. 14B). Most DICs were similarly increased in both the saline and TCA solution treated groups on both the left and right parts, but only a statistically significant difference was observed between the saline group and the high-concentration TCA solution (1.5 mg/mL) group in the left part of the cartilage. Severe cartilage destruction may occur for two reasons why 1) the toxic potential of direct and high-dose TCA exposure in a short period of time and 2) the sustained anti-inflammatory effect due to rapid TCA clearance are inadequate.

In addition, the contrasting results that cartilage degeneration was well prevented in the TePN hydrogel group, whereas OA treatment failed in the TCA solution direct injection group, were proven by a histological staining method (FIG. 15). Hematoxylin and eosin (H&E) and safranin O staining showed the morphological characteristics of each experimental group. In the saline and TCA solution-treated groups, the cartilage appearance became thinner, the tissue containing the chondrocytes was lost, and the characteristic red staining of glycosaminoglycans identified in healthy cartilage disappeared. These results indicate that treatment with saline and TCA solutions has little effect on the progression of OA after initial onset. However, the TePN hydrogel group showed morphological similarity to normal cartilage, and the cellular characteristics of chondrocytes were well maintained. In this experiment, it could be confirmed that sustained release of TCA through single injection of the TePN hydrogel system may effectively prevent additional inflammation and OA progression.

**Example 8. Confirmation of effects of sustained TCA release using TePN hydrogel system on pro-inflammatory gene inhibition and anti-inflammatory gene activation**

Changes in inflammatory gene expression were evaluated during an experimental period. Expression levels of pro-inflammatory and anti-inflammatory genes were evaluated from corrected blood samples. One week after the onset of MIA-induced osteoarthritis, the gene expression levels of pro-inflammatory cytokines MMP-3, MMP-13, IL-6, and TNF-α were increased 5.60-, 4.25-, 4.80-, and 5.35-fold, respectively, compared to a normal group (FIG. 16A). Contrasting gene expression patterns were observed in each experimental group at 1, 4, and 8 weeks after injection of the TePN or TCA solution. The increased gene expression levels of pro-inflammatory cytokines decreased over time in the TePN hydrogel

group, but continued to increase in a TCA solution group. Since the expression of pro-inflammatory cytokines and MMPs causes cartilage degeneration, it is possible to successfully prevent the cartilage degeneration by effectively lowering the levels of proinflammatory cytokines and MMPs cytokines. After 8 weeks, it was confirmed that the levels of cytokine genes MMP-3, MMP-13, IL-6, and TNF-$\alpha$ in the TePN hydrogel with the highest TCA concentration were decrease by 1.62-fold, 1.67-fold, 1.49-fold, and 1.62-fold, respectively, compared to the saline treatment group. In contrast, in the TCA solution group, the level of each pro-inflammatory cytokine gene continued to increase over time. Since directly administered TCA was rapidly eliminated, the anti-inflammatory effect of a single IA injection of the TCA solution was insufficient to prevent the degradation of the extracellular matrix and collagen degradation of cartilage.

Anti-inflammatory cytokine genes such as IL-4, IL-10, and IL-13 were simultaneously monitored (FIG. 16B). These gene expressions are known to be associated with stimulation of the survival, proliferation, and differentiation of immune cells such as macrophages and specific T cells (Th2). In addition, the expression levels of these cytokines may serve as a criterion for inhibiting the synthesis of pro-inflammatory cytokines such as interferon-gamma, interleukin, and TNF-$\alpha$. One week after the onset of MIA-induced osteoarthritis, the expression levels of IL-4, IL-10, and IL-13 genes were similar to normal in all treatment groups. There was no significant difference between the treatment groups up to 4 weeks after inflammation induction. However, after 8 weeks, the expression level of each gene significantly increased in the TePN hydrogel treated group. In addition, the margin of the increase in gene expression expanded depending on the concentration of loaded TCA. After 8 weeks, the gene expression levels of IL-4, IL-10, and IL-13 in the TePN hydrogel with the highest TCA concentration were 1.91-fold, 2.25-fold, and 1.92-fold higher than those in the saline-treated group, respectively. These results support the efficacy of sustained TCA release and the long-term anti-inflammatory effect following a single injection. There were no abnormal symptoms for the weight (FIG. 17). There were no changes in the expression levels of pro-inflammatory and anti-inflammatory genes by the treatment with the PN hydrogel without TCA. That is, the carrier PN hydrogel system does not affect the systemic inflammatory response.

**Example 9. Optimization of release characteristics according to IleOEt/AMPEG molar ratio**

Since the hydrophobic property of PN, and the drug loading capacity and sustained-release capacity related to the hydrophobic property varied depending on a molar ratio of IleOEt/AMPEG, it was intended to identify a polymer composition with optimal release characteristics by controlling the molar ratio of IleOEt/AMPEG.

Poly[(isoleucine ethyl ester)$_{1.47}$(aminomethoxypolyethylene glycol 750)$_{0.24}$(ethanol)$_{0.21}$(acid)$_{0.08}$phosphazene]$_n$ (hereinafter PPZ 43), poly[(isoleucine ethyl ester)$_{1.37}$(aminomethoxypolyethylene glycol 750)$_{0.25}$(ethanol)$_{0.27}$(acid)$_{0.11}$phosphazene]$_n$ (hereinafter PPZ 46), and poly[(isoleucine ethyl ester)$_{1.44}$(aminomethoxypolyethylene glycol 750)$_{0.30}$(ethanol)$_{0.08}$(acid)$_{0.18}$phosphazene]$_n$ (hereinafter PPZ 24) were prepared as follows, and NMR data of the prepared PPZs were shown in FIG. 18.

PPZ 43 was prepared as follows: A precursor polymer (PP) was prepared as follows. First, IleOEt · HCl (24.65g, 112.98mmol) was suspended in anhydrous THF (200 mL) and a reaction flask was cooled. Second, poly(dichlorophosphazene) (10.00 g, 8.63 mmol) was dissolved in anhydrous THF (200 mL), and then slowly added to a reaction flask containing IleOEt.HCl (24.65 g, 112.98 mmol) dissolved in anhydrous THA and TEA. The reaction mixture was stirred in a dry ice bath for 12 hours and then at 45°C for 24 hours. Third, AEtOH (1.05 g, 17.26 mmol) and AMPEG750 (22 g, 29.34 mmol) were dissolved in anhydrous THF and added to the reaction mixture. The reaction mixture was stirred at room temperature for 24 hours and then further stirred at 45°C for 24 hours. The reaction mixture was filtered and poured into n-hexane to obtain a precipitate, and then reprecipitated twice in the same solvent system. The polymer product was further purified by dialysis against methanol at room temperature for 4 days and against distilled water at 4°C for 4 days through a dialysis membrane (Spectra/Por, MWCO: 10-12 kDa). PP was obtained by freeze-drying a dialysate.

To substitute a hydroxyl terminal with a carboxylic acid terminal, PP (10 g, 12.25 mmol) was dissolved in dry THF. Anhydrous glutaric acid (2.80 g, 24.5 mmol) and dimethylaminopyridine (2.99 g, 24.5 mmol) were added therein, and the reaction mixture was stirred at room temperature for 12 hours, and then further heated to 40 to 45°C for 24 hours. A final product, CP, was dialyzed against methanol at room temperature for 3 days and against water at 4°C for 3 days through a dialysis membrane. Purified CP was obtained by freeze-drying. A peak newly shown at 2.1 to 2.32 ppm (b, 4H, $CH_2$) represented a generated glutaric acid terminal that was not present in CP $^1$H NMR.

PPZ 46 was prepared as follows: A precursor polymer (PP) was prepared as follows. First, IleOEt · HCl (23.98 g, 122.53 mmol) was suspended in anhydrous THF (200 mL) and a reaction flask was cooled. Second, poly(dichlorophosphazene) (10.00 g, 8.63 mmol) was dissolved in anhydrous THF (200 mL), and then slowly added to a reaction flask containing IleOEt.HCl (23.98 g, 122.53 mmol) dissolved in anhydrous THA and TEA. The reaction mixture was stirred in a dry ice bath for 12 hours and then at 45°C for 24 hours. Third, AEtOH (1.32g, 21.57 mmol) and AMPEG750 (21.36 g, 28.48 mmol) were dissolved in anhydrous THF and added to the reaction mixture. The reaction mixture was stirred at room temperature for 24 hours and then further stirred at 45°C for 24 hours. The reaction mixture was filtered and poured into n-hexane to obtain a precipitate, and then reprecipitated twice in the same solvent system. The polymer product was further purified by dialysis against methanol at room temperature for 4 days and against distilled water at 4°C for 4 days through a

dialysis membrane (Spectra/Por, MWCO: 10-12 kDa). PP was obtained by freeze-drying a dialysate.

To substitute a hydroxyl terminal with a carboxylic acid terminal, PP (10 g, 12.25 mmol) was dissolved in dry THF. Anhydrous glutaric acid (2.80 g, 24.5 mmol) and dimethylaminopyridine (2.99 g, 24.5 mmol) were added therein, and the reaction mixture was stirred at room temperature for 12 hours, and then further heated to 40 to 45°C for 24 hours. A final product, CP, was dialyzed against methanol at room temperature for 3 days and against water at 4°C for 3 days through a dialysis membrane. Purified CP was obtained by freeze-drying. A peak newly shown at 2.1 to 2.32 ppm (b, 4H, $CH_2$) represented a generated glutaric acid terminal that was not present in CP $^1$HNMR.

PPZ 24 was prepared as follows, and a precursor polymer (PP) was prepared as follows. First, IleOEt · HCl (26.43 g, 134.61 mmol) was suspended in anhydrous THF (200 mL) and a reaction flask was cooled. Second, poly(dichlorophosphazene) (10.00 g, 8.63 mmol) was dissolved in anhydrous THF (200 mL), and then slowly added to a reaction flask containing IleOEt · HCl (26.43 g, 134.61 mmol) dissolved in anhydrous THA and TEA. The reaction mixture was stirred in a dry ice bath for 12 hours and then at 45°C for 24 hours. Third, AEtOH (1.05 g, 17.26 mmol) and AMPEG750 (15.53 g, 20.71 mmol) were dissolved in anhydrous THF and added to the reaction mixture. The reaction mixture was stirred at room temperature for 24 hours and then further stirred at 45°C for 24 hours. The reaction mixture was filtered and poured into n-hexane to obtain a precipitate, and then reprecipitated twice in the same solvent system. The polymer product was further purified by dialysis against methanol at room temperature for 4 days and against distilled water at 4°C for 4 days through a dialysis membrane (Spectra/Por, MWCO: 10-12 kDa). PP was obtained by freeze-drying a dialysate.

To substitute a hydroxyl terminal with a carboxylic acid terminal, PP (10 g, 12.25 mmol) was dissolved in dry THF. Anhydrous glutaric acid (2.80 g, 24.5 mmol) and dimethylaminopyridine (2.99 g, 24.5 mmol) were added therein, and the reaction mixture was stirred at room temperature for 12 hours, and then further heated to 40 to 45°C for 24 hours. A final product, CP, was dialyzed against methanol at room temperature for 3 days and against water at 4°C for 3 days through a dialysis membrane. Purified CP was obtained by freeze-drying. A peak newly shown at 2.1 to 2.32 ppm (b, 4H, $CH_2$) represented a generated glutaric acid terminal that was not present in CP $^1$H NMR.

The $T_{max}$ of the prepared PPZ 43, 46, and 24 were measured and shown in FIG. 19A. As shown in FIG. 19A, as the molar ratio of IleOEt/AMPEG increased, the hydrophobicity increased and the $T_{max}$ value decreased. In FIG. 19B, the cumulative release amount over time after loading each compound with TCA is represented by mg and %, respectively. It was confirmed that as the hydrophobic property became stronger, the hydrophobic drug TCA was further slowly released.

Referring to the release profile of TCA shown in Example 6 and FIG. 12, the release rate of TCA in TePN 1.5 (TCA encapsulated PN loaded at a concentration of 1.5 mg/ml) was 0.011 to 0.018 mg/day (11 to 18 ug/day). TePN is applied to the body in an amount of about 200 to 300 μl and gradually degraded over about 30 days to release TCA. Therefore, it was attempted to calculate the amount of TCA that may be released and exposed in the body during a day and apply the calculated amount to an in vitro experiment. To this end, 1.5 mg/ml of TCA which had been loaded on TePN 1.5 was diluted 20-fold to 160-fold, respectively, and loaded at concentrations of 9.375, 18.75, 37.5, and 75 ug/ml, respectively.

A complex of the loaded TCA and PPZ was treated to chondrocytes in an inflammatory environment, and the expression of inflammation-related factors was measured by PCR. To induce an inflammatory environment, an in vitro arthritis model was formed by treating human chondrocytes with IL-1β at 10 ng/ml. When the TCA-PPZ complex loaded at each concentration was treated in the model, the relative expression levels of MMP3, MMP13, IL-6, and TNF-α were measured and shown in FIG. 20. As can be seen in the FIG. 20, it was confirmed that MMP3, MMP13, and IL-6 (inflammatory cytokines) increased rapidly after treatment with IL-1β, and thus the inflammatory environment was well induced. At this time, when treated with 9.375 to 37.5 ug/ml of the TCA-PPZ complex, it was confirmed that the expression of MMP3, MMP13, and IL-6 decreased rapidly, whereas at the highest dose of 75 ug/ml, the relative decrease in expression level was small, and thus the inflammation relief effect was reduced further than at the concentration of 9.375 to 37.5 ug/ml. That is, the high dose of TCA was shown to have a negative effect, and it was suggested that the characteristics of drug delivery systems that slowly released a constant concentration of drug may be more helpful in the treatment of OA.

To determine the optimal IleOEt/AMPEG molar ratio, the following experiment was conducted. In an in vitro release experiment, it was confirmed that 200 μl of the TCA-PPZ complex was released in the amounts shown in Table 2.

[Table 2]

| TCA release amount (ug) | 43GA | 46GA | 24GA |
|---|---|---|---|
| DAY 1 | 19.32 | 58 | 146.8 |
| DAY 2 | 10.28 | 34.16 | |
| DAY 3 | 5.2 | 17.06 | |
| DAY 4 | 4.4 | 9.71 | |

Based on this, an expected amount of TCA to be released was calculated assuming treatment with 5 ml of a TCA-PPZ solution and was shown in Table 3 below. The In vitro OA model assumed a case where chondrocytes were mainly treated with IL-1β, and since 10 ml of culture medium was often used, 5 ml of TCA-PPZ hydrogel, which was half the volume of the culture medium, was treated.

[Table 3]

| TCA release amount (ug, expected value) | 43GA | 46GA | 24GA |
|---|---|---|---|
| DAY 1 | 483 | 1450 | 3670 |
| DAY 2 | 257 | 854 | |
| DAY 3 | 130 | 426.5 | |
| DAY 4 | 110 | 242.8 | |

Based on the predicted values in Table 3 above, the optimal IleOEt/AMPEG molar ratio was calculated as follows. First, since the total culture medium volume was 10 ml, a value obtained by dividing the predicted TCA release amount in Table 3 above by 10 was predicted as the TCA release concentration in ug/ml unit. The predicted TCA release concentration was indicated for each day as shown in FIG. 21. Referring to FIG. 21, it could be seen that the polymer composition in which the expression levels of MMP3, MMP13, and IL-6 were included within the optimal range of 9.375 ug/ml to 37.5 ug/ml from day 1 to day 4 corresponded to 43GA, and the IleOEt/AMPEG molar ratio thereof was confirmed to be approximately 6.125 to exhibit the best sustained-release characteristics in the corresponding molar ratio range.

The following Example 10 discloses the preparation of poly(organophosphazene) for loading celecoxib (CXB) and effects thereof. A schematic diagram of treatment of Achilles tendonitis (AT) with poly(organophosphazene) loaded with celecoxib was shown in FIG. 22.

**Example 10. Experimental Method B**

Example 10-1. Materials

Hexachlorocyclotriphosphazene (Sigma-Aldrich, USA) was recrystallized at 60°C under vacuum for purification. Poly(dichlorophosphazene) was synthesized as described in Example 2 above. l-Isoleucine ethyl ester hydrochloride (IleOEt HCl) (A&Z food additives, Hangzhou, China) was purchased and dried under vacuum at 55°C for 10 days, and Methoxy Polyethylene glycol (750 Da) was purchased from Sigma-Aldrich (USA) and replaced with α-amino-ω-methoxy-poly(ethylene glycol) (AMPEG). Ethanolamine (Sigma-Aldrich, USA), glutaric anhydride (Alfa Aesar, USA), and 4-(dimethylamino) pyridine (DMAP) (Alfa Aesar, USA) were prepared. Tetrahydrofuran (THF) and triethylamine (TEA) were dissolved in nitrogen gas, and THF was dried using sodium metal (Aldrich, USA) and benzophenone (Daejeong, Korea). TEA was dried using barium oxide (Daejeong, Korea). Celecoxib (CXB) was purchased from EDQA (Europe).

Example 10-2. PNP synthesis

A reaction was carried out in a dry nitrogen atmosphere using a standard Schlenk line. IleOEt·HCl (10.38 g, 53.06 mmol) was dissolved in anhydrous THF. The reactor was stirred in a dry ice bath and 50 mL of dry TEA was added. Poly(dichlorophosphazene) (5.00 g, 43.14 mmol) was dissolved in dried THF and added to the reactor. The reaction temperature was set at 50°C for 24 hours, and the polymer was further reacted for 48 hours by adding ethanolamine (0.91 g, 15.1 mmol) and AMPEG (13.59 g, 18.12 mmol). The polymer solution was filtered and the solvent was removed using a rotary evaporator. The polymer was precipitated twice by pouring into n-hexane (Daejeong, Korea), and further purified using a membrane dialysis (Spectra/Por, MWCO: 10-12 kDa) against methanol for 4 days and then using distilled water for 4 days. The purified polymer solution was filtered using a syringe filter with 0.45 μm pores and lyophilized to obtain aminoethanol-bound poly(organophosphazene). Dry THF was added to aminoethanol-poly(organophosphazene) (10 g, 158.93 mmol) to bind carboxylic acid groups. Glutaric anhydride (5.98 g, 52.41 mmol) and DMAP (6.40 g, 52.41 mmol) were dissolved in anhydrous THF and added to an aminoethanol PPZ solution. Then, the solution was stirred and incubated at 45°C for 24 hours. Thereafter, the polymer was dialyzed against methanol using a dialysis membrane for 4 days and distilled water was used for 4 days before being freeze-dried. Through the preparing process, a polymer composed of (IleOEt), (AMPEG750), (EtOH) and (Acid) was obtained.

Example 10-3. Confirmation of PNP characteristics

$^1H$ NMR results were as follows.

$^1H$ NMR($CDCl_3$), δ(ppm): 0.8-1.0 (s, 6H), 1.1-1.3 (b, 3H), 1.3-1.6 (b, 2H), 1.6-1.9 (b, 1H), 2.1-2.3 (b, 4H), 2.8-3.3 (b, 2H), 3.4-3.8 (b, 62H), 3.9 (s, 1H), and 4.0-4.3 (b, 3H)

The molecular weight of the polymer was measured using a gel permeation chromatography system (EcoSEC HLC-8320 GPC, Tosoh) and a refractive index detector. Two Styragel columns (TSKgel SupermultiporeHZ-M and TSKgel SuperHZ-2500) were connected and used at a flow rate of 0.35 mL $min^{-1}$ at 40°C. THF was used with 0.1% (w/v) tetrabutylammonium bromide as the mobile phase. Polystyrene (MW: 162; 580; 1920; 3090; 9590; 27 810; 70 500; 133 500; 290 300; 729 500; 1 074 000; and 2 703 000 Da) was used as a standard. Fourier transform infrared spectroscopy (FTIR) was performed to characterize the presence of CXB and PNP in PCNP using an FTIR spectrometer (Nicolet i20 FTIR spectrometer, Thermo Fisher Scientific). The sample was dissolved in chloroform at 1 wt% and coated on a KBr circular cell (Sigma-Aldrich, USA). After drying the chloroform, the coated sample was analyzed and an FTIR spectrum was obtained by scanning 32 times in a wavenumber range of 4000 to 500 $cm^{-1}$.

Example 10-4. Cell viability test

A NIH3T3 fibroblast cell line and serially diluted PNP hydrogels (0 to 10 mg/mL) were used. Cells were dispensed at a density of $2 \times 10^4$ cells/well in a 96-well cell culture plate (SPL, Korea) and incubated in a Dulbecco's modified Eagle's medium (DMEM) supplemented with 10% fetal bovine serum and 1% penicillin/streptomycin in a 37°C $CO_2$ incubator for 24 hours. The culture medium was replaced with an FBS-free DMEM with different concentrations of PNP, CXB, and PCNP. After 24 hours, an MTT solution (3-(4,5-dimethylthiazol-2-yl)-2,5 diphenyltetrazolium bromide) was added and then incubated for 2 hours in a 37°C $CO_2$ incubator. Dimethyl sulfoxide (200 μl; Daejung, Korea) was added, and the absorbance of the solution was measured at 570 nm using a Spectra MAX 340 spectrophotometer (BIO-RAD, Hercules, CA). The viability of NIH3T3 cells was calculated using the absorbance of experimental and control groups using the following Equation.

Cell viability (%) = Absorbance (experimental group)/Absorbance (control group) $\times$ 100

Example 10-5. Rheological Test

The thermo-sensitive sol-gel transition of PPZ was measured using a rheometer (Anton Parr, AT). PNP and PCNP hydrogel solutions were prepared at a 10 wt% concentration. The modulus was measured at a precise temperature. The rheometer was used with a 25.0 mm parallel plate and a 0.5 mm zero gap. The modulus was measured under vibration stress at a frequency of 0.8 Hz and 10% strain at various temperatures. A frequency sweep test was also performed under 10% shear strain at 37°C. All modulus data were analyzed using Anton Parr software from Rheocompass.

Example 10-6. Acquisition of cryogenic scanning electron microscope (cryo-SEM) images

A Quanta 3D FEG (FEI, Netherlands) equipped with an Alto 2500 cryo-transfer system (Gatan, UK) was used for a cryo-SEM experiment. PNP and PCNP samples were rapidly immersed in a liquid nitrogen slush over 0.5 second. A freezer was emptied after a few seconds and the samples were transferred to a pre-emptied preparation chamber to $10^{-5}$ mbar at about - 190°C. Metal deposition was performed by plasma sputtering with a current of 3 mA for 60 seconds. The metal-coated samples were pre-evacuated to a pressure of $10^{-5}$ mbar and transferred to a microscope chamber pre-cooled to a temperature of - 190°C. Cryo-SEM images were acquired with a 5-keV beam at 11.8 pA.

Example 10-7. Dynamic Light Scattering (DLS)

PNP, CXB, and PCNP particle sizes were examined using a Zetasizer Nano ZS (Malvern Instruments Ltd., Malvern, UK). The nanoparticles were dissolved in phosphate buffered saline (Well Gene, KR) at a concentration of 0.1 wt% and the particle sizes were measured at 5, 25, 37, and 45°C for two cycles.

For zeta potential evaluation, 0.5 wt% of PNP and PCNP (4 mg of CXB) were prepared at room temperature. The measurement was performed using DLS (Malvern Instruments Ltd., Malvern, UK).

Example 10-8. In vitro and in vivo hydrogel degradation

To determine in vitro swelling and degradation, 200 μl of a 10 wt% PNP hydrogel was weighed in an insert well and incubated in an oven at 37°C for 5 min to induce complete gelation. Thereafter, hydrogel blocks were immersed in PBS at 37°C. The mass change of the hydrogel was measured at a determined time point.

To measure the swelling and degradation of PNP, PNP was dissolved in PBS at 10 wt% and stirred at 4°C. Next, 200 μl of a polymer solution was slowly injected subcutaneously into the back of each mouse (CrljOri:CD1 (ICR), 6 weeks

old, male, Orient Bio, Korea). The injected PNP was extracted and the residual amount was measured at a certain time point.

Example 10-9. In vitro release of CXB from PCNP

A PCNP hydrogel was prepared by stirring 10 wt% PNP with CXB. Then, PNCP (200 μl) was added into Millicell (diameter: 12 mm, Millipore, US) and placed in an oven at 37°C for 5 minutes. Thereafter, PCNP was immersed in a 15 mL conical tube containing 6 mL of PBS and incubated in a shaker (KMC-12-55W1, Vision, KR) at 37°C and 50 rpm. PBS was changed at a specific time point. The released CXB was measured using high-performance liquid chromatography (Agilent Technologies) with methanol (75%) and water (25%) as a mobile phase.

A kinetic model was used to fit experimental data. The model included zero-order model ( $\frac{m_t}{m_\infty} = k_0 t$ ), first-order model ( $\frac{m_t}{m_\infty} = 1 - \exp(-k_1 t)$ ), Higuchi model ( $\frac{m_t}{m_\infty} = k_H t^{1/2}$ ), and Ritger-Peppas model ( $\frac{m_t}{m_\infty} = k_P t^n$ ). The left side ( $\frac{m_t}{m_\infty}$ ) of each equation represents the percentage of drug release at time t. k and n represent the kinetic constant and diffusion exponent, respectively.

Example 10-10. In vivo tendon regeneration test

All experiments using mice were conducted in compliance with the relevant laws and institutional guidelines of the Institutional Animal Care and Use Committee (IACUC) of Korea University Medical Center and received IACUC approval (Approval No. KOREA-2018-0049). Collagenase (Thermo Fisher Inc., US, 50 μL) was injected into 9-week-old mice (DooYeol Biotec, Seoul, KR) and left for 1 week, and then injected with a CXB solution at a concentration of 1 or 4 mg/(100 μL) dissolved in DMSO to treat the defective tendon. PCNP (CXB 1 mg) and PCNP (CXB 4 mg) hydrogels were prepared by stirring 1 mg or 4 mg of CXB into 100 μL of the 10 wt% PNP hydrogels at 4°C. Then, the PCNP hydrogel was injected into the defective tendon model.

Example 10-11. Real-time polymerase chain reaction (RT-PCR)

Blood samples were collected 4 weeks after injection of a CXB solution or PCNP into the defective tendon. An RNA blood kit (QIAGEN, QIAamp) was used for RNA isolation. cDNA was synthesized using Accupower CycleScript RT premix (dT20) (BIO-RAD). Real-time polymerase chain reaction (RT-PCR) was performed using an ABI7300 real-time thermocycler (Applied Biosystems, USA), and pro-inflammatory cytokines COX-2, IL-1, IL-6, MMP-3, MMP-13, and TNF-α and anti-inflammatory cytokines IL-4 and IL-10 were tested. Genetic marker sequences were shown in Table 4 below.

[Table 4]

| Gene | Primer (forward) | Primer (reverse) |
|---|---|---|
| IL-4 | ACA GGA GAA GGG ACG CCA T | GAA GCC CTA CAG ACG AGC TCA |
| IL-10 | GGT TGC CAA GCC TTA TCG GA | ACC TGC TCC ACT GCC TTG CT |
| COX-2 | CAG CCA TAC TAT GCC TCG GA | GGA TGT CTT GCT CGT CGT TC |
| IL-1 | CCA CCT CCA GGG ACA GGA TA | AAC ACG CAG GAC AGG TAC AG |
| MMP-3 | ACC TGT CCC TCC AGA ACC TG | AAC TTC ATA TGC GGC ATC CA |
| MMP-13 | AAG GAG CAT GGC GAC TTC TA | GGT CCT TGG AGT GGT CAA GA |
| IL-6 | CCG TTT CTA CCT GGA GTT TG | GTT TGC CGA GTA GAC CTC AT |
| TNF-α | CTC CCA GAA AAG CAA GCA AC | CGA GCA GGA ATG AGA AGA GG |

Example 10-12. Histological processing and analysis

Defective tendons treated with a PCNP or CXB solution were extracted and washed with DPBS. Each tendon

was soaked and fixed in 4% paraformaldehyde for 24 hours, rinsed, and sectioned in paraffin (5 μm sections). Paraffin sections were placed in an oven at 60°C and deparaffinized in xylene containing 100%, 90%, 80%, and 70% ethanol. The sections were stained with H&E and MT. Each group was three times (n = 3).

Example 10-13. Hydroxyproline content analysis

To demonstrate the alleviation of AT by treatment of the PCNP and CXB solutions, the collagen content in mouse tendon tissue was assessed using hydroxyproline analysis. To hydrolyze the tendon tissue, 5 mg was immersed in a 6N HCl solution and then heated at 120°C for 3 hours. After hydrolysis, the tendon tissue was centrifuged at 3000 rpm for 3 minutes, and the supernatant was collected and transferred to a 96-well plate. Thereafter, the 96-well plate was incubated in a dry oven at 60°C for 12 hours. A Chloramine T reagent (6 μL) was mixed with an oxidation buffer (94 μL), and the mixture was added to each sample or a standard solution. The resulting solution was slowly transferred to a 96-well plate, and a p-dimethylaminobenzaldehyde reagent (100 μL) was added to each sample or standard, and then incubated at 60°C for 90 minutes. The hydroxyproline content was measured at an optical density of 450 nm using a Flash Multimode Reader (Varioskan™, Thermo Scientific, USA).

Example 10-14. Biomechanical test

To confirm the presence of AT, a biomechanical test was performed using selected calcaneal-Achilles tendons from rats. Each sample was loaded onto an Instron Mechanical Tester (AG-10KNX, Shimadzu, Japan) using a specially designed device and then pulled sagittally at a crosshead speed of 5 mm/min with a preload of 1 N. The tensile strength and stiffness of the Achilles tendon were measured.

Example 10-15. Statistical data analysis

All quantitative results are represented as mean value and standard deviation. The graph was drawn using Origin software. Statistical analysis was performed using Student's t-test and expressed as *P < 0.05, **P < 0.01.

**Example 11. Synthesis and characterization of PCNP hydrogels**

Thermo-sensitive PPZ nanoparticle (PNP) hydrogels were designed for in situ gelation and hydrophobic drug encapsulation. PNP was synthesized by substituting hydrophobic IleOEt and hydrophilic AMPEG into a PPZ main chain. The synthesized PNP structure was confirmed by $^1$H NMR spectroscopy and was shown in FIG. 23. Carboxylic acid groups, such as chondroitin sulfate and hyaluronic acid, were known to be replaced by negatively charged functional groups in the polymer backbone, mimicking natural ECM and inducing interactions with cytokines during tissue regeneration. The carboxylic acid group of PNP was quantified using $^1$H NMR. The peaks of 2.0 and 2.5 indicated that acidic groups were successfully conjugated to the PNP hydrogel backbone. A molecular weight of 62,920 Da was obtained for PNP using GPC, and polystyrene was used as a standard material (FIG. 23C). The ratio of substituted side chains was calculated using the $^1$H NMR spectrum, and the results indicated the presence of side chains with IleOEt, AMPEG, EtOH and carboxylic acid groups in the proportions of 71%, 17%, 1% and 11%, respectively. The high hydrophobic IleOEt content induced both hydrophobic gelation and close hydrophobic interactions with CXB. The FTIR spectra of PCNP, PNP, and CXB were analyzed to confirm the CXB loading of PCNP, which was shown in FIG. 24. The aromatic C=C peaks at 1530 to 1630 cm$^{-1}$ observed in the PCNP data indicated CXB loading.

Although CXB was generally used for medical use by dissolving in organic solvents or undergoing chemical modification, CXB was simply mixed in a solution state without any solvent or chemical modification to be successfully dissolved in PNP. The gel state was maintained by the thermo-sensitive gelation properties of PNP, which were shown in FIG. 25. PCNP was prepared by encapsulating CXB within PNP by stirring CXB in a 10 wt% PNP solution at 4°C. Temperature-dependent gelation of PCNPs was confirmed by injecting the polymer solution into water at 4°C and 37°C. Unlike the polymer solution at 4°C, when the 37°C group was injected into the aqueous solution, a gel-like 3D structure was observed, as shown in FIG. 26. This means that stable 3D structures may be formed without dispersion, as the sol-gel transition is likely to occur within several seconds after injection into the body. The storage and loss modulus of PNP and PCNP hydrogels were monitored at precisely controlled temperatures between 5 to 60°C to identify the temperature-dependent quantitative rheology of the gels. The sol-gel transition of the PNP and PCNP hydrogels occurred within a specific temperature range (20 to 25°C) and the gel state was maintained at a body temperature (37°C). The storage modulus began to increase with temperature, and the highest value was observed at approximately 35 to 38°C. This was shown in FIG. 27.

The storage and loss modulus of PCNP were affected by the CXB concentration (FIGS. 27B to 27D). The maximum storage modulus of the PNP hydrogel was 454.3 Pa at 35.1°C. However, the maximum storage moduli of PCNP

(CXB 1 mg) and PCNP (CXB 4 mg) hydrogels were 655.0 Pa at 38.1°C and 994.3 Pa at 37.6°C, respectively. No significant differences were observed in temperature and maximum storage modulus between the PNP and PCNP hydrogel groups. However, the maximum storage modulus value increased after incorporating CXB. It is suggested that the hydrophobic interactions between the PNP hydrogel and CXB molecules increase the modulus because the CXB acts as a hydrophobic cross-linker. These data indicate successful interactions between the PNP and the CXB molecules.

**Example 12. Complexation between CXB and PNP**

The hydrophobic CXB was found to be homogeneously dissolved and well distributed within the PNP solution. PCNP was formed after simple stirring at a low temperature, which corresponded to a clear advantage of this system. Toxic solvents or surfactants are usually required to improve the solubility of CXB in clinical trials due to its hydrophobicity. However, the fact that CXB may be readily incorporated into PNP at a low temperature without toxic solvents or surfactants serves as a significant advantage.

Amphiphilic PNP hydrogel networks were observed to be formed at specific concentrations and body temperatures via hydrophobic interactions between polymer chains. However, the amphiphilic property of PNP means that the polymer structure was self-assembled to form polymer micelle particles at a low concentration (0.5 wt%) via single-chain folding. The shapes of PNP and PCNP were characterized in both the gel and diluted states (FIG. 28A), and the results showed that the PNP and PCNP hydrogels exhibited porous structures with pore sizes of 6.2 and 4.9 $\mu$m, respectively. PCNP showed a rougher surface structure than PNP. However, no CXB aggregation was observed, which indicated that the hydrophobic CXB was well incorporated into a PCNP hydrogel matrix. After 20-fold dilution, micelle formation by self-assembly occurred in both PNP and PCNP, and the particle structures could be observed. A wide range of particle sizes were measured. However, the particles of the PCNP group were smaller than those of the PNP group. Therefore, the hydrogel and particle shapes of the PNP and PCNP hydrogels suggest that CXB was successfully encapsulated into PNP and the PCNP shape was influenced by the encapsulated CXB.

To further confirm the hydrophobic interaction between PNP and CXB, a change in the size of polymeric nanoparticles before and after mixing was investigated by adding different concentrations of CXBs (FIG. 28B). The experiment was performed under precise temperature conditions to observe a difference in the particle size due to hydrophobic interaction between PNP and CXB molecules. The particle size of PNP was observed to gradually decrease from 240.0 nm to 126.2 nm as the temperature increased from 4°C to 45°C. This phenomenon was a result of a correlation between the temperature and hydrophobicity. Hydrophobic moieties interact closely at a high temperature to produce polymeric nanoparticles in a contracted form. The sizes of PNP and PCNP particles were similar regardless of temperature. This indicates that hydrogel networks have stable thermo-sensitive and reversible physical properties due to non-covalent interactions, which provides various advantages including simple drug loading and injectability. After CXB was incorporated into PNP, the particle size decreased as the CXB concentration increased at all temperatures. It is meant that the CXB molecules inside PNP act as hydrophobic cross-linkers to induce PCNP to form a more contracted structure. Therefore, the interaction between the CXB and PNP hydrogel means that CXB may be encapsulated into PNP at various concentrations without surfactants, which may positively improve a therapeutic effect and reduce the side effects associated with this strategy.

A zeta potential was examined along with the particle size and was shown in FIG. 28C. The zeta potential of PNP was measured as - 19.9 mV due to the carboxyl group of the PNP backbone. However, the zeta potential significantly decreased to - 13.1 and -9.5 mV for PCNP (CXB 1 mg) and PCNP (CXB 4 mg), respectively, after CXB was included in PNP. This is because hydrophobic CXB, which has a smaller particle size than PNP, is encapsulated in the core of the PCNP micelle structure (FIG. 28B). This means that hydrophilic PEG on the PCNP surface covers a negative charge, and thus the hydrophobic interaction formed PCNP with small pores and particle size by successfully binding CXB to PNP.

**Example 13. In vitro and in vivo PCNP hydrogel mass loss and sustained release of CXB from hydrogels**

To predict the retention requirements of the PCNP hydrogel after body injection, the mass loss of the hydrogel was measured and the release of CXB from the hydrogel was analyzed to determine whether the PCNP hydrogel could retain and release CXB in the long term, which was shown in FIG. 29. In vitro degradation data showed swelling and degradation patterns in an aqueous environment. The PNP hydrogel swelled to 147% in 3 days and then was slowly dissolved over 5 weeks (FIG. 30). As shown in FIG. 29A, the CXB release rate from the PCNP hydrogel was almost constantly maintained for 29 days even when high swelling occurred due to the stable interaction between the PNP hydrogel and CXB. The CXB release rate was higher in the group with 1 mg CXB, which may be because the increased hydrophobic interactions between CXB and PNP with higher CXB concentrations would induce a more compact structure.

PCNP was biodegraded through hydrolysis and CXB was released over 28 days. The observed correlation between the degradation and release profiles indicates that CXB release is associated with the degradation of the PCNP hydrogel. In addition, the PCNP swelling observed early in the experiment (to 7 days) did not lead to a burst release of CXB,

which indicated that the release was related to dissolution of the PCNP hydrogel rather than swelling.

CXB release data were analyzed according to various kinetic models, including zero-order, first-order, Higuchi, and Ritger-Peppas models (Table 5 below). Comparison of correlation coefficients (R2) indicates that the Ritger-Peppas model is the best suitable for both PCNP (CXB 1 mg) and PCNP (CXB 4 mg). When using the Ritger-Peppas model, mixed release mechanisms including swelling, erosion, and polymer porosity may be involved. This kinetic model is suitable to be used with PCNP because CXB is released by swelling and degradation of PCNP. In addition, a diffusion mechanism may be explained by a diffusion index (n). The diffusion indices (n) of PCNP (CXB 1 mg) and PCNP (CXB 4 mg), calculated as 0.62 and 0.57, respectively, both indicate non-Fickian diffusion.

[Table 5]

| Empty Cell | Zero-order | | First-order | | Higuchi | | Ritger and Peppas | | | Empty Cell |
|---|---|---|---|---|---|---|---|---|---|---|
| Empty Cell | $K_0$ | $R^2$ | $K_1$ | $R^2$ | $k_H$ | $R^2$ | n | $K_P$ | $R^2$ | Release mechanism |
| PCNP(1 mg) | 0.38 | 0.90 | 0.08 | 0.97 | 17 | 0.97 | 0.62 | 11.56 | 0.98 | non-Fickian |
| PCNP(4 mg) | 2.48 | 0.86 | 0.04 | 0.98 | 12.6 | 0.98 | 0.57 | 10.25 | 0.98 | non-Fickian |

To measure an in vivo biodegradation rate, the PNP hydrogel was injected subcutaneously into the back of mice. The thermo-sensitive gelation property of the PNP hydrogel means that gelation occurs rapidly at body temperature, unlike the dispersion of the polymer solution observed at 4°C (FIG. 26). In addition, the PNP hydrogel was shown to maintain a stable modulus at an angular frequency in the range of 1 to 100 rad/s at 37°C (FIG. 31). This indicates that the PNP hydrogel is suitable for injection and maintains the gel structure in vivo. The mass of the injected PNP hydrogel increased to 111% on day 2 in an in vivo environment and then decreased continuously for more than 28 days (FIG. 29B). This shows that the in vivo PNP hydrogel began to gradually degrade after the initial water uptake into the PNP hydrogel network. FIG. 32 shows the gradual reduction in the size of the PNP hydrogel in vivo. These results show that CXB was slowly released after in vivo PCNP hydrogel injection and the PCNP hydrogel remained at the injection site for about 1 month.

Example 14. **Pro-inflammatory cytokine inhibition and anti-inflammatory effects of PCNP hydrogel on defective tendon**

Before an in vivo experiment, the in vitro cytotoxicity of the PNP hydrogel was examined to identify side effects (FIG. 33A). Cell viability was maintained above 70% even at a high concentration of PNP (10 mg mL-1). Mass loss data from the in vivo experiment suggest that 200 μg of the injected PNP hydrogel was degraded within 30 days, which indicated that approximately 6.6 μg of PNP was released a day from the injected hydrogel structure (FIG. 29). Therefore, the PNP hydrogel was confirmed as a biocompatible drug carrier without side effects, and also, the PNP hydrogel is degraded into non-toxic substances such as phosphate, poly(ethylene glycol), and ammonia through hydrolysis.

The cytotoxicity of CXB and PCNP was also confirmed and shown in FIGS. 33B and 33C. The CXB group showed high cytotoxicity at 625 μg/ml, whereas no significant cytotoxicity was observed in the PCNP group at 40,000 μg/ml CXB. This is because the low solubility of CXB causes aggregation, which is because the cells are exposed to a high concentration of a drug with a high cytotoxicity level. PNP may be considered a carrier suitable for CXB because the PNP may deliver drugs without aggregation or cytotoxicity.

The PCNP may effectively encapsulate various CXB doses to be useful for controlling an appropriate therapeutic dose to successfully treat many inflammatory diseases. In the present disclosure, the AT therapeutic effect was evaluated using PCNP (CXB 1 mg) and PCNP (CXB 4 mg), which were doses used as an injection.

CXB reduces the inflammation and pain by increasing the expression of anti-inflammatory cytokines such as IL-4 and IL-10. The CXB treatment has also been shown to reduce the expression of pro-inflammatory cytokines TNF-α, IL-1, IL-6, COX-2, MMP-3, and MMP-13 in cartilage and synovium. These anti-inflammatory and proinflammatory cytokines are directly related to tendon healing. The pro-inflammatory cytokines affect ECM homeostasis and tendon remodeling, but may promote tendon cell apoptosis. IL-4 increased the proliferation of tenocytes involved in tendon healing, whereas IL-10 influences cell proliferation and survival via a STAT3 signaling pathway.

To analyze the proinflammatory cytokine inhibition and anti-inflammatory effects of the developed delivery system, the PCNP hydrogel was injected into a defective Achilles tendon model and the levels of anti-inflammatory and pro-inflammatory cytokines were measured using RT-PCR (FIG. 34). The levels of anti-inflammatory cytokines IL-4 and IL-10 in the blood were measured after 4 weeks of treatment (FIGS. 34A and 34B). Compared with a control group, IL-4 expression increased 1.2-fold in a group treated with CXB once and 1.3-fold in a group treated with CXB three times. Compared with the control group, IL-4 expression increased 1.5-fold and 1.7-fold in the PCNP (1 mg and 4 mg CXB) injection groups, respectively, and IL-10 showed a similar pattern of increasing 1.3-fold and 1.5-fold, respectively. The PCNP (1 and 4 mg CXB)-groups showed 1.8-fold and 2.2-fold increases, respectively. Compared with the control group,

there was no significant change in the expression of anti-inflammatory cytokines in a non-treated group, which suggested that the anti-inflammatory effect did not occur without treatment. The gene expression levels of anti-inflammatory cytokines were higher in a CXB solution-treated group, and the expression of anti-inflammatory cytokines was higher after injection of the PCNP hydrogel. It was shown that the CXB injection induced an anti-inflammatory effect, and the PCNP injection further enhanced the anti-inflammatory effect due to local and sustained release of CXB in the defective Achilles tendon.

The expression of proinflammatory cytokines COX-2, IL-1, IL-6, MMP-3, MMP13, and TNF-$\alpha$ was also investigated and shown in FIGS. 34C to 34H. Severe inflammation in the non-treated group increased 7.6-, 6.4-, 7.1-, 7.1-, 8.1-, and 7.8-fold in COX-2, IL-1, IL-6, MMP-3, MMP13, and TNF-$\alpha$ expression, respectively, compared with the control group. The expression of pro-inflammatory cytokines COX-2, IL-1, IL-6, MMP-3, MMP-13, and TNF-$\alpha$ was increased 6.3-, 5.4-, 6.0-, 5.9-, 7.2-, and 6.7-fold, respectively, in the CXB solution compared to the control group, which was significantly lower than that observed in the non-treated group. In the PCNP (CXB 4 mg) hydrogel injection group, pro-inflammatory cytokine levels increased 5.6-fold, 4.7-fold, 5.2-fold, 5.1-fold, 6.6-fold, and 5.8-fold compared to the control group. All the PCNP injection groups showed lower expression levels of most pro-inflammatory cytokines than the CXB solution injection group. Pro- and anti-inflammatory cytokine expression data indicate that a single local injection of the PCNP hydrogel exhibits a higher anti-inflammatory effect than multiple injections of the CXB solution, which was again evaluated because CXB was slowly released into the defective area.

**Example 15. In vivo regeneration of defective Achilles tendon**

To analyze an in vivo tendon regeneration effect by local sustained release of CXB from the PCNP hydrogel, defective Achilles tendons were generated using collagenase, and the same concentration groups were used to investigate the regeneration of defective Achilles tendon (FIG. 35). Hematoxylin and eosin (H&E) and Masson's trichrome (MT) staining data showed destruction of ECM alignment and collagen degradation in a collagenase 50 group, which indicated that the tendon was completely damaged by collagenase (FIGS. 35B and 35H). The damaged tendons were partially regenerated in the CXB solution injection group after 4 weeks (FIGS. 35C and 35D). MT staining data clearly showed that more collagen was regenerated in the group administered with the CXB solution three times than in the group administered with only one CXB injection (FIGS. 35I and 35J). It is shown that the time when the defective tendon is exposed to CXB has a positive effect on tendon regeneration. H&E staining results showed that the tendons in the PCNP hydrogel-treated group were almost completely recovered with tissue alignment (FIGS. 35E and 35F). MT staining results showed that the PCNP hydrogel group exhibited enhanced collagen regeneration compared to the CXB solution injection group. The CXB concentration in the PCNP hydrogel also affected the tendon regeneration (FIGS. 35E to 35L). These results suggest that continuous CXB exposure of the defective tendon is required to induce long-term regeneration of the defective tendon.

In vivo regeneration and functional recovery of the tendon component were examined using hydroxyproline analysis, mechanical stiffness and strength tests (FIG. 36). Collagen, one of the main components of the tendon, provides stiff mechanical properties. The collagen mainly consists of hydroxyproline. Therefore, the amount of the substance present in the tissue represents the amount of collagen. 35.5 $\mu$g/mg of the hydroxyproline content of the normal tendon tissue was significantly reduced to 11.3 $\mu$g/mg in the non-treated group (FIG. 36A). The increased hydroxyproline content was observed in the CXB solution groups (injected once and three times per week) with values of 15.3 and 17.2 $\mu$g/mg, respectively, and a further increase in the hydroxyproline content was observed in the PCNP hydrogel injection groups (CXB 1 mg and 4 mg), and the content reached values of 21.4 and 24.4 $\mu$g/mg, respectively. The number of CXB solution injections and the CXB concentration of the PCNP hydrogel proportionally affected the hydroxyproline content. The PCNP hydrogel provided a longer CXB exposure time to Achilles tendon defects than the CXB solution, and various concentrations of CXB encapsulation could directly affect the therapeutic effect.

The stiffness and tensile strength of the treated tendons were measured and mechanical and functional regeneration was evaluated compared with normal tendons (FIGS. 36B and 36C). In the non-treated group, the stiffness of normal tendon significantly decreased from 36.3 N/mm to 13.7 N/mm. The groups treated with the CXB solution (injected once and three times per week) showed higher stiffness values (16.4 and 18.6 N/mm), whereas the stiffness increased to 24.5 and 26.4 N/mm in the PCNP hydrogel-treated groups (CXB 1 and 4 mg), which were similar to the values obtained for the normal tendons. The CXB solution-treated group showed improved tendon regeneration ability, but this effect was much more prominent in the PCNP hydrogel-treated group. The tensile strength data showed a similar pattern to the stiffness results, as the tensile strength of the normal tendon of 33.7 MPa decreased to 11.4 MPa in the non-treated group. The tensile strength was observed to be 12.5 and 14.7 MPa in the CXB solution treated groups (injected once and three times per week), respectively, and the PCNP hydrogel treatment groups (CXB 1 and 4 mg) showed a recovery of approximately 66% and 71% of the normal Achilles tendon tissue, respectively. This indicates that the PCNP hydrogel induced both aligned tendon ECM regeneration and restoration of tendon mechanical functions.

While the embodiments are described with reference to drawings, it will be apparent to one of ordinary skill in the

art that various alterations and modifications in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents.

Therefore, other implementations, other embodiments, and equivalents to the claims are also within the scope of the following claims.

The present disclosure was completed with the support of the projects of Development of Intelligent Solution Technology for Disaster Safety (Project No. 2E31600, Project Period: 20220101 to 20221231), Development of Local Drug Delivery Platform Technology for Treating General Musculoskeletal Diseases (Project Unique No. 1711056093, Project Period: 20170401 to 20171231), and Development of Hydrogel Engineering Technology for Reconstructing Neural Networks (Project Unique No. 1711134231, Project Period: 20210701 to 20211231).

## Claims

**1.** A composition for preventing or treating inflammatory diseases comprising:

a polyphosphazene polymer comprising, on a phosphorus atom of a polyphosphazene backbone represented by the following Chemical Formula 1:

an amino acid ester first moiety represented by the following Chemical Formula 2;
a second moiety of polyethylene glycol represented by the following Chemical Formula 3; and
a third moiety comprising aminoethanol and an organic acid, each in a molar ratio of a : b : c; and

a therapeutically effective amount of a drug for controlling and treating inflammation,

[Chemical Formula 1]

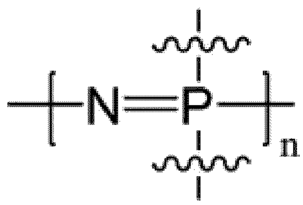

[Chemical Formula 2]

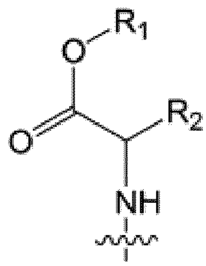

[Chemical Formula 3]

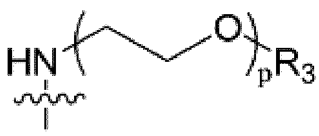

wherein,

$R_1$ is $C_{1-6}$ alkyl, $C_{1-6}$ alkenyl, or $C_{6-10}$ aryl-$C_{1-6}$ alkyl;
$R_2$ is hydrogen, methyl, isopropyl, 1-methylpropyl, 2-methylpropyl, thiomethyl, methylthioethyl, benzyl,

hydroxybenzyl or 2-indolylmethyl;
$R_3$ is $C_{1-6}$ alkyl;
n is an integer of 3 to 100,000,
p is an integer of 1 to 20,
a molar ratio of a : b is 5.5 : 1 to 7.5 : 1, and
a is 70 to 80 mol%.

2. The composition for preventing or treating inflammatory diseases of claim 1, wherein the organic acid in the third moiety comprises glutaric acid.

3. The composition for preventing or treating inflammatory diseases of claim 1, wherein the $R_1$ is methyl, ethyl, propyl, butyl, benzyl or 2-propenyl.

4. The composition for preventing or treating inflammatory diseases of claim 1, wherein the $R_3$ is methyl.

5. The composition for preventing or treating inflammatory diseases of claim 1, wherein the drug for treating the inflammatory diseases is hydrophobic.

6. The composition for preventing or treating inflammatory diseases of claim 5, wherein the hydrophobic drug for treating the inflammatory diseases is at least one selected from the group consisting of triamcinolone acetonide, methylprednisolone, dexamethasone, celecoxib, ibuprofen, naproxen, indomethacin, ketoprofen, etodolac, meloxicam, rofecoxib, etoricoxib, valdecoxib, lumiracoxib, and diclofenac.

7. The composition for preventing or treating inflammatory diseases of claim 1, wherein the polymer slowly releases the drug for treating the inflammatory diseases.

8. The composition for preventing or treating inflammatory diseases of claim 1, wherein the slowly releasing is releasing the drug at a drug concentration in a therapeutically effective range.

9. The composition for preventing or treating inflammatory diseases of claim 1, wherein the polyphosphazene polymer is dissolved in a solvent at a concentration of 1 to 50% by weight (wt%).

10. The composition for preventing or treating inflammatory diseases of claim 9, wherein the solvent is at least one selected from the group consisting of water, a buffer solution, an acid solution, a basic solution, a salt solution, a saline solution, water for injection, a cell culture solution, and a glucose saline solution.

11. The composition for preventing or treating inflammatory diseases of claim 1, wherein the polymer exhibits sol-gel transition behavior in the range of 5 to 70°C and forms a hydrogel at a predetermined temperature.

12. The composition for preventing or treating inflammatory diseases of claim 1, wherein the polymer gels when applied to a living body or in an in vitro environment, but loses a thermo-sensitivity at a predetermined temperature and maintains a gel state regardless of a change in temperature.

13. The composition for preventing or treating inflammatory diseases of claim 1, wherein the inflammatory disease is any one selected from the group consisting of osteoarthritis, degenerative arthritis, rheumatoid arthritis, osteoporosis, and Achilles tendonitis.

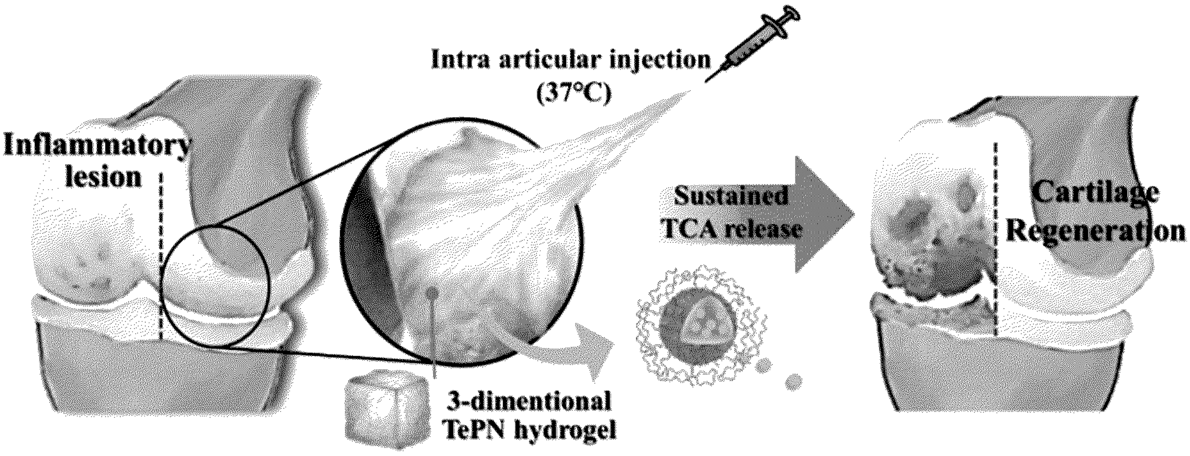
Intra articular injection
(37°C)
Inflammatory
lesion
Sustained
TCA release
Cartilage
Regeneration
3-dimentional
TePN hydrogel

FIG. 1

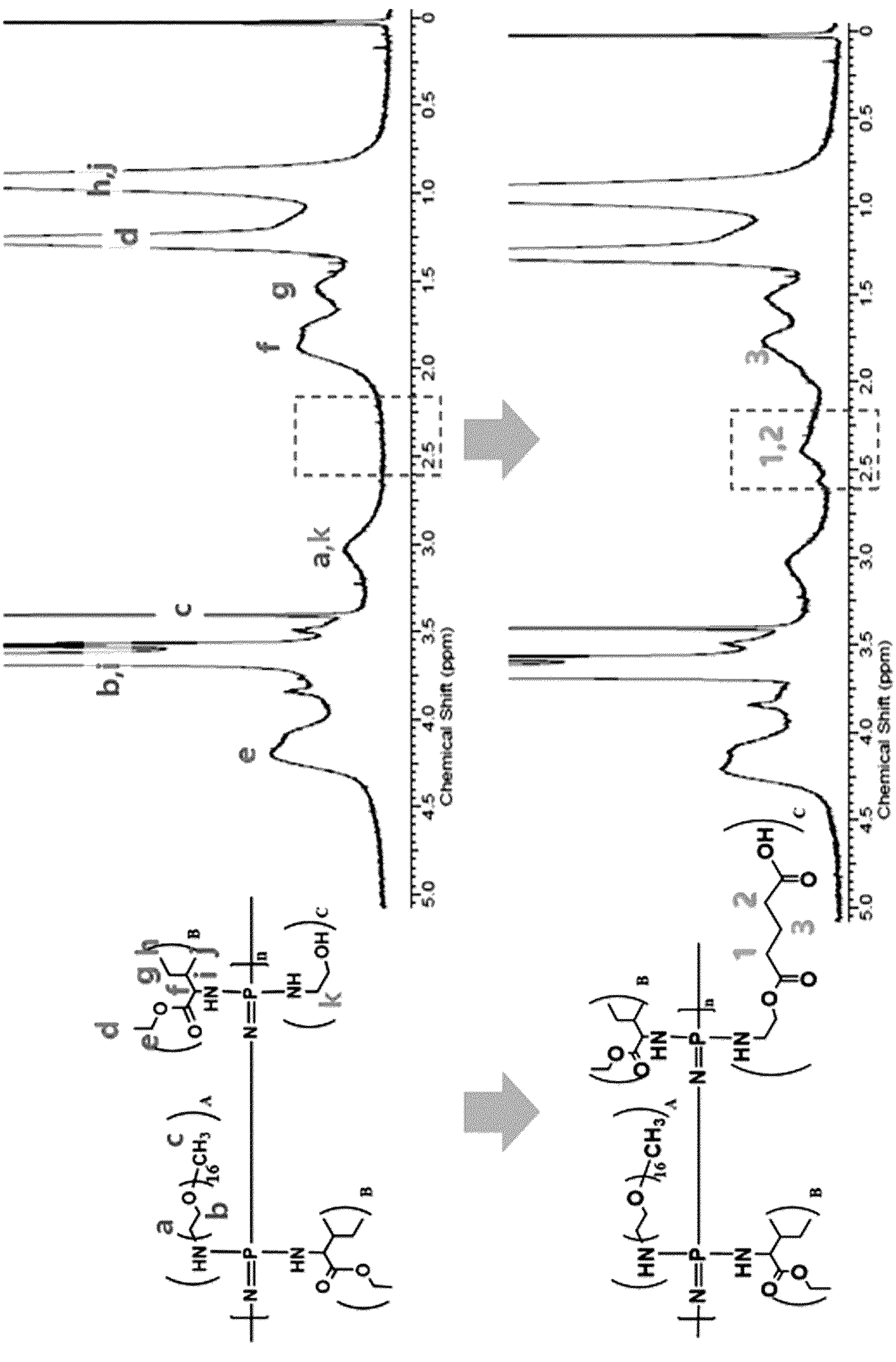


FIG. 2

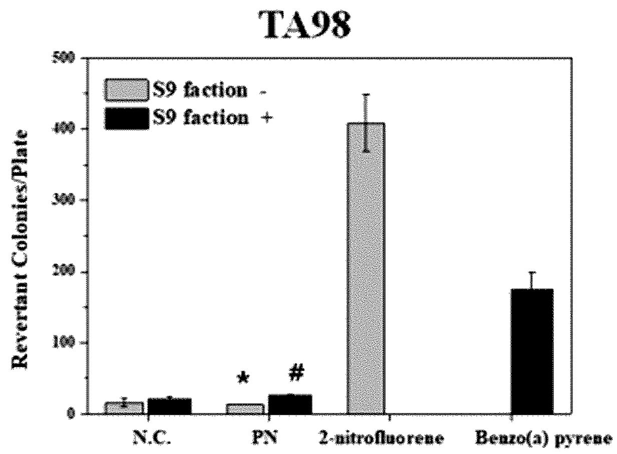


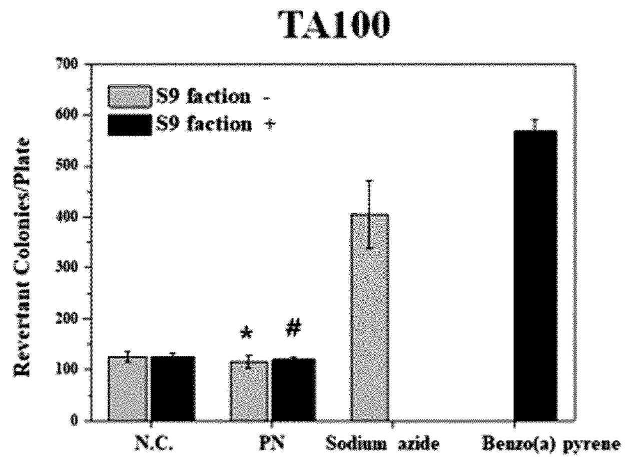


| TA98 | Revertant Colonies/Plate | | | |
|---|---|---|---|---|
| | N.C. | PN | 2-nitrofluorene | Benzo(a) pyrene |
| S9 - | 16 ± 6 | 13 ± 0 | 408 ± 40 | ND |
| S9 + | 21 ± 4 | 26 ± 1 | ND | 175 ± 25 |

| TA100 | Revertant Colonies/Plate | | | |
|---|---|---|---|---|
| | N.C. | PN | Sodium azide | Benzo(a) pyrene |
| S9 - | 126 ± 10 | 115 ± 12 | 404 ± 67 | ND |
| S9 + | 125 ± 9 | 120 ± 5 | ND | 568 ± 23 |

FIG. 3A

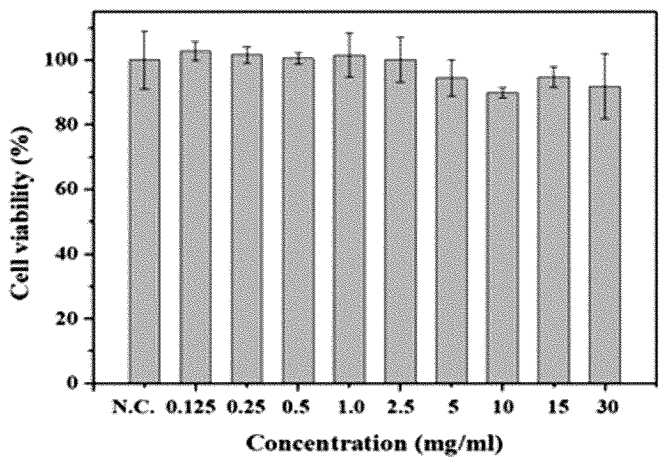


FIG. 3B

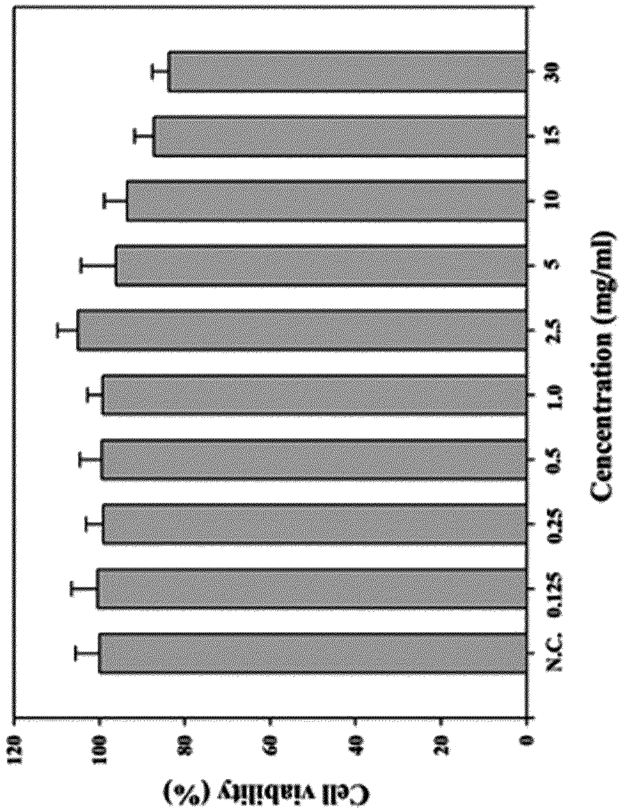
Cell viability (%)
0
20
40
60
80
100
120
N.C.
0.125
0.25
0.5
1.0
2.5
5
10
15
30
Cencentration (mg/ml)

FIG. 4A

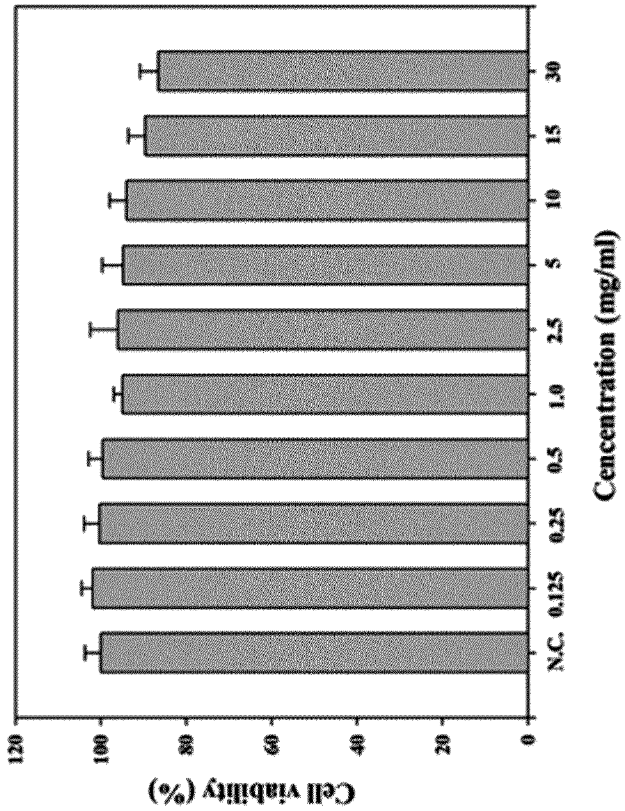
Cell viability (%)
0
20
40
60
80
100
120
N.C.
0.125
0.25
0.5
1.0
2.5
5
10
15
30
Cencentration (mg/ml)

FIG. 4B

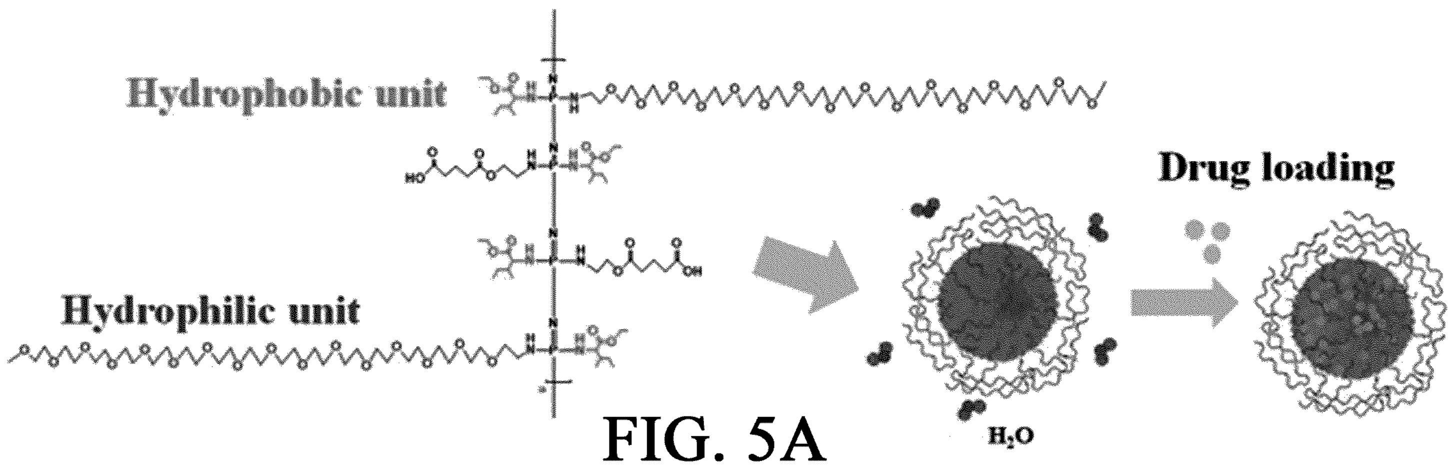


FIG. 5A

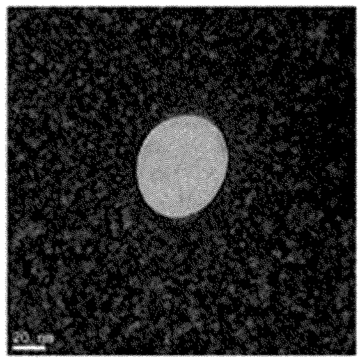

FIG. 5B

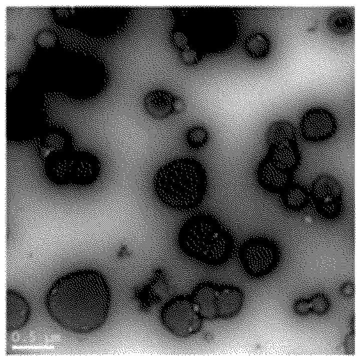

FIG. 5C

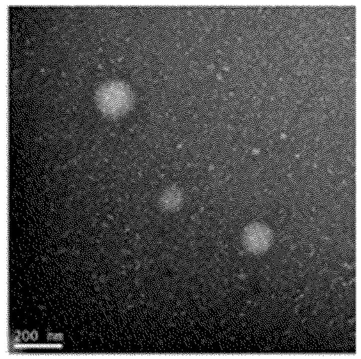

FIG. 5D

| | PN | TCA | | | TePN | | |
|---|---|---|---|---|---|---|---|
| Composition | A | B | C | D | A+B | A+C | A+D |
| Concentration | 120 mg/ml | 0.3 mg/ml | 1.0 mg/ml | 1.5 mg/ml | | | |
| Size (nm) | 140.1 ± 3.8 | 313.9 ±99.9 | 346.1 ± 40.8 | 282.3 ± 81.8 | 139.5 ± 2.8 | 135.1 ± 6.4 | 140.1 ±3.6 |
| PDI | 0.29 | 1 | 1 | 1 | 0.3 | 0.29 | 0.27 |

FIG. 5E

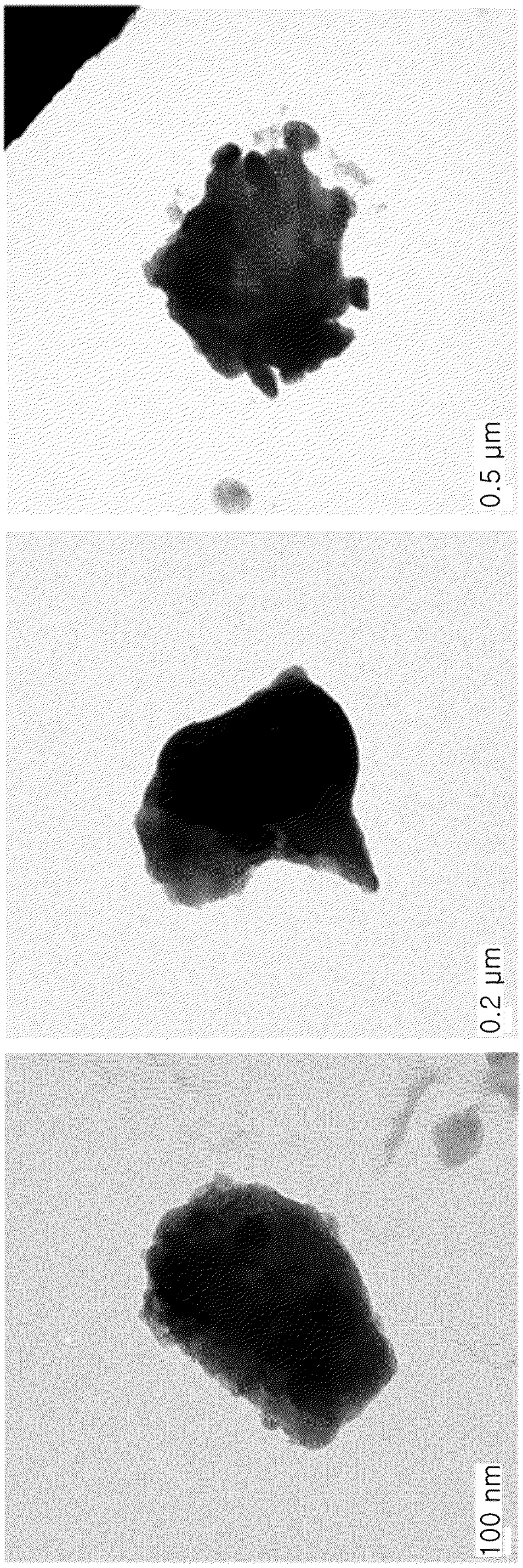


FIG. 6

FIG. 7

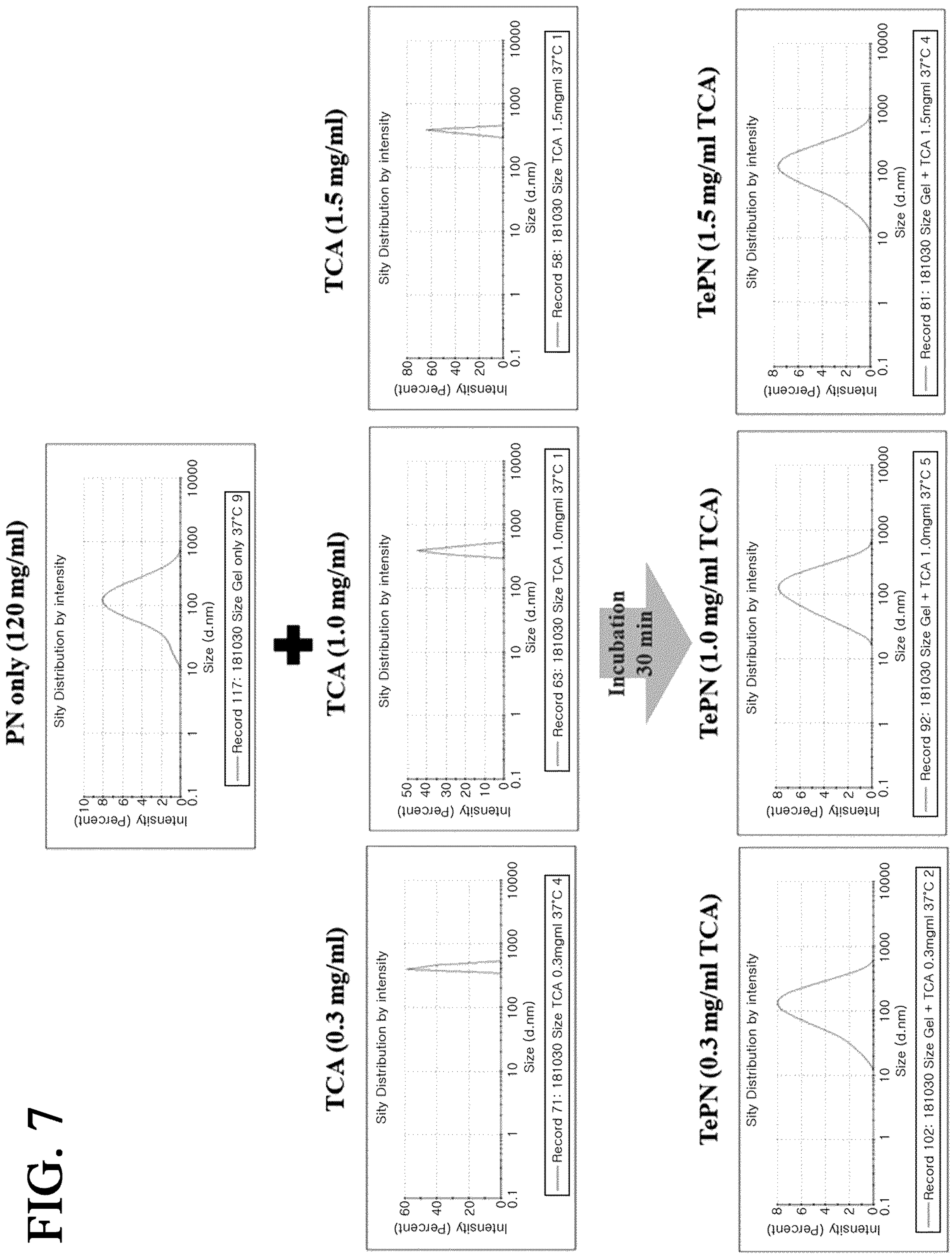
PN only (120 mg/ml)
Sity Distribution by intensity
Intensity (Percent)
Size (d.nm)
Record 117: 181030 Size Gel only 37°C 9
TCA (0.3 mg/ml)
Record 71: 181030 Size TCA 0.3mgml 37°C 4
TCA (1.0 mg/ml)
Record 63: 181030 Size TCA 1.0mgml 37°C 1
TCA (1.5 mg/ml)
Record 58: 181030 Size TCA 1.5mgml 37°C 1
Incubation 30 min
TePN (0.3 mg/ml TCA)
Record 102: 181030 Size Gel + TCA 0.3mgml 37°C 2
TePN (1.0 mg/ml TCA)
Record 92: 181030 Size Gel + TCA 1.0mgml 37°C 5
TePN (1.5 mg/ml TCA)
Record 81: 181030 Size Gel + TCA 1.5mgml 37°C 4

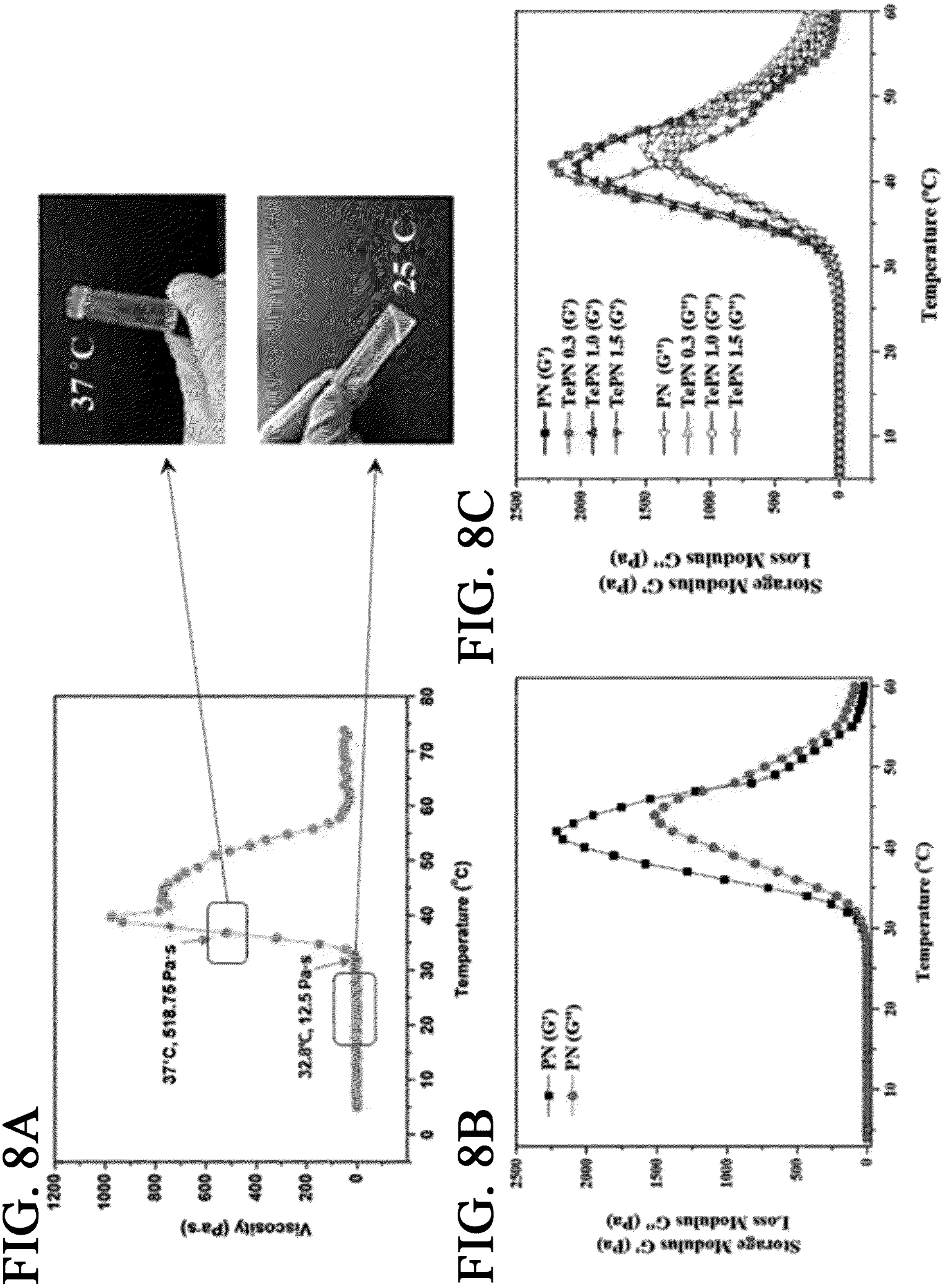


FIG. 8A

FIG. 8B

FIG. 8C

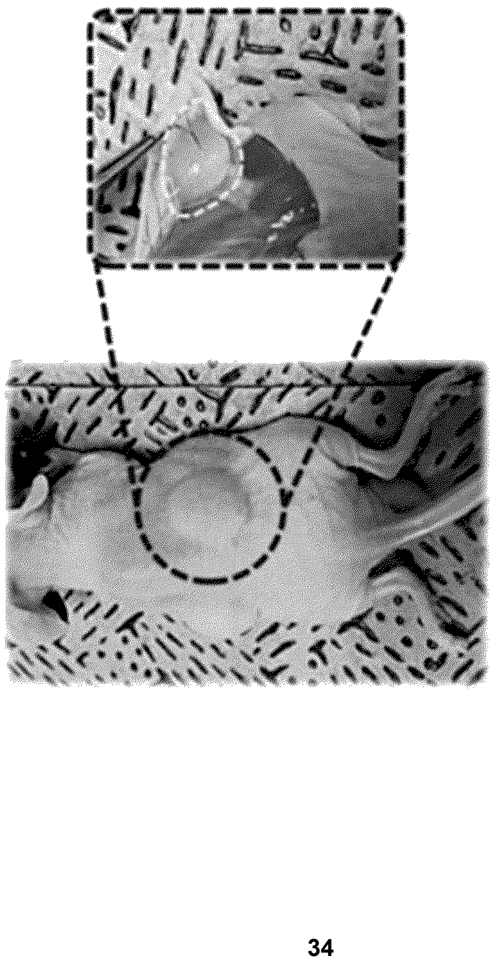

FIG. 9A

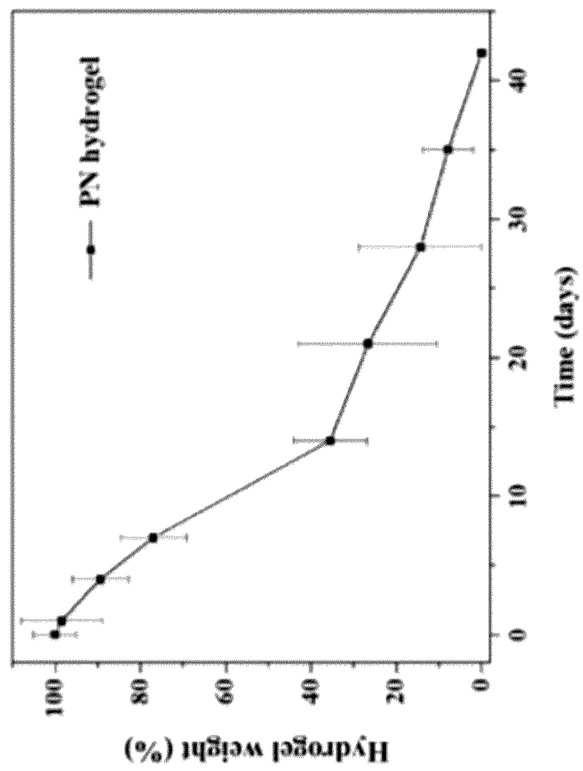
PN hydrogel
Hydrogel weight (%)
Time (days)
0
20
40
60
80
100
0
10
20
30
40

FIG. 9B

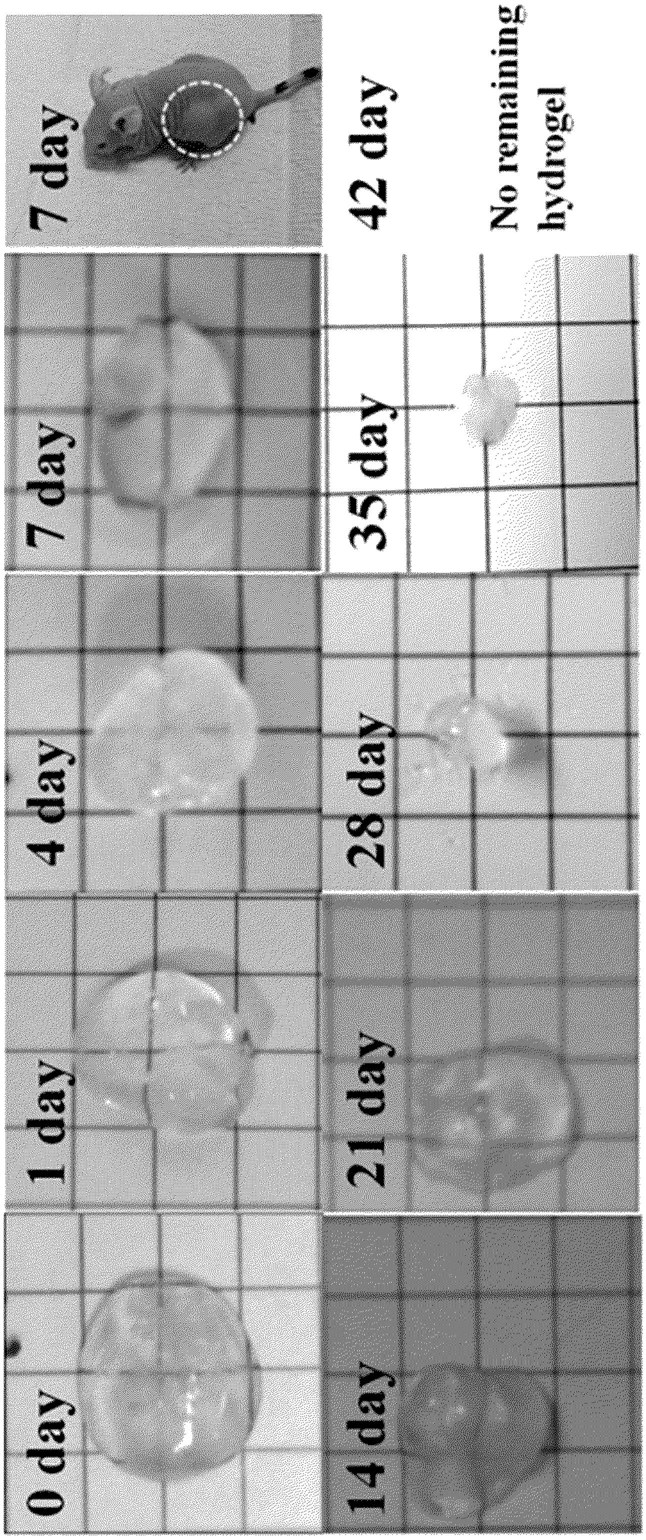
0 day
1 day
4 day
7 day
7 day
14 day
21 day
28 day
35 day
42 day
No remaining hydrogel

FIG. 10

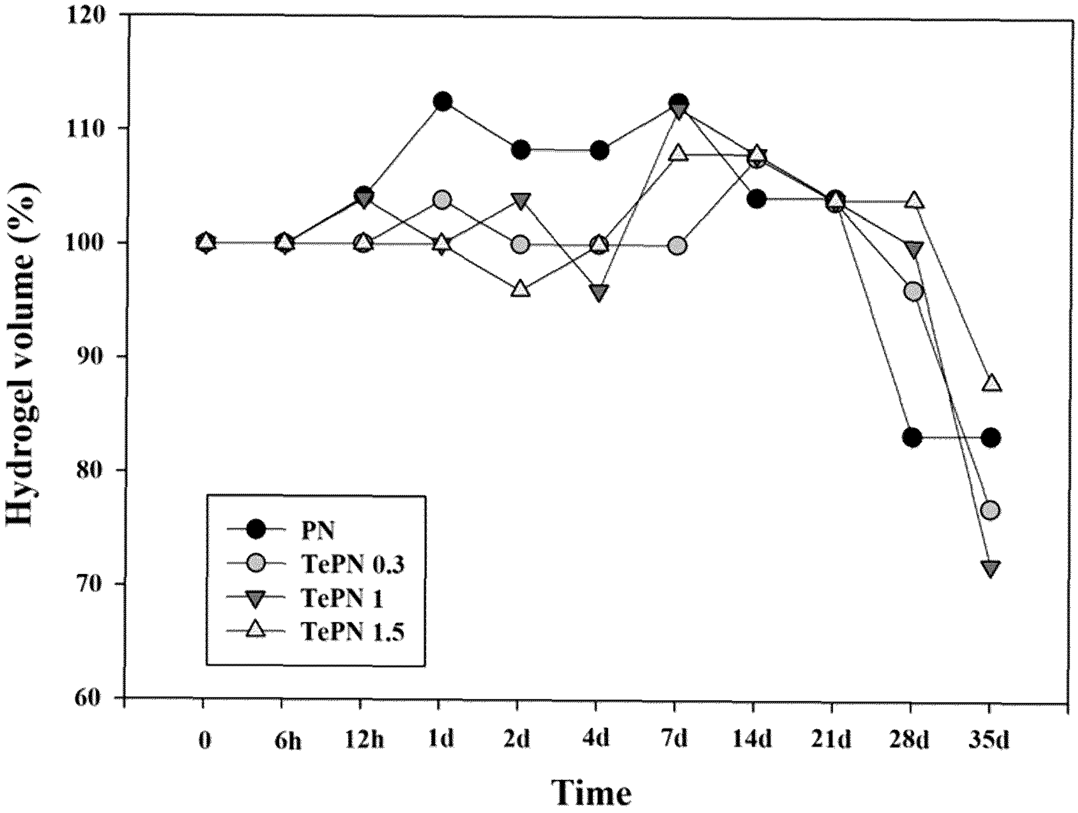
120
110
100
90
80
70
60
Hydrogel volume (%)
PN
TePN 0.3
TePN 1
TePN 1.5
0
6h
12h
1d
2d
4d
7d
14d
21d
28d
35d
Time

FIG. 11

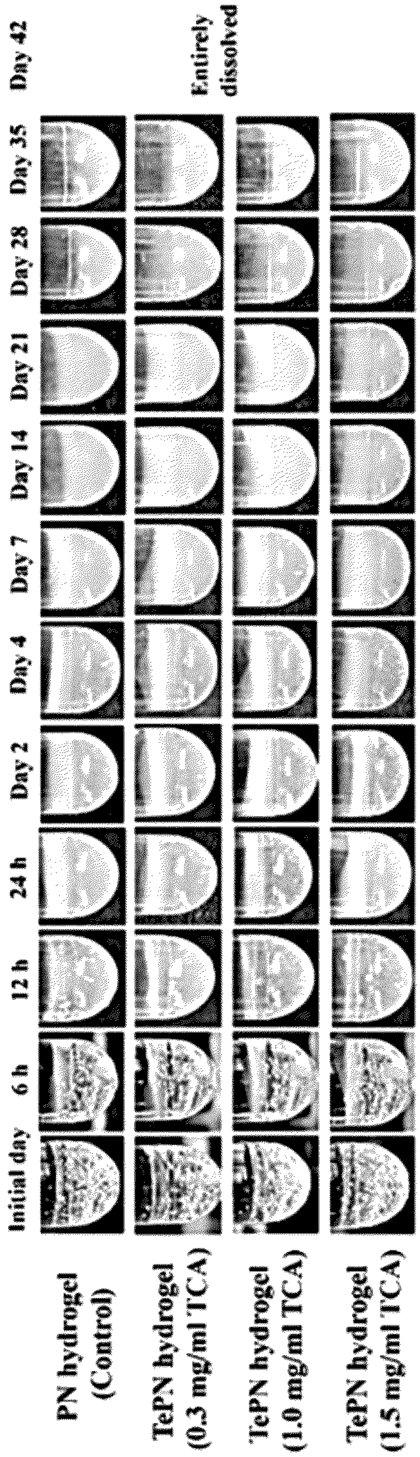


FIG. 12A

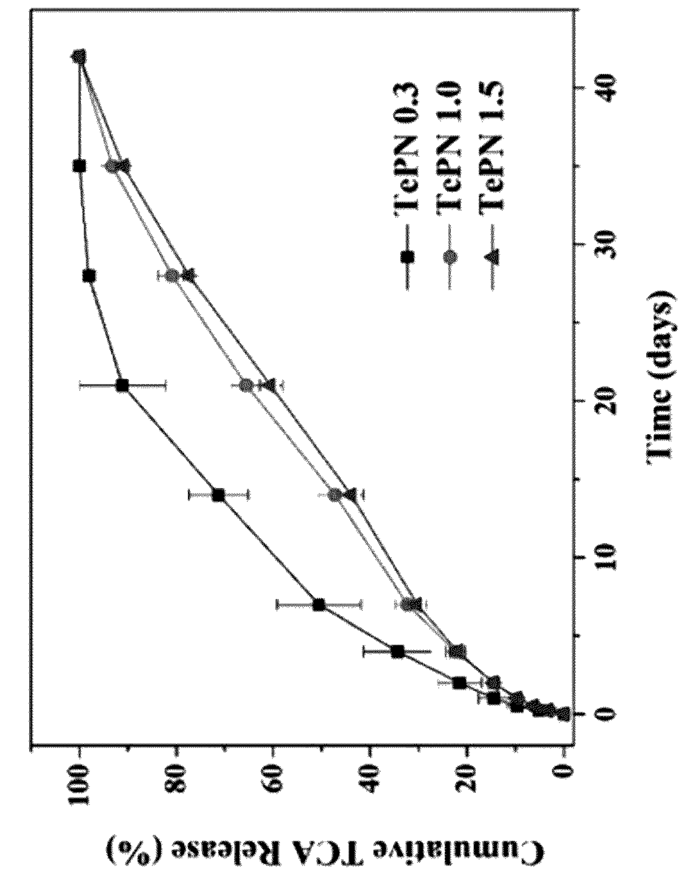


FIG. 12B

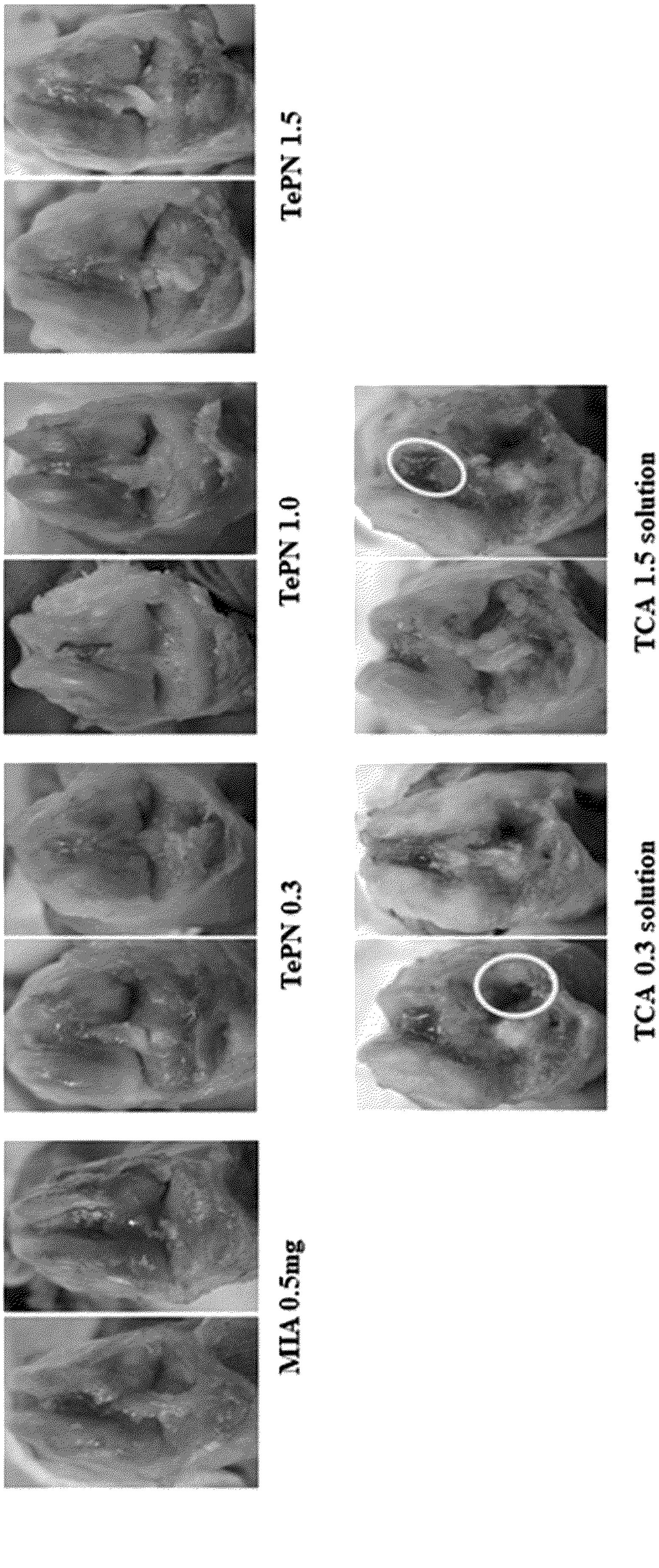
TePN 1.5
TePN 1.0
TePN 0.3
MIA 0.5mg
TCA 1.5 solution
TCA 0.3 solution

FIG. 13

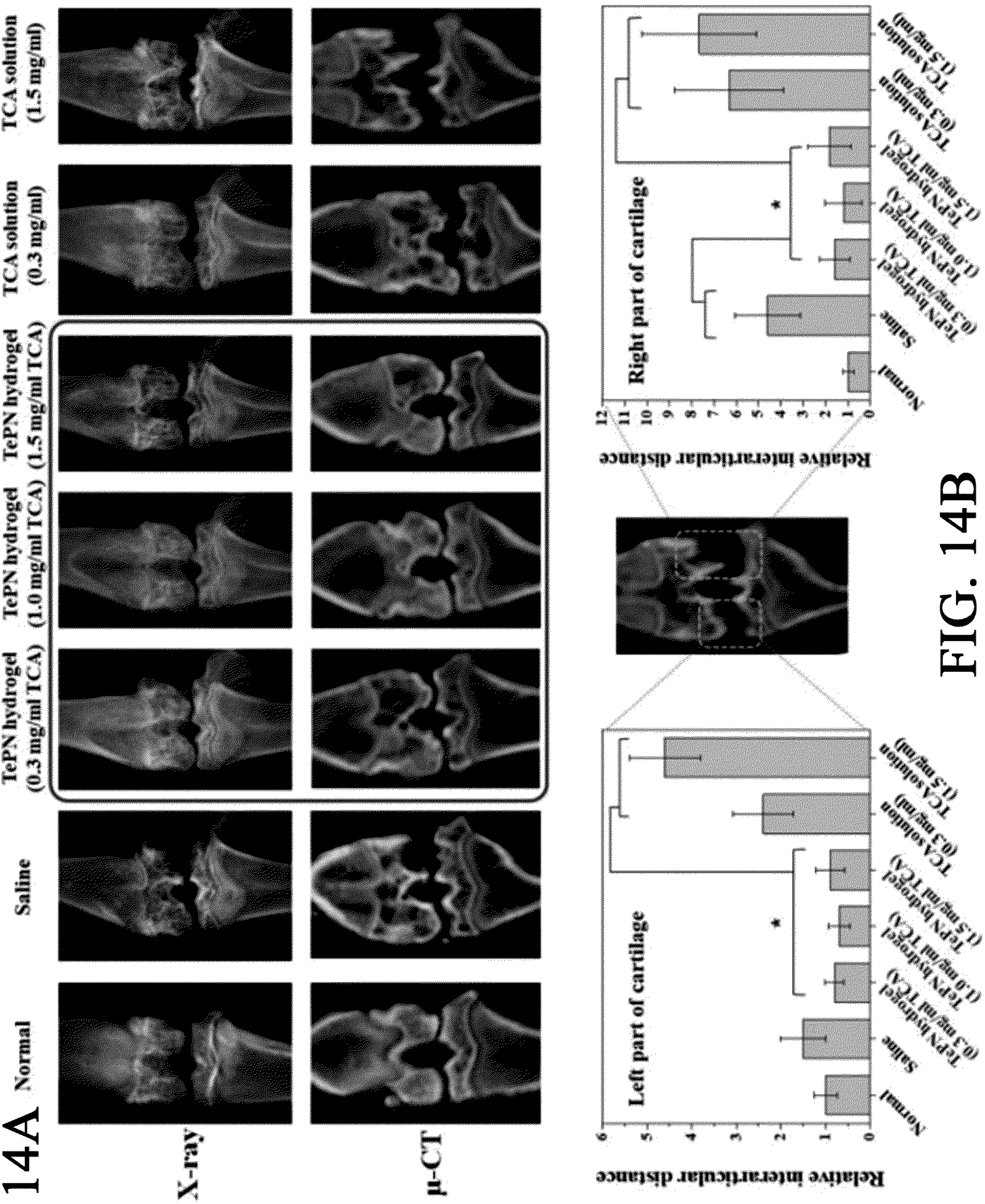


FIG. 14A

FIG. 14B

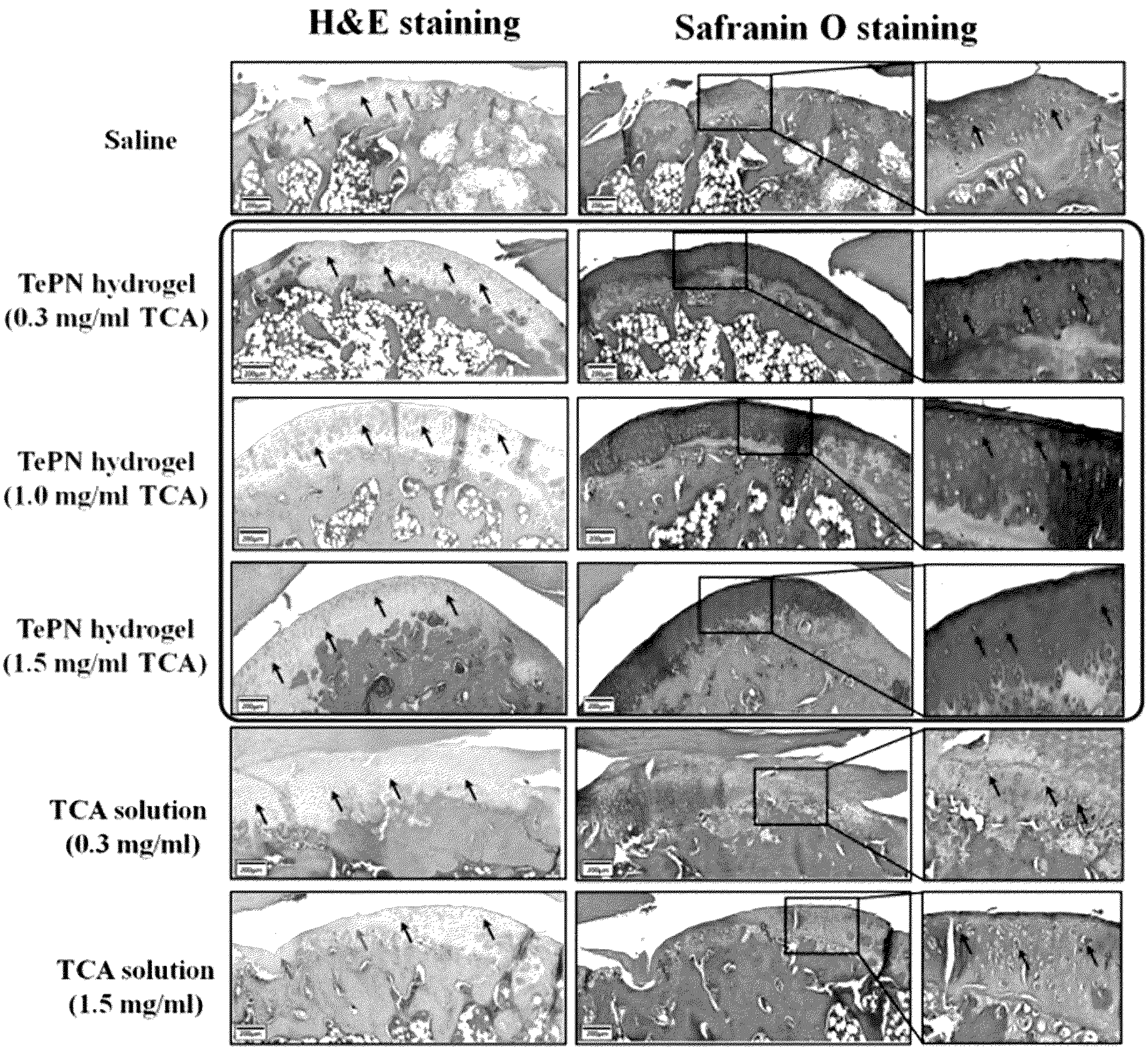
H&E staining
Safranin O staining
Saline
TePN hydrogel
(0.3 mg/ml TCA)
TePN hydrogel
(1.0 mg/ml TCA)
TePN hydrogel
(1.5 mg/ml TCA)
TCA solution
(0.3 mg/ml)
TCA solution
(1.5 mg/ml)

FIG. 15

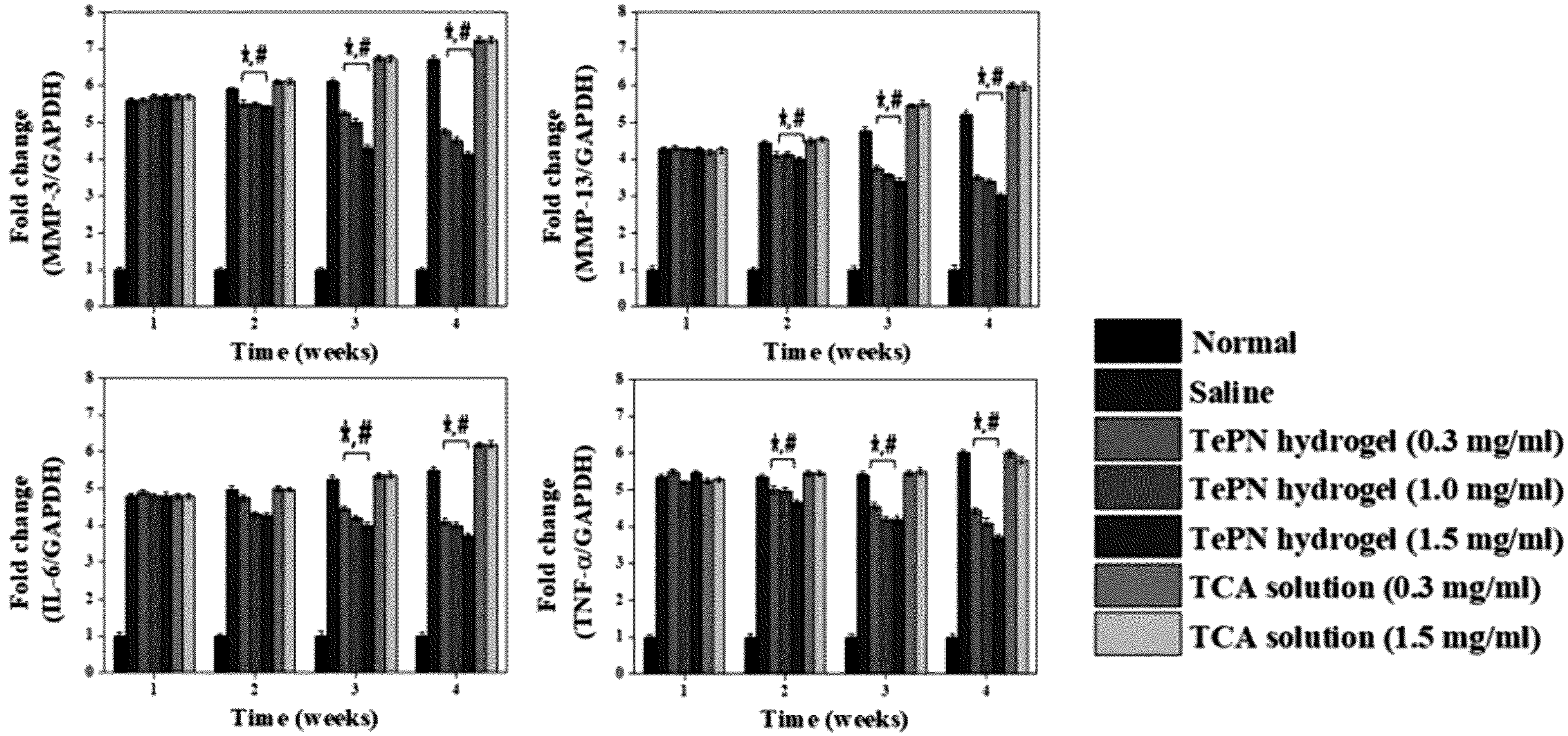


FIG. 16A

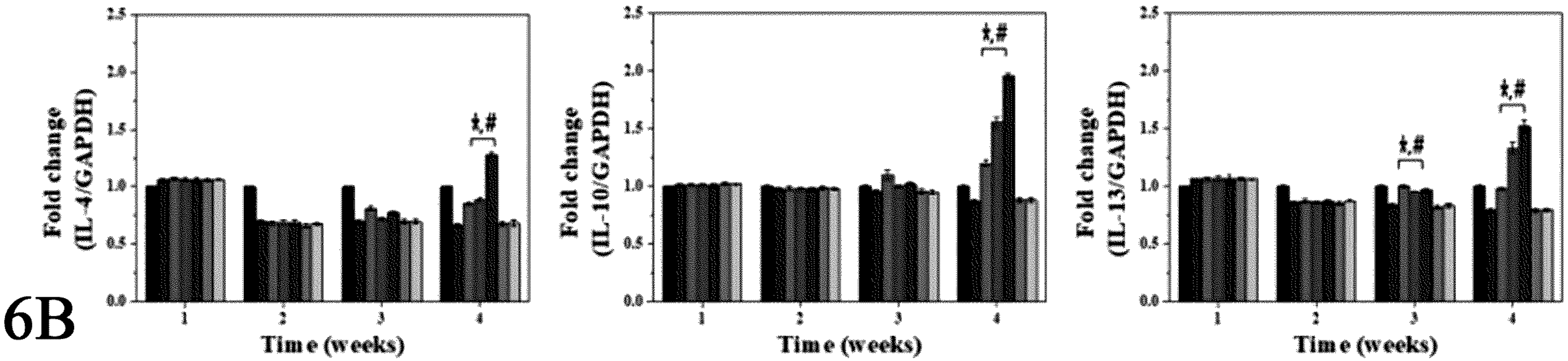


FIG. 16B

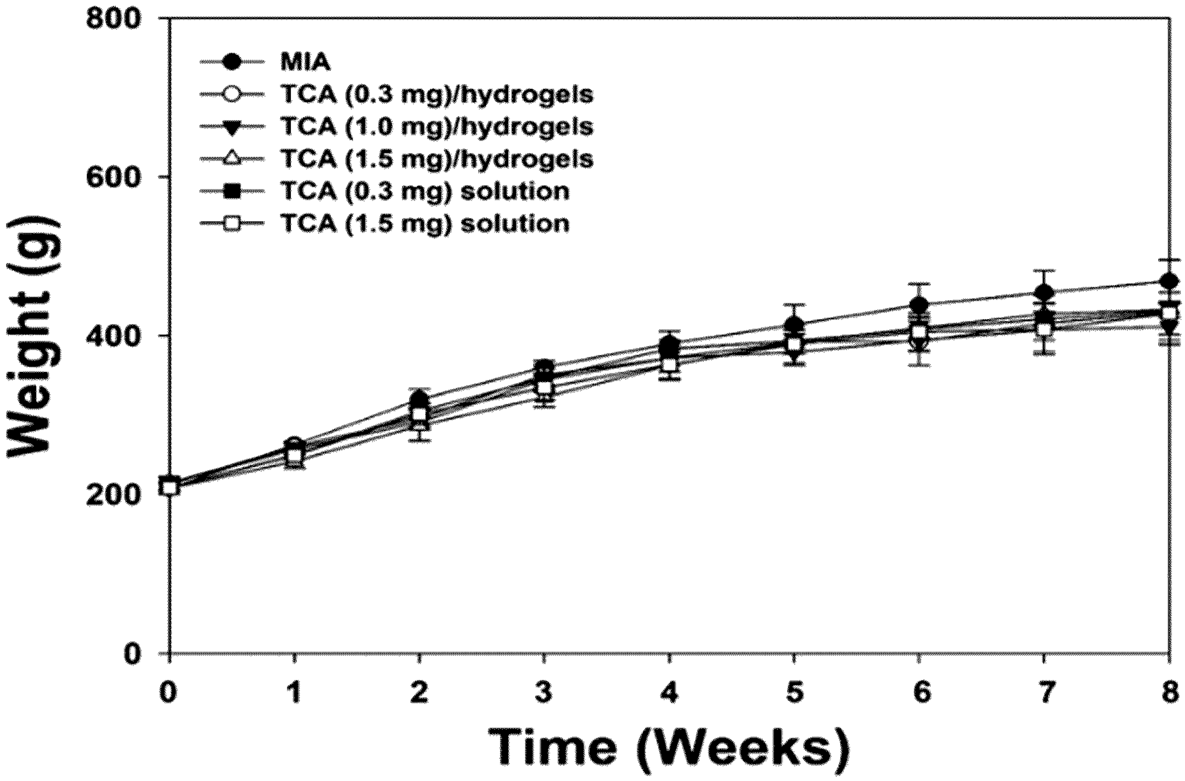
800
600
400
200
0
Weight (g)
0
1
2
3
4
5
6
7
8
Time (Weeks)
MIA
TCA (0.3 mg)/hydrogels
TCA (1.0 mg)/hydrogels
TCA (1.5 mg)/hydrogels
TCA (0.3 mg) solution
TCA (1.5 mg) solution

FIG. 17

46GA

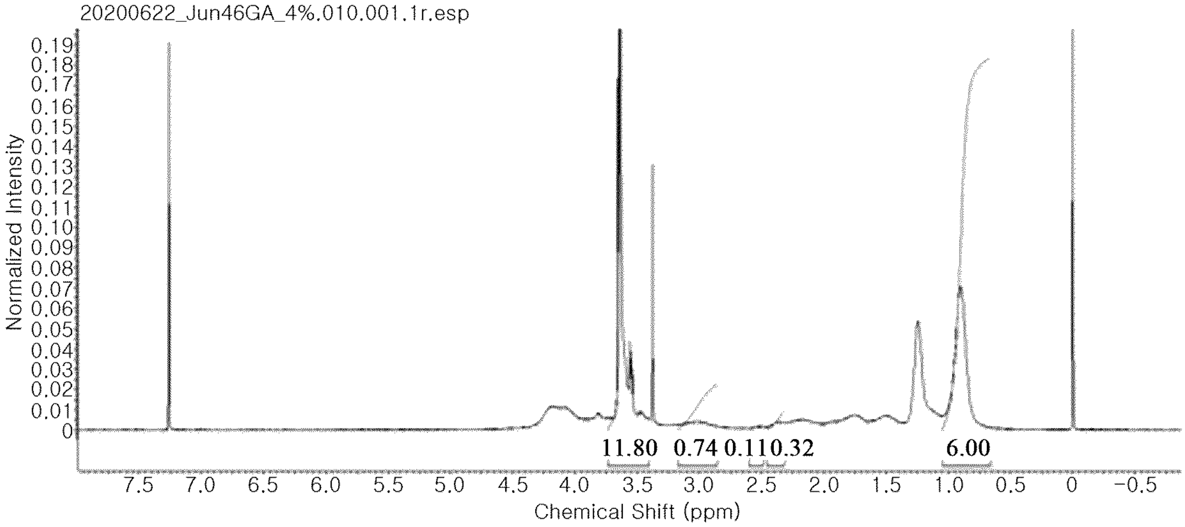


43GA

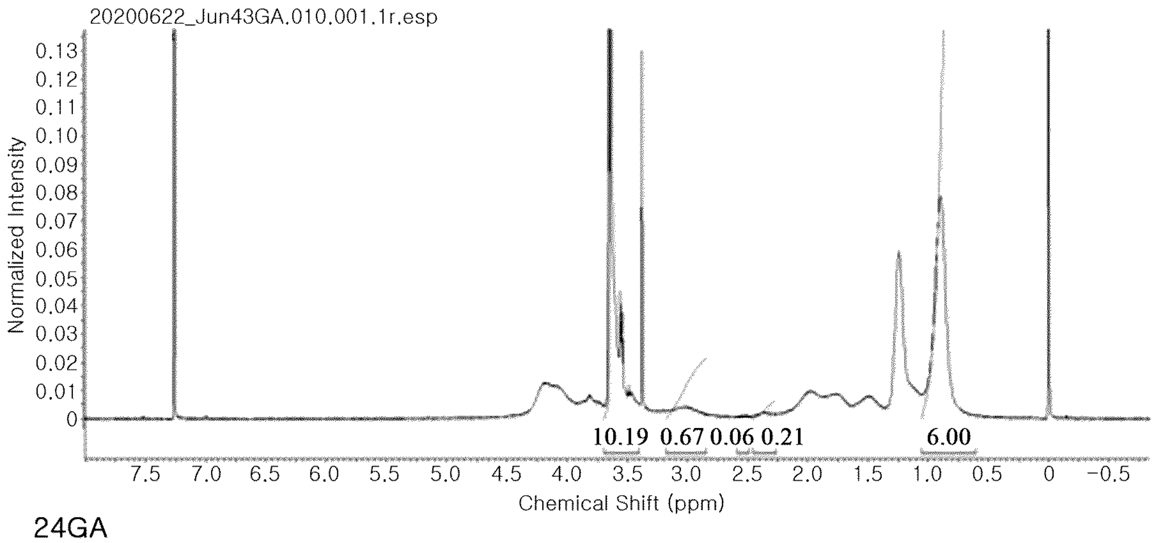


24GA

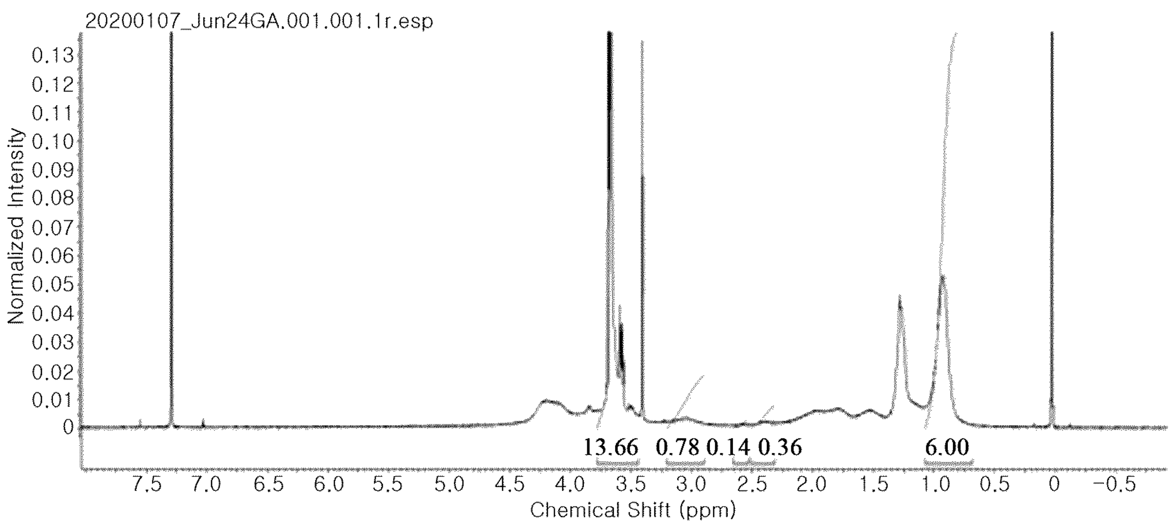


FIG. 18

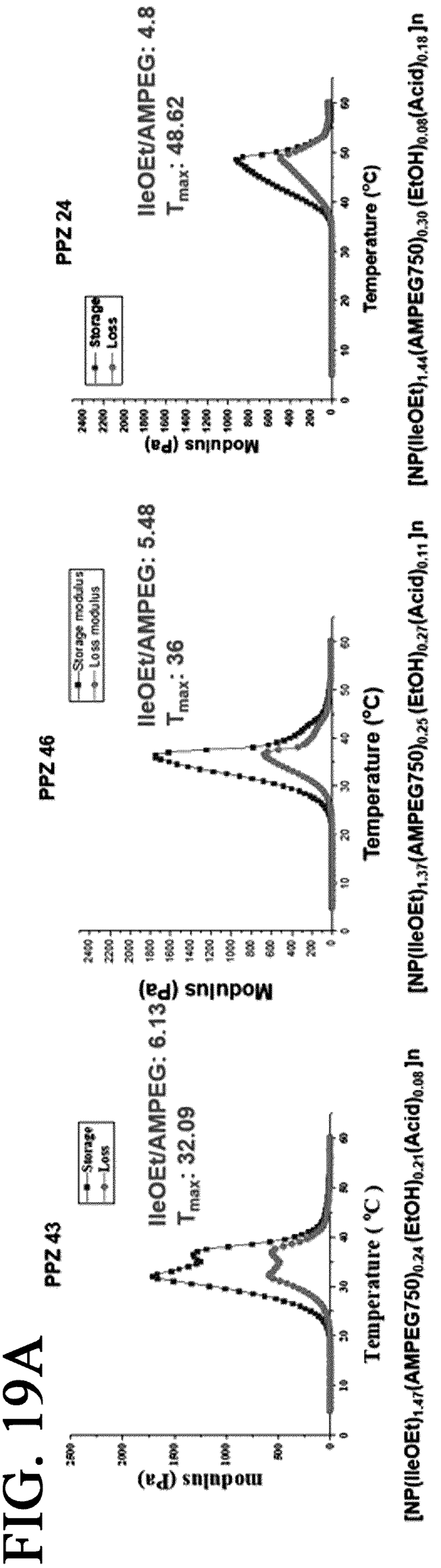


FIG. 19A

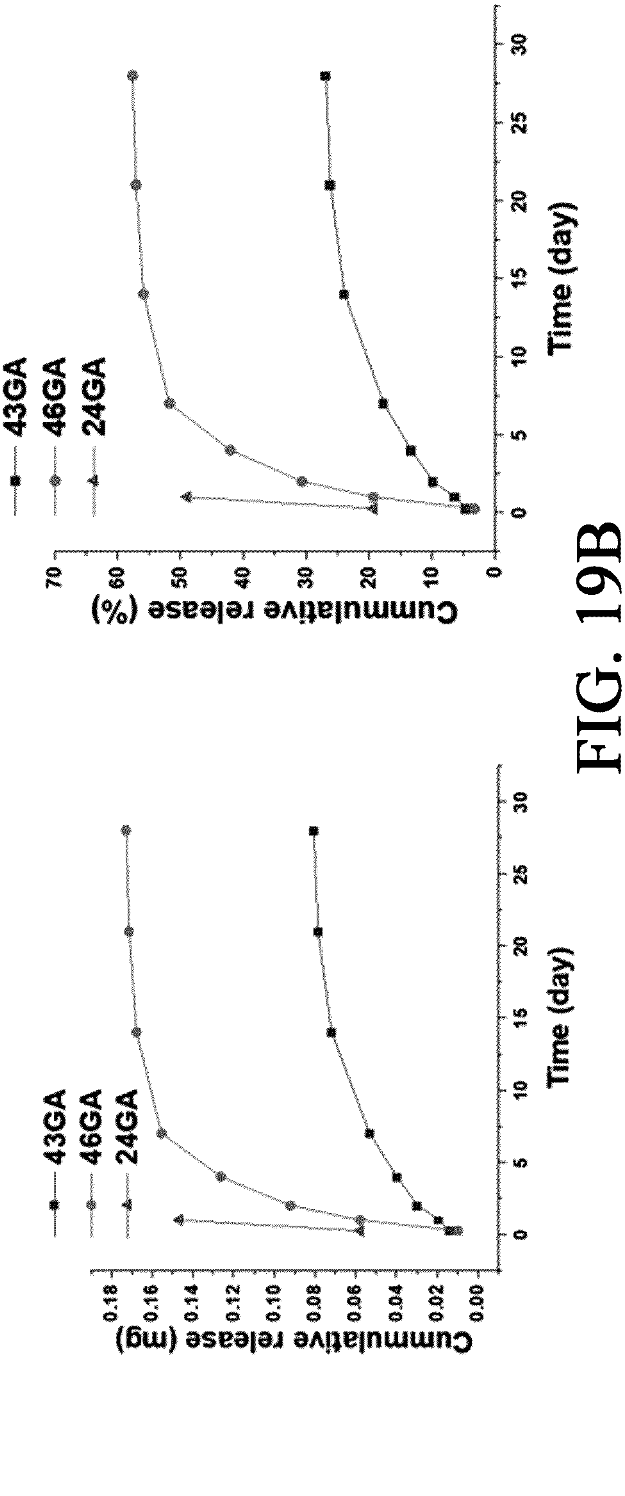


FIG. 19B

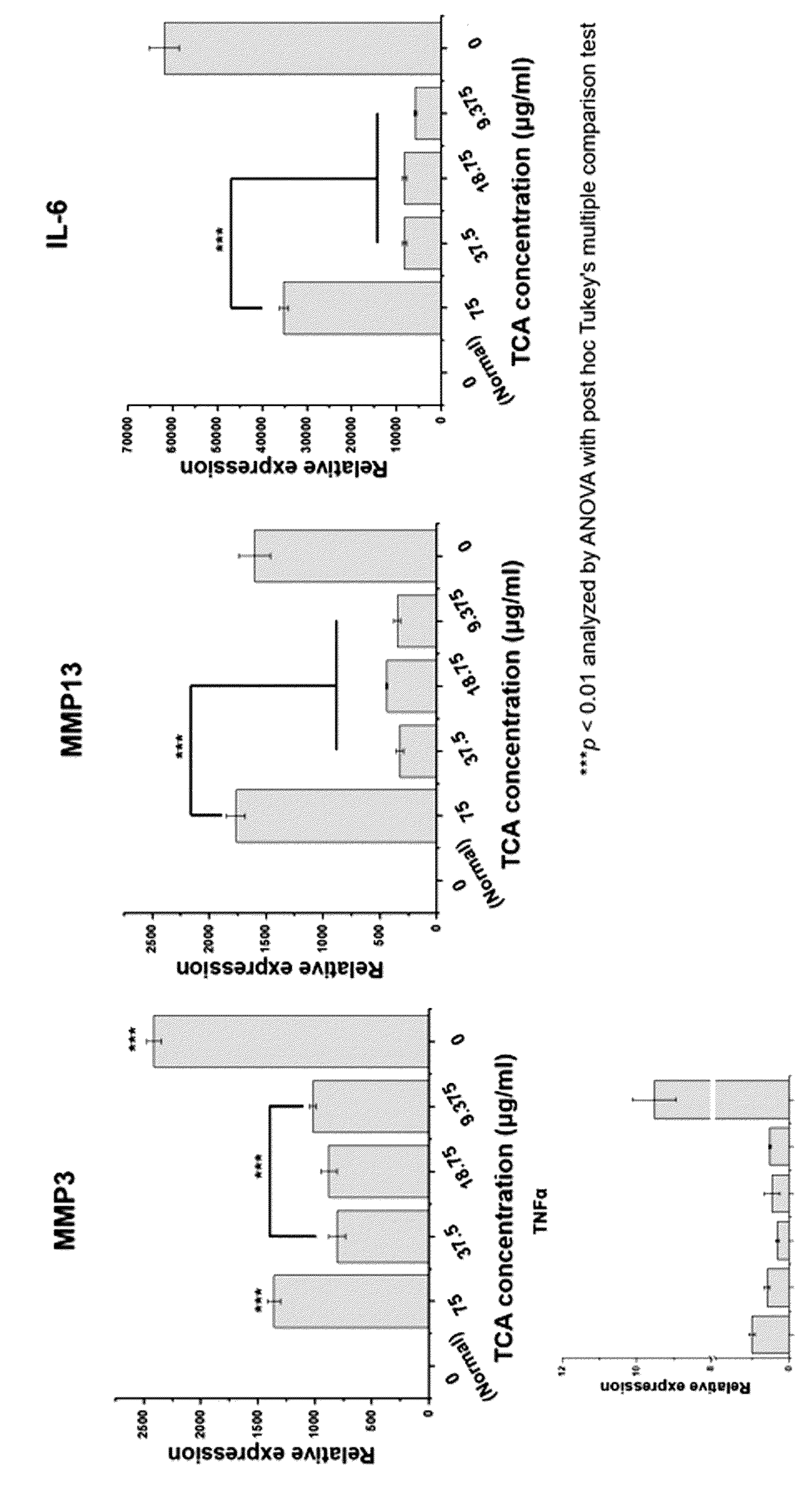
MMP3
MMP13
IL-6
TNFα
Relative expression
TCA concentration (μg/ml)
0 (Normal)
75
37.5
18.75
9.375
0
***
***p < 0.01 analyzed by ANOVA with post hoc Tukey's multiple comparison test

FIG. 20

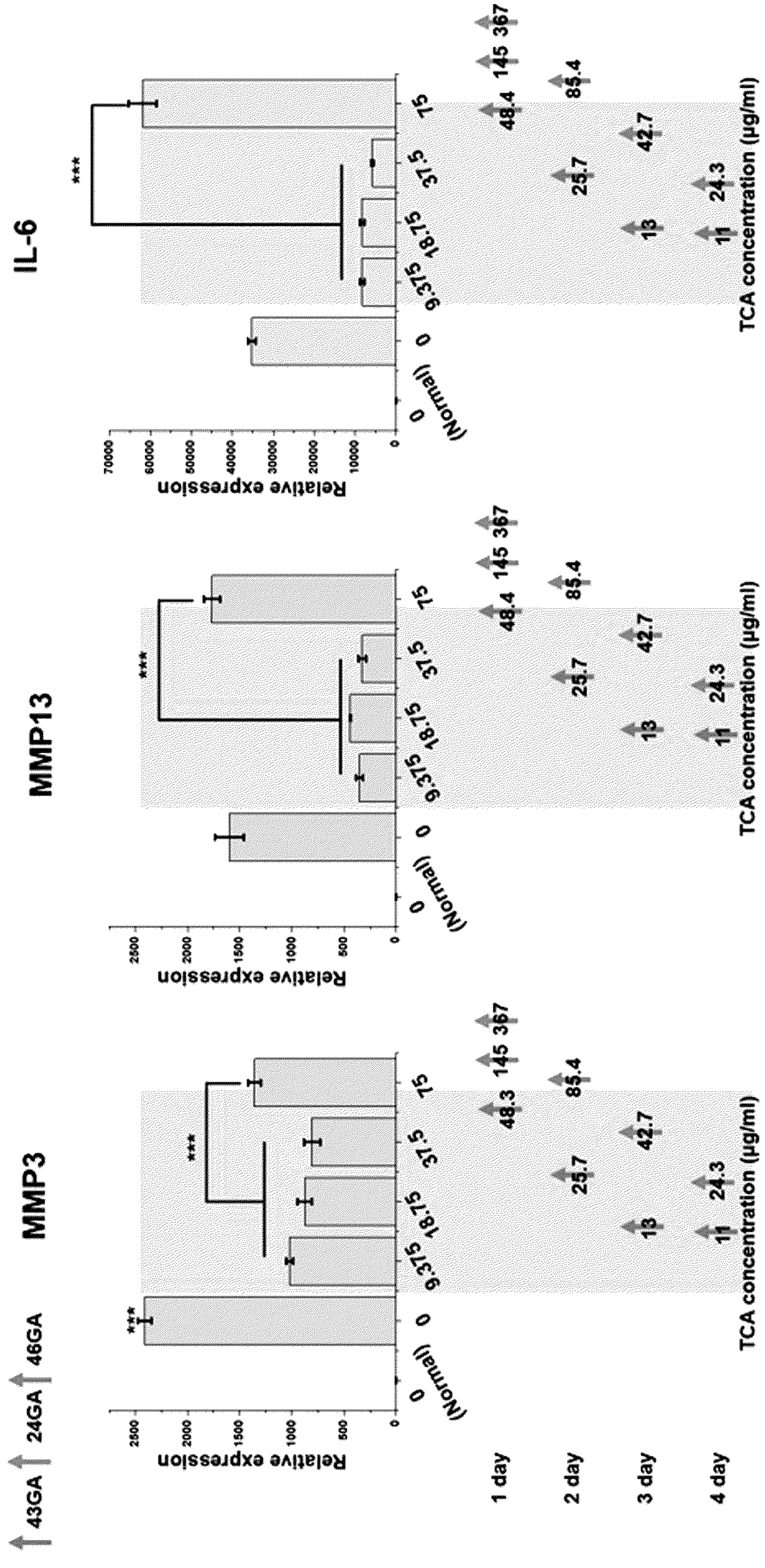
MMP3
MMP13
IL-6
43GA
24GA
46GA
Relative expression
TCA concentration (μg/ml)
0 (Normal)
0
9.375
18.75
37.5
75
***
1 day
2 day
3 day
4 day
48.3
48.4
145
367
25.7
85.4
13
42.7
11
24.3

FIG. 21

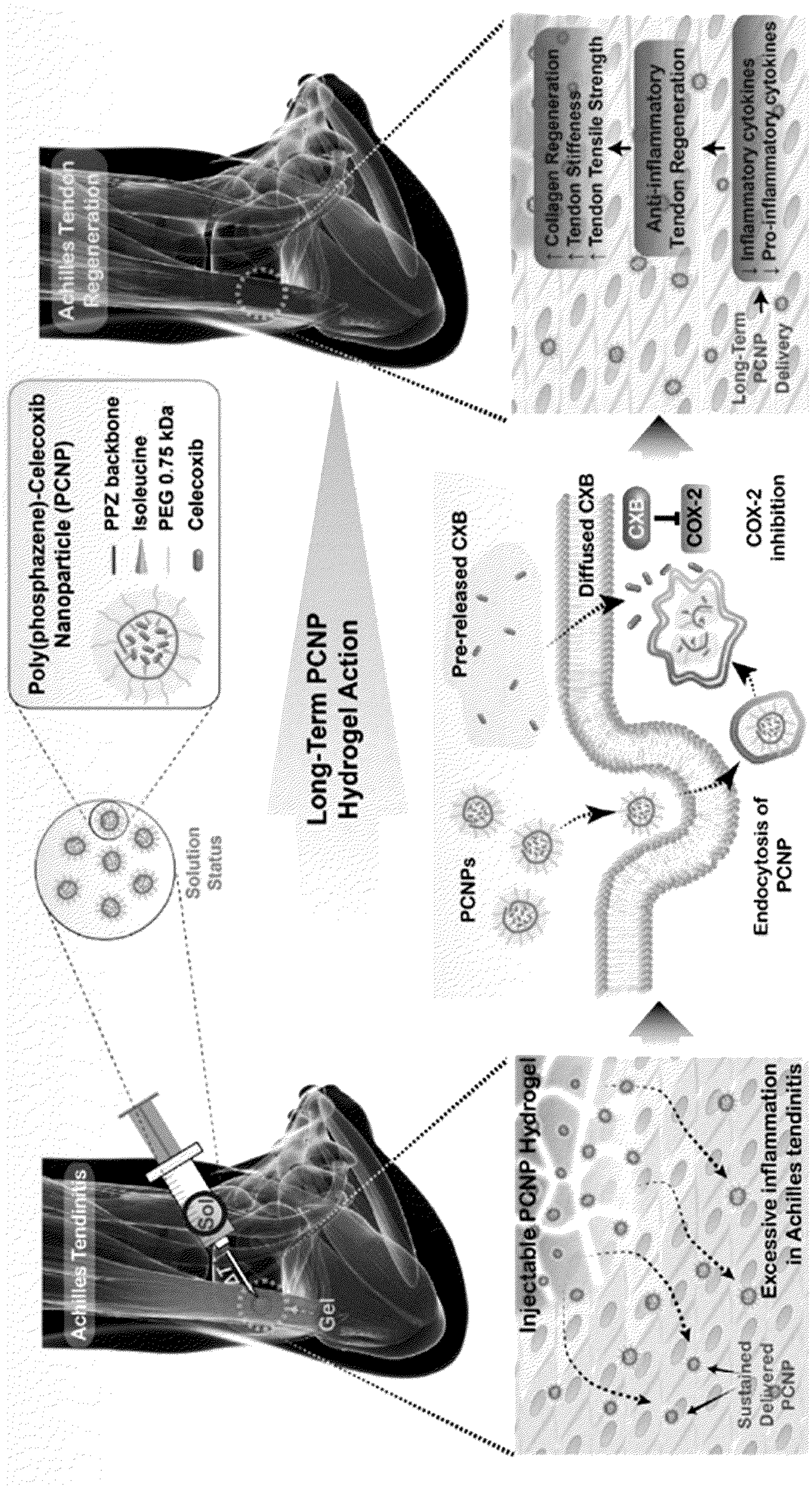
Achilles Tendinitis
Sol
Gel
Solution Status
Poly(phosphazene)-Celecoxib Nanoparticle (PCNP)
PPZ backbone
Isoleucine
PEG 0.75 kDa
Celecoxib
Achilles Tendon Regeneration
Long-Term PCNP Hydrogel Action
Injectable PCNP Hydrogel
Sustained Delivered PCNP
Excessive inflammation in Achilles tendinitis
PCNPs
Pre-released CXB
Diffused CXB
CXB
COX-2
COX-2 inhibition
Endocytosis of PCNP
Long-Term PCNP Delivery
↓ Inflammatory cytokines
↓ Pro-inflammatory cytokines
Anti-inflammatory
Tendon Regeneration
↑ Collagen Regeneration
↑ Tendon Stiffeness
↑ Tendon Tensile Strength

FIG. 22

FIG. 23A

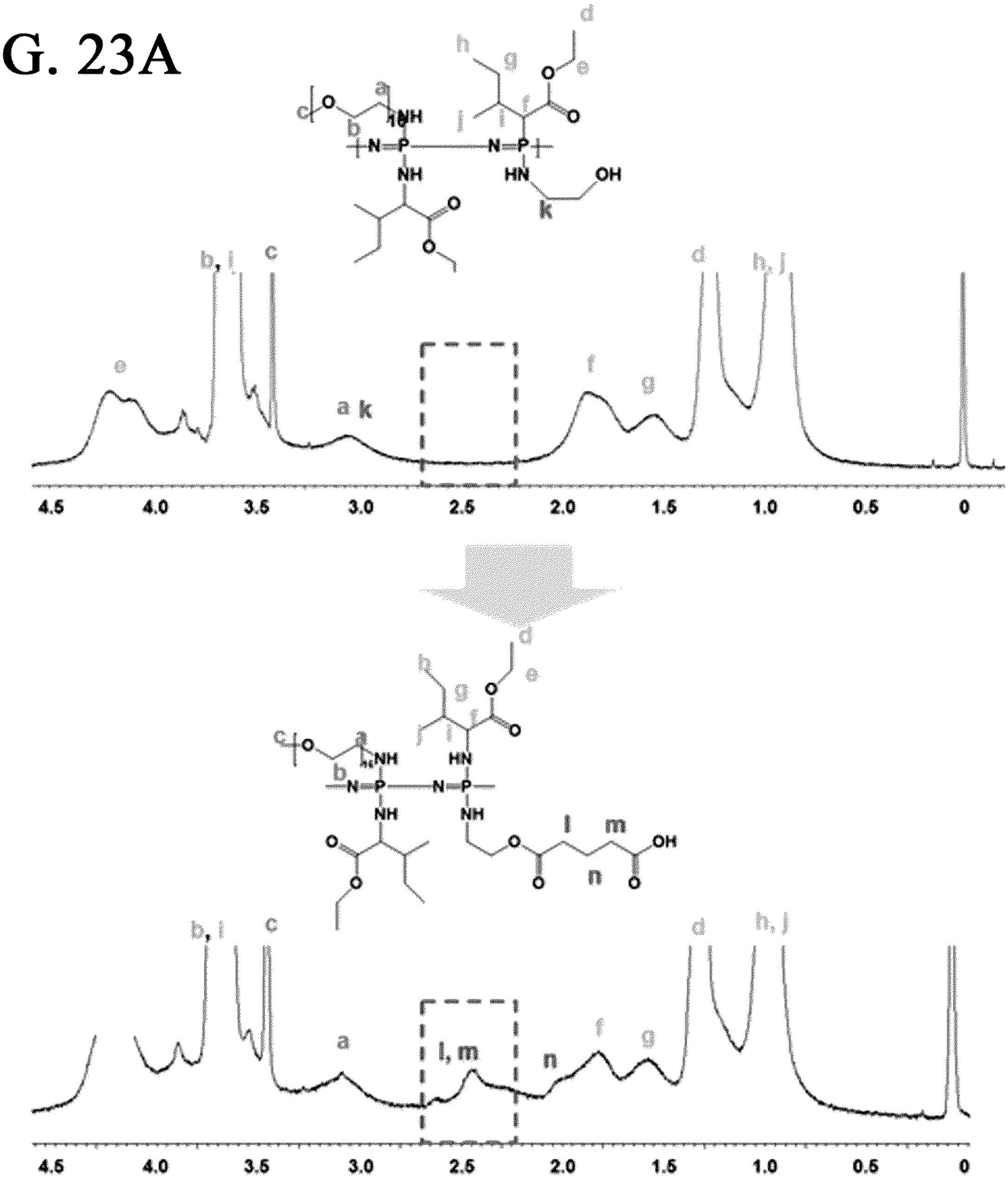

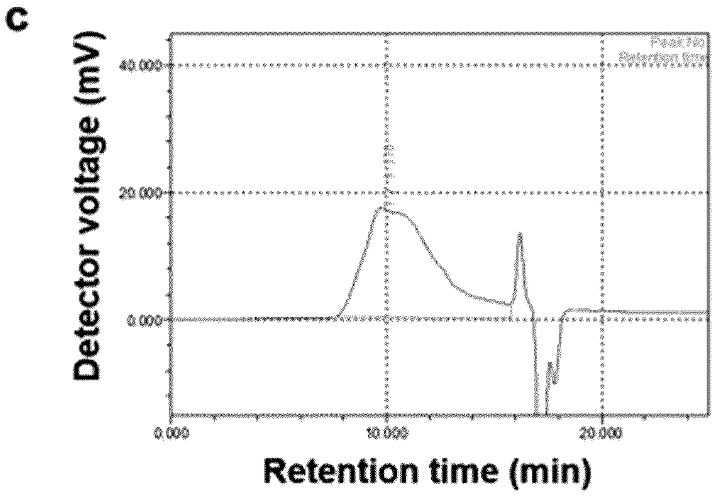


FIG. 23B

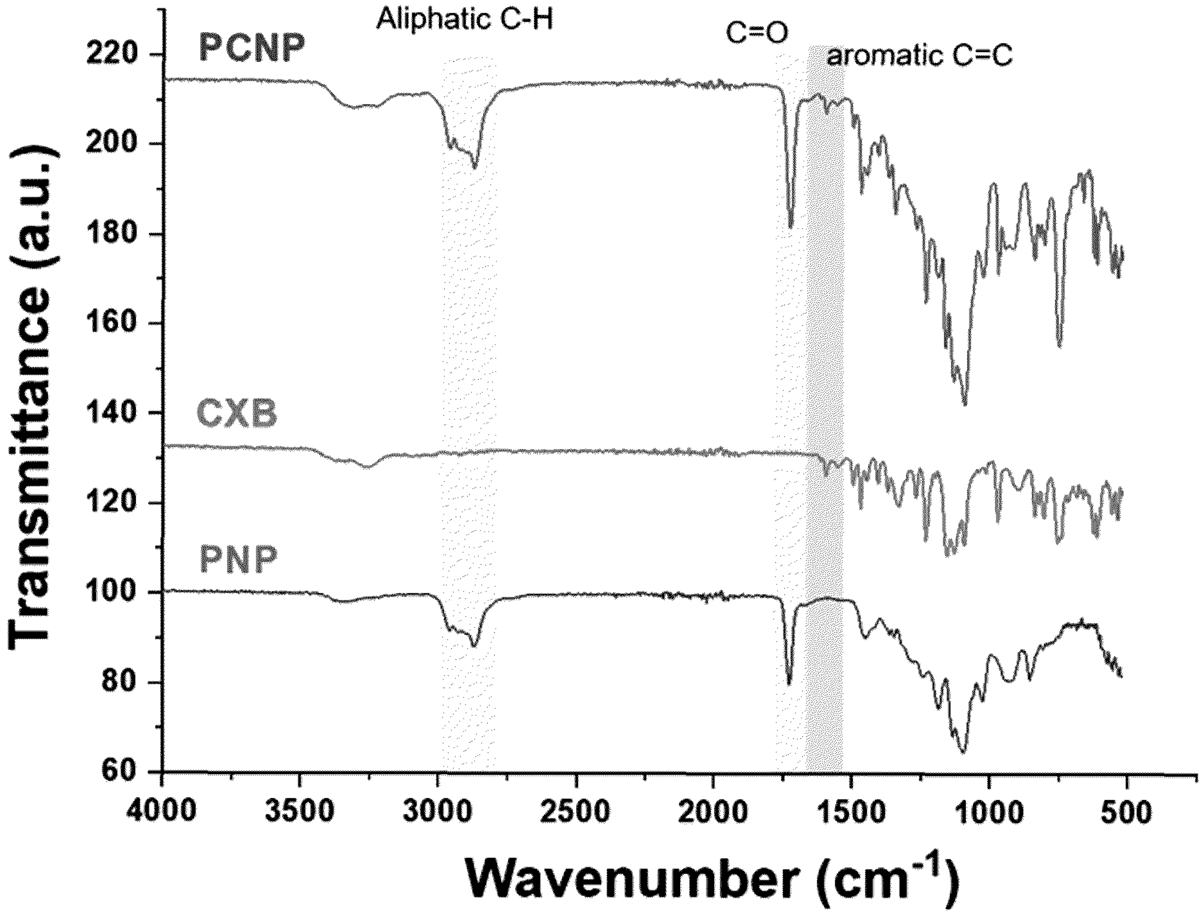
PCNP
Aliphatic C-H
C=O
aromatic C=C
CXB
PNP
220
200
180
160
140
120
100
80
60
Transmittance (a.u.)
4000
3500
3000
2500
2000
1500
1000
500
Wavenumber (cm-1)

FIG. 24

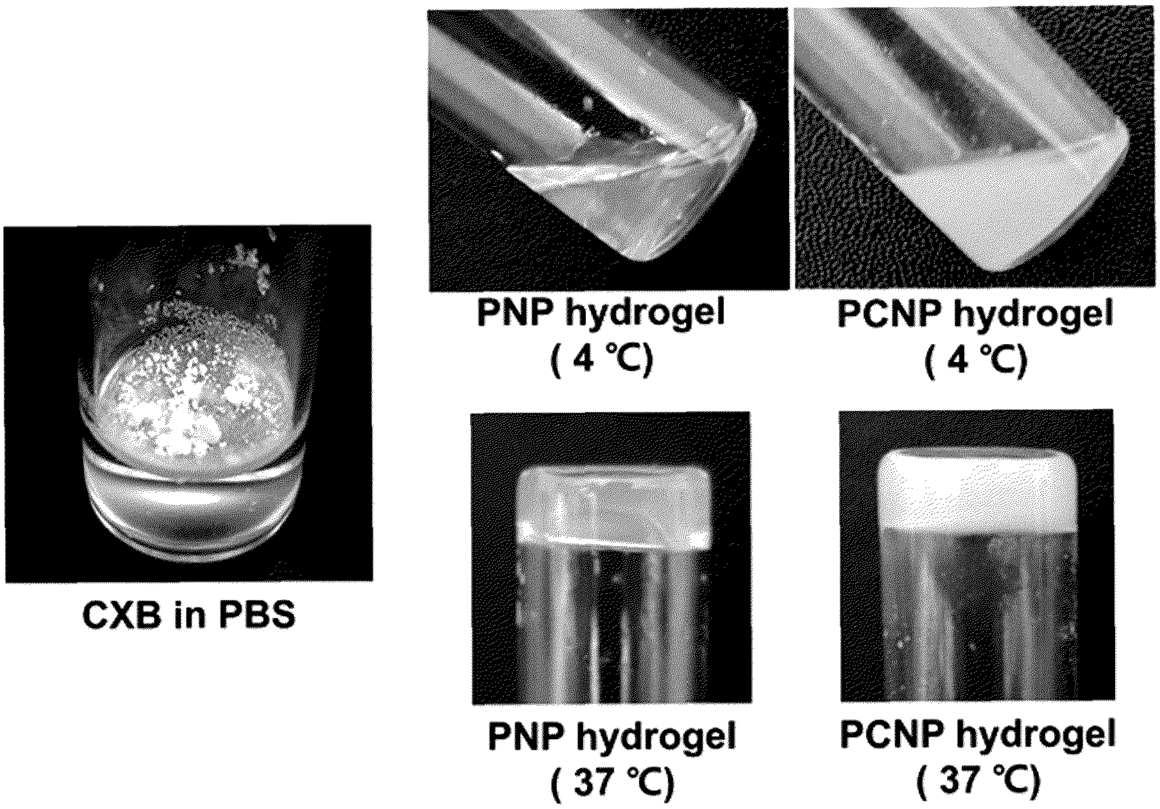
CXB in PBS
PNP hydrogel
( 4 ℃)
PCNP hydrogel
( 4 ℃)
PNP hydrogel
( 37 ℃)
PCNP hydrogel
( 37 ℃)

FIG. 25

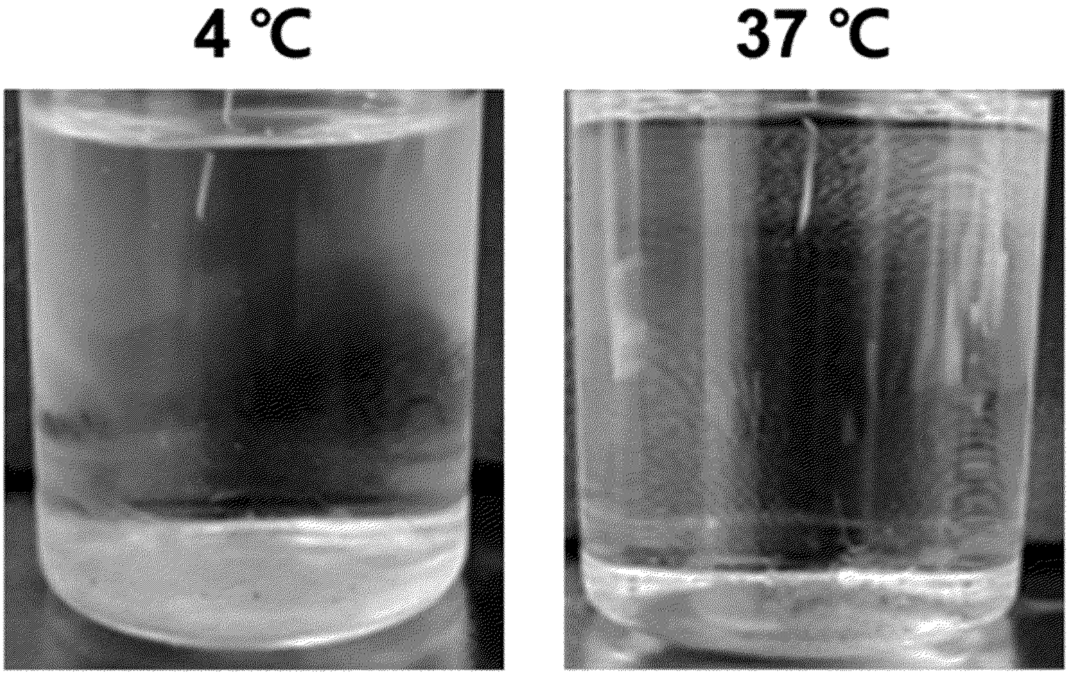


FIG. 26

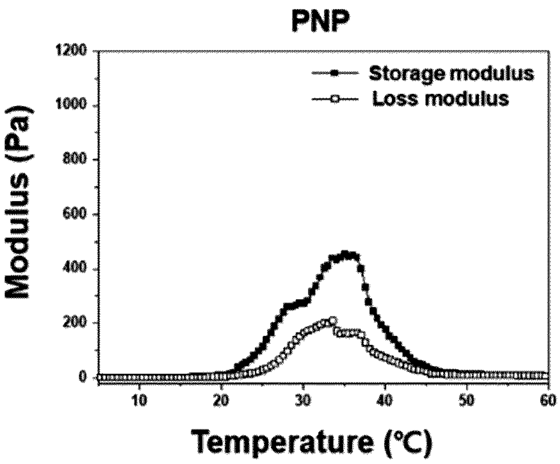


FIG. 27A

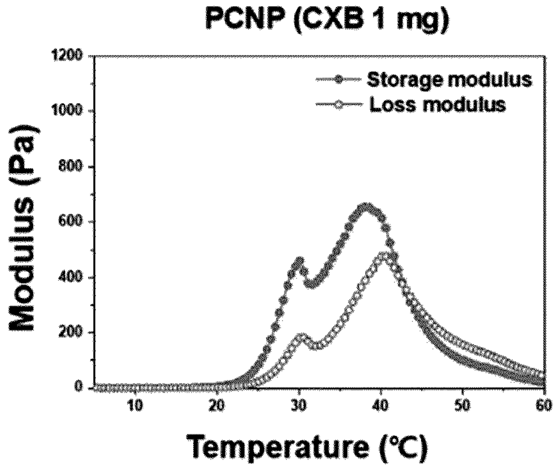


FIG. 27B

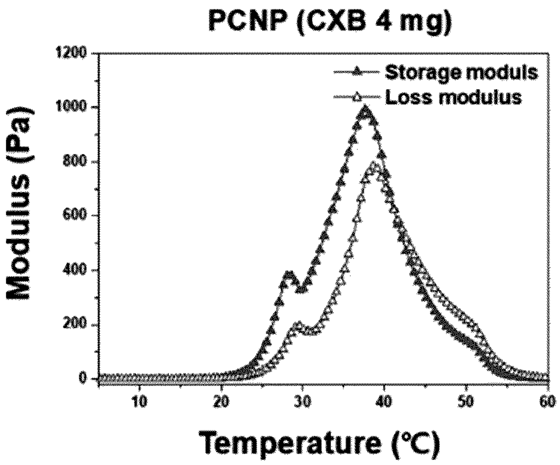


FIG. 27C

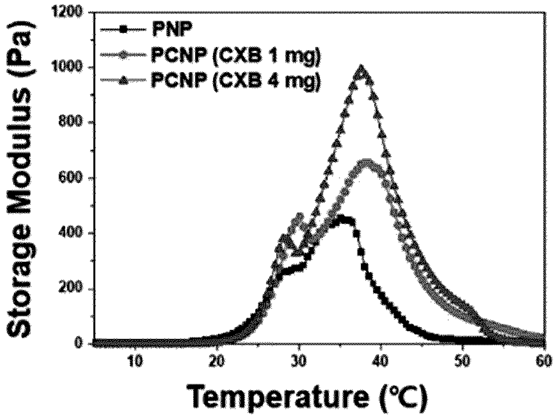


FIG. 27D

FIG.28A

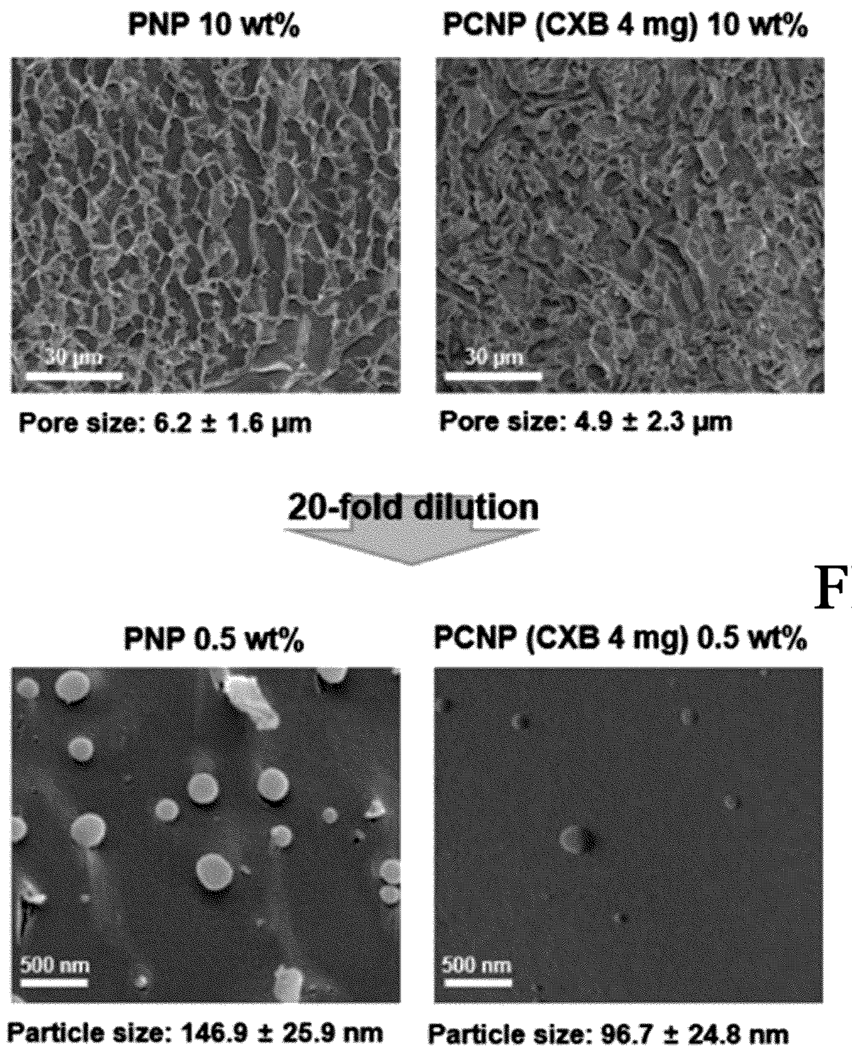


FIG.28B

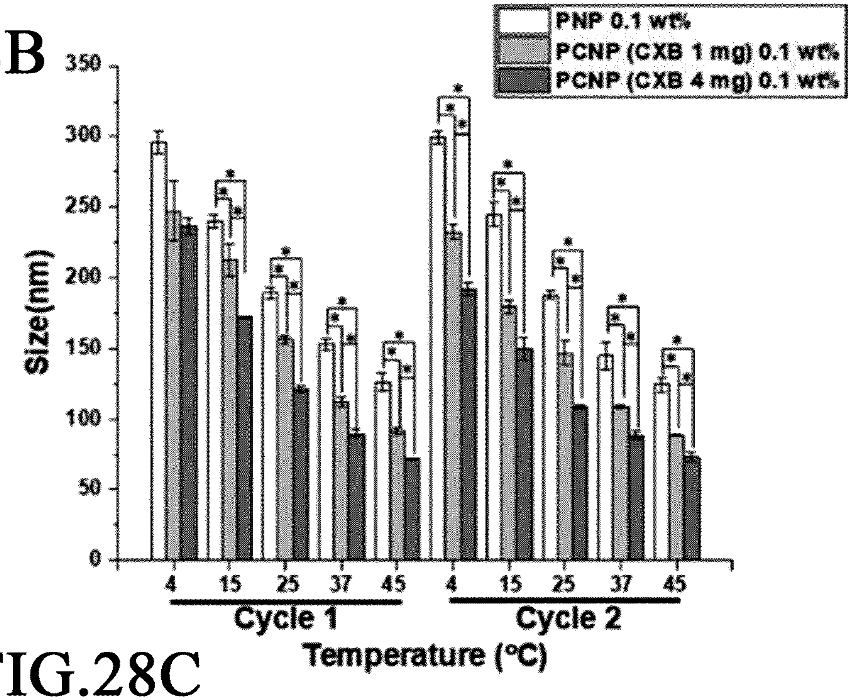


FIG.28C

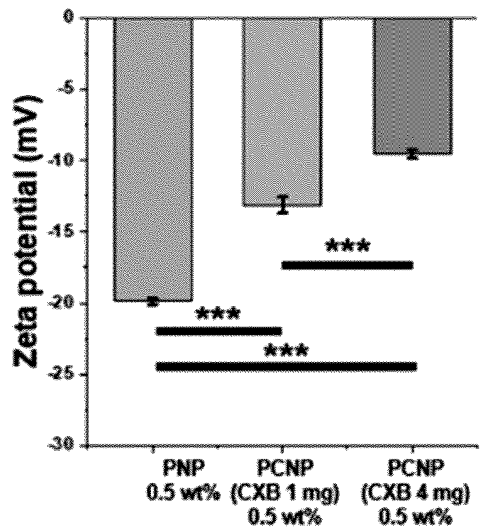

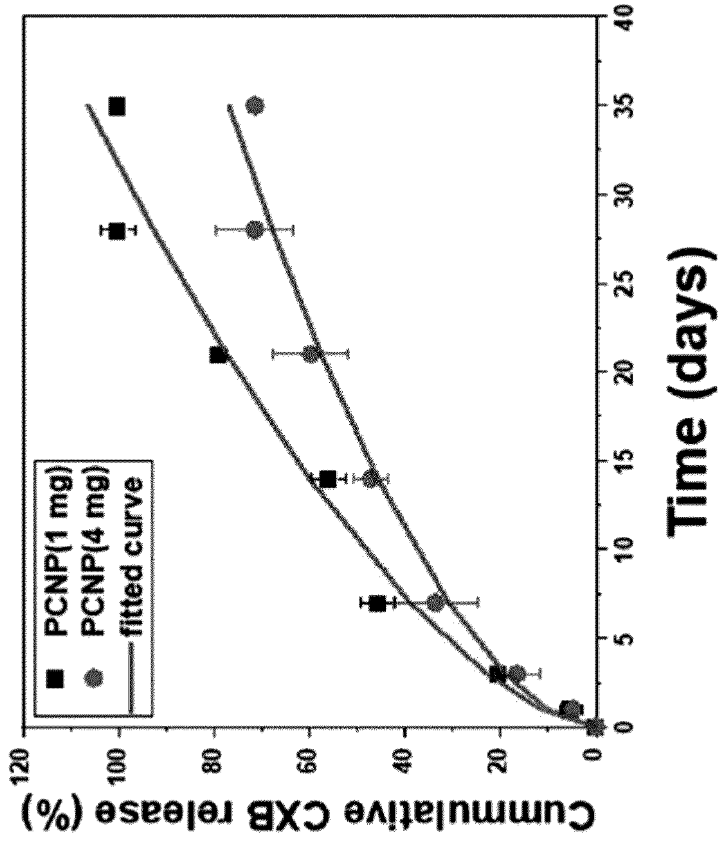


FIG. 29A

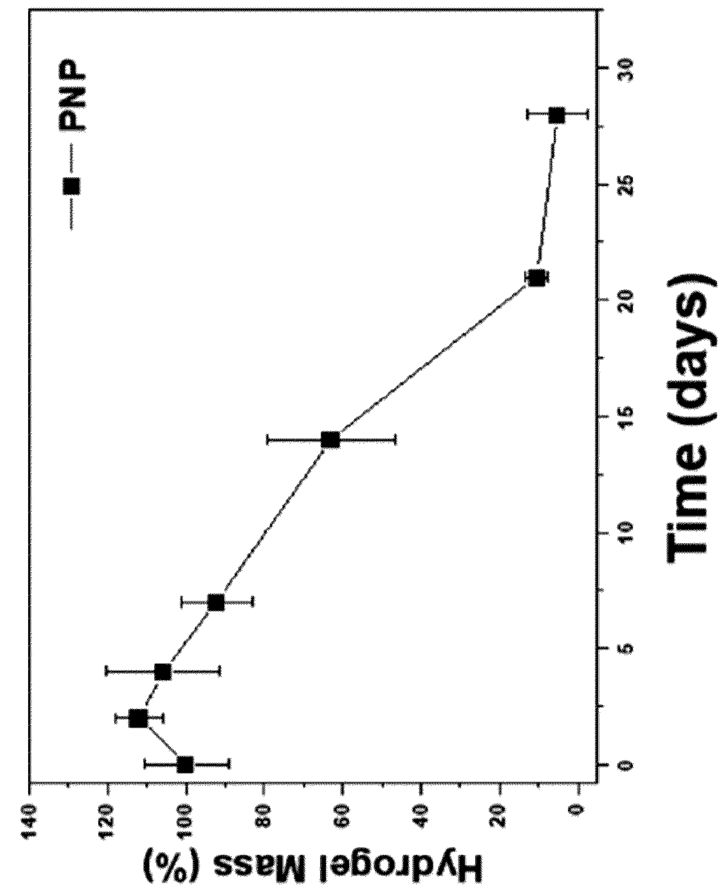


FIG. 29B

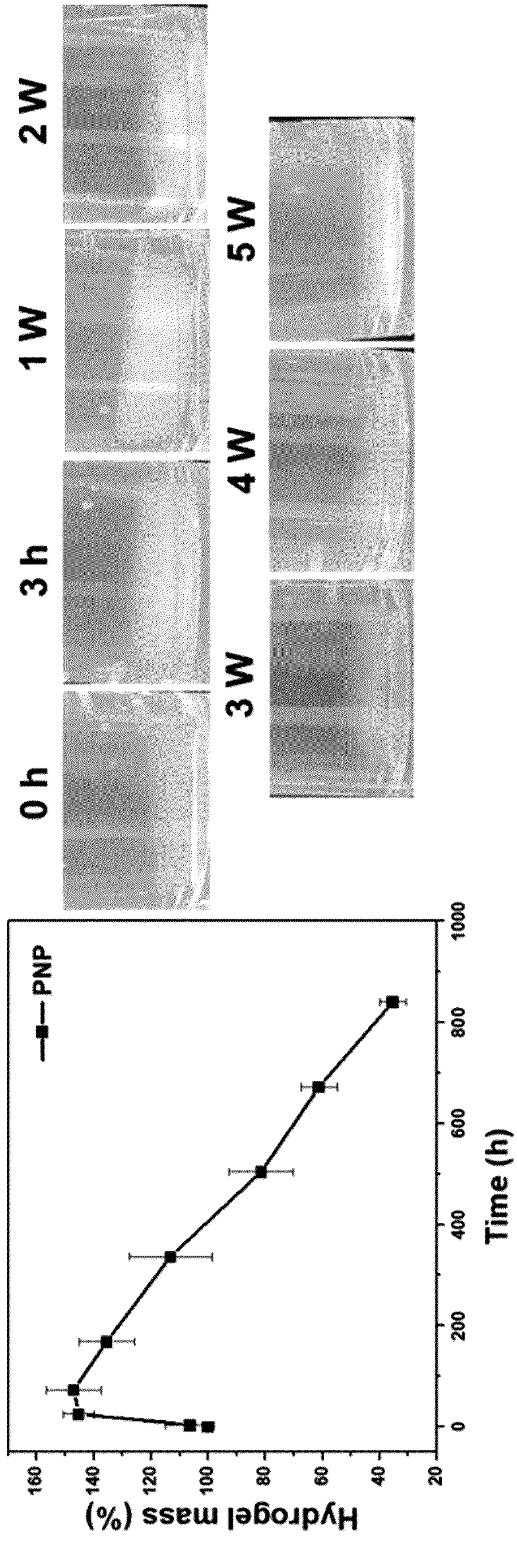


FIG. 30

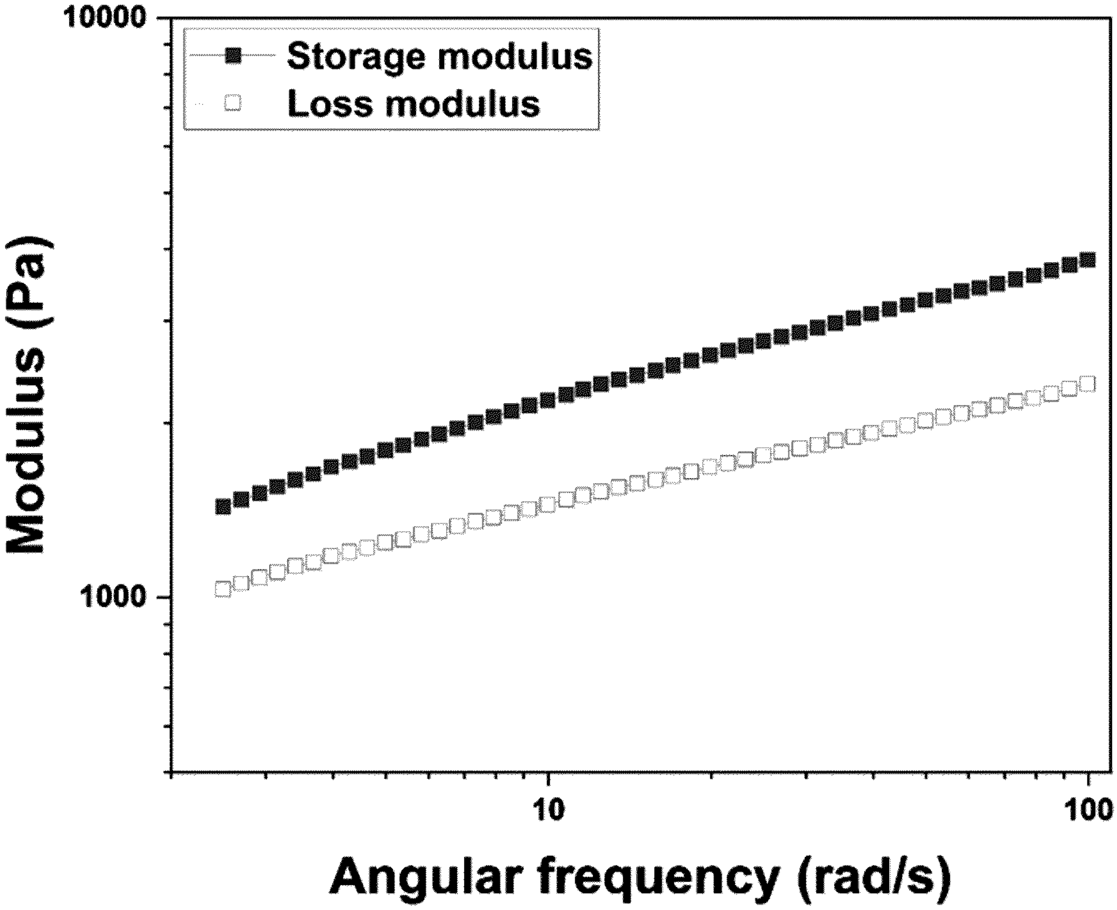
10000
1000
Modulus (Pa)
10
100
Angular frequency (rad/s)
Storage modulus
Loss modulus

FIG .31

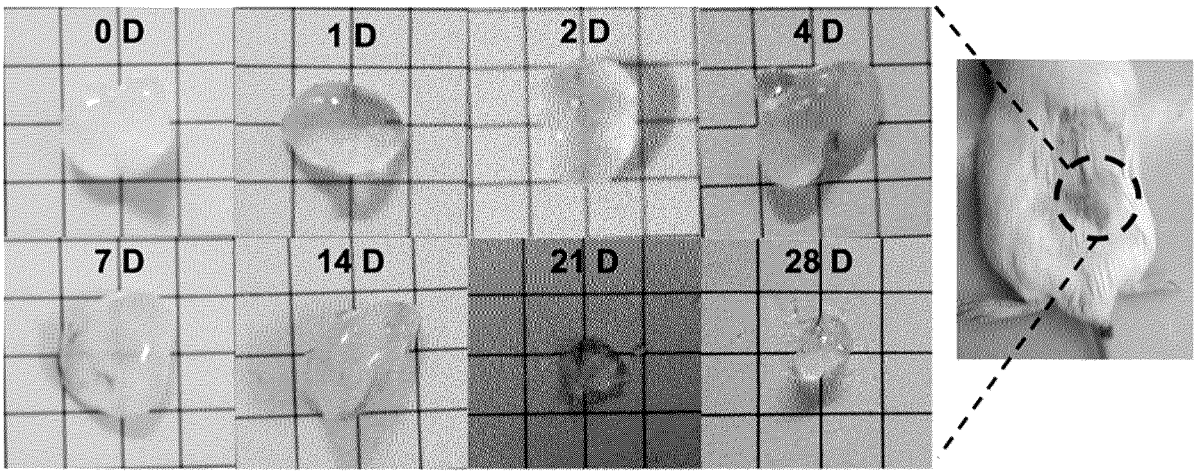


FIG. 32

FIG. 33A

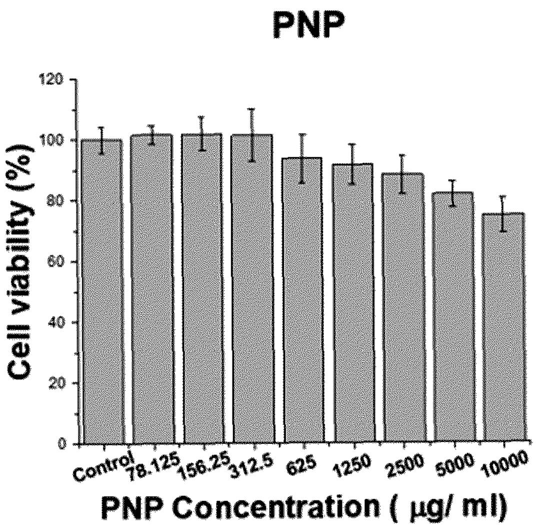


FIG. 33B

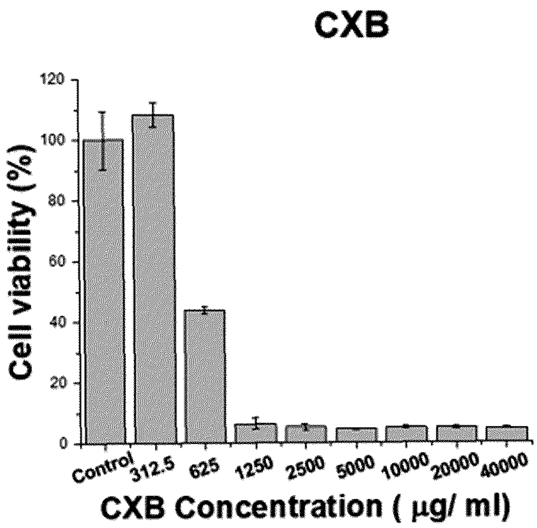


FIG. 33C

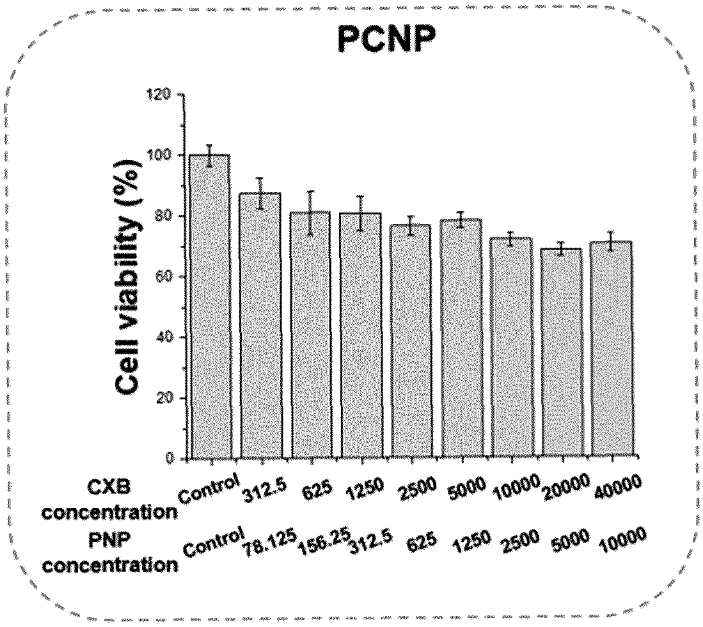

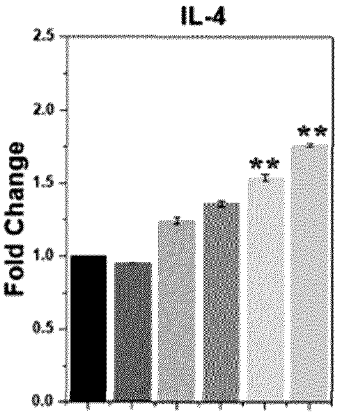


FIG. 34A

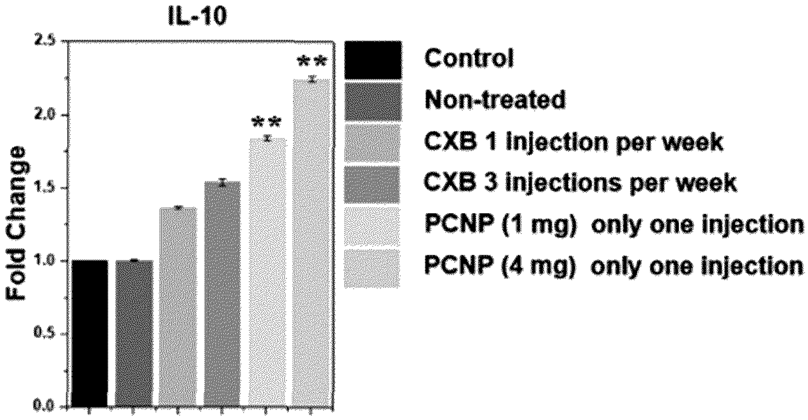


FIG. 34B

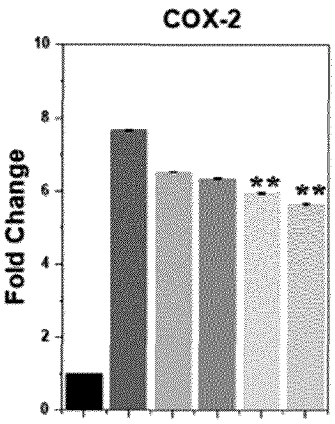


FIG. 34C

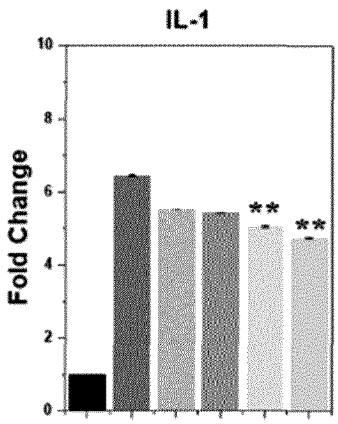


FIG. 34D

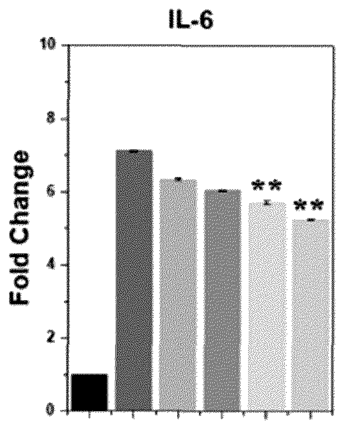


FIG. 34E

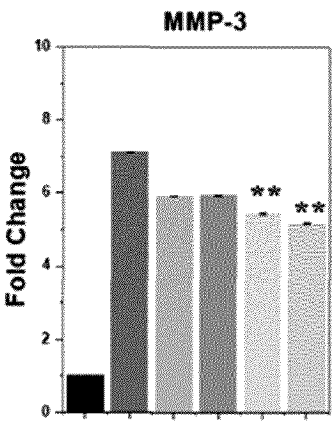


FIG. 34F

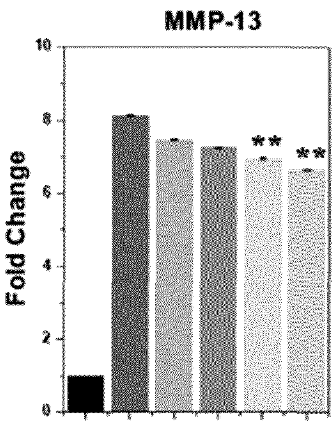


FIG. 34G

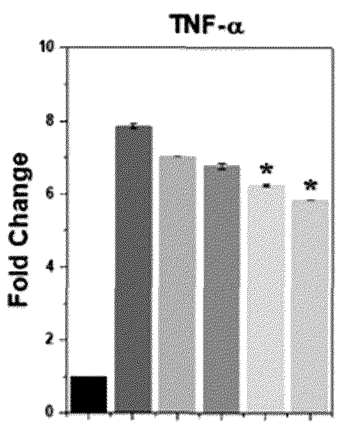


FIG. 34H

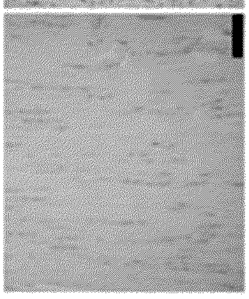

FIG. 35A

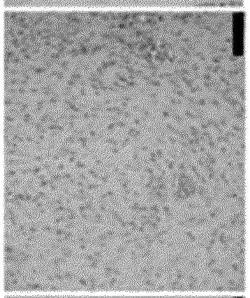

FIG. 35C

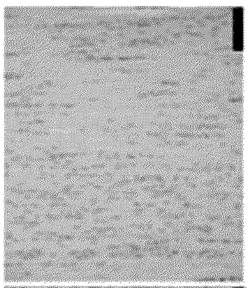

FIG. 35E

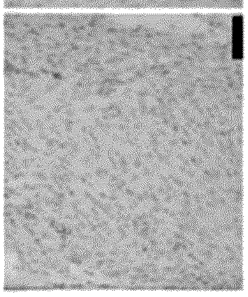

FIG. 35B

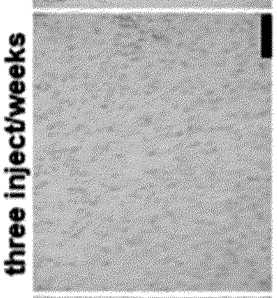

FIG. 35D

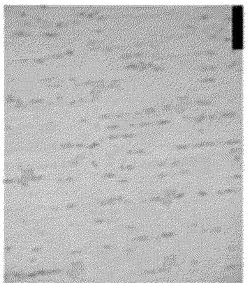

FIG. 35F

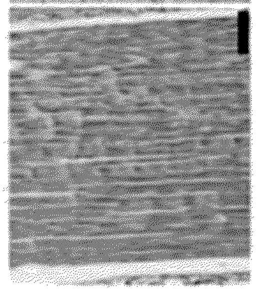

FIG. 35G

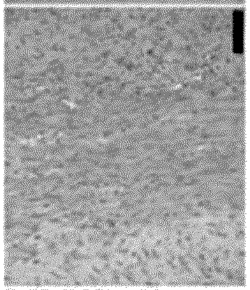

FIG. 35I

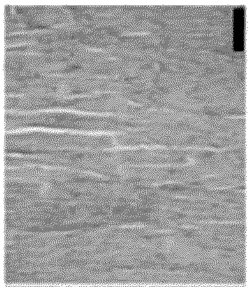

FIG. 35K

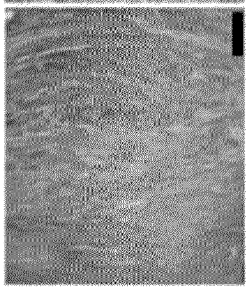

FIG. 35H

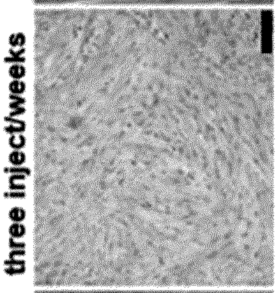

FIG. 35J

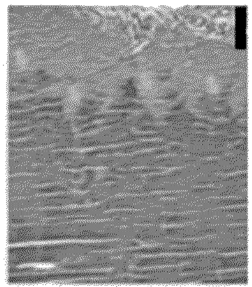

FIG. 35L

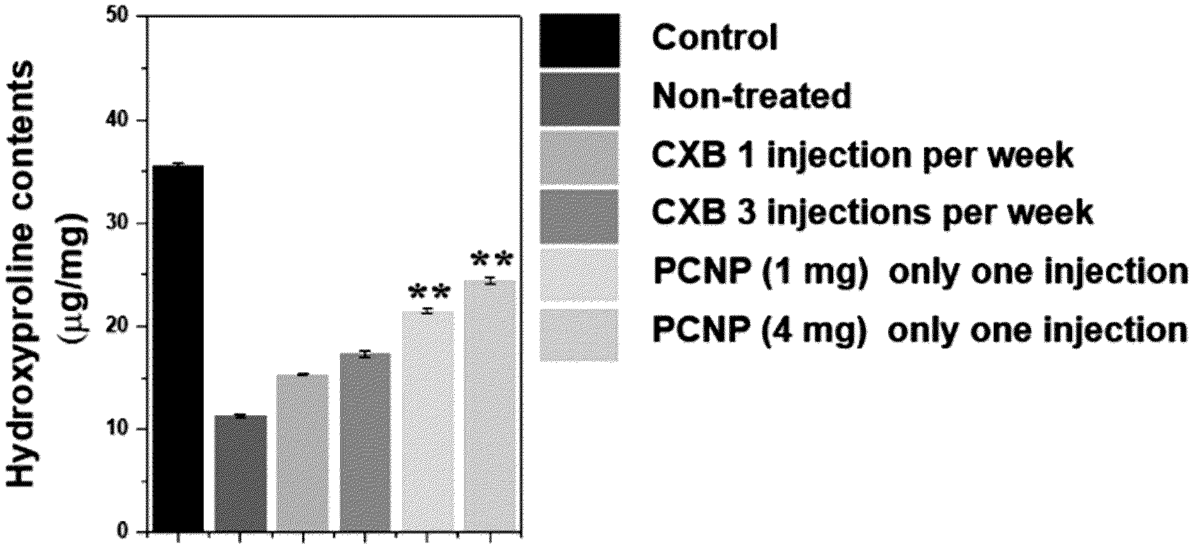


FIG. 36A

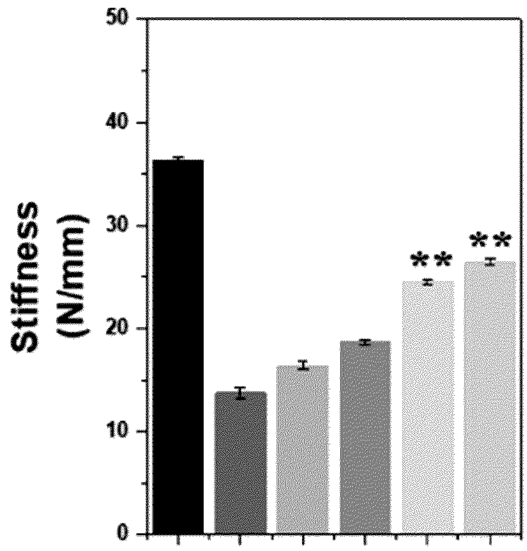


FIG. 36B

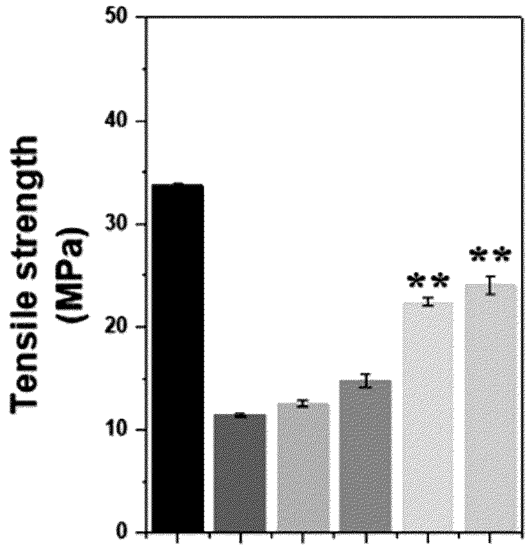


FIG. 36C

**INTERNATIONAL SEARCH REPORT**

International application No. **PCT/KR2023/007948**

**A. CLASSIFICATION OF SUBJECT MATTER**

**A61K 9/06**(2006.01)i; **A61K 47/34**(2006.01)i; **A61K 9/00**(2006.01)i; **A61P 29/00**(2006.01)i; **A61P 19/02**(2006.01)i

According to International Patent Classification (IPC) or to both national classification and IPC

**B. FIELDS SEARCHED**

Minimum documentation searched (classification system followed by classification symbols)

A61K 9/06(2006.01); A61K 31/13(2006.01); C08G 79/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 폴리포스파젠(polyphosphazene), 아미노산 에스테르(amino acid ester), 폴리에틸렌글리콜(polyethylene glycol), 아미노에탄올(aminoethanol), 글루타르산(glutaric acid), 염증성 질환(inflammatory disease)

**C. DOCUMENTS CONSIDERED TO BE RELEVANT**

| Category* | Citation of document, with indication, where appropriate, of the relevant passages | Relevant to claim No. |
|---|---|---|
| A | STRASSER, P. et al. Main-chain phosphorus-containing polymers for therapeutic applications. Molecules. 2020, vol. 25, article no. 1716, pp. 1-47.<br>See abstract; pages 12 and 15; and figure 17. | 1-13 |
| A | HONG, L. T. A. et al. An injectable hydrogel enhances tissue repair after spinal cord injury by promoting extracellular matrix remodeling. Nature communications. 2017, vol. 8, article no. 533, pp. 1-14.<br>See entire document. | 1-13 |
| A | CHUN, C. et al. Doxorubicin-polyphosphazene conjugate hydrogels for locally controlled delivery of cancer therapeutics. Biomaterials. 2009, vol. 30, pp. 4752-4762.<br>See entire document. | 1-13 |
| A | KR 10-2014-0016521 A (KOREA INSTITUTE OF SCIENCE AND TECHNOLOGY) 10 February 2014 (2014-02-10)<br>See entire document. | 1-13 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 September 2023** | **08 September 2023** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2023/007948**

**C. DOCUMENTS CONSIDERED TO BE RELEVANT**

| Category* | Citation of document, with indication, where appropriate, of the relevant passages | Relevant to claim No. |
|---|---|---|
| X | SEO, B.-B. et al. Injectable polymeric nanoparticle hydrogel system for long-term anti-inflammatory effect to treat osteoarthritis. Bioactive materials. January 2022, vol. 7, pp. 14-25. See abstract; pages 14-17, 19, 21 and 22; scheme 1; and figure 2. ※ This document is a known document declaring exceptions to lack of novelty by the applicant. | 1-13 |
| X | KIM, J. et al. Long-term anti-inflammatory effects of injectable celecoxib nanoparticle hydrogels for Achilles tendon regeneration. Acta biomaterialia. May 2022, vol. 144, pp. 183-194. See abstract; pages 184, 186 and 190; scheme 1; and figures 1, 3, 5 and 6. ※ This document is a known document declaring exceptions to lack of novelty by the applicant. | 1-13 |

Form PCT/ISA/210 (second sheet) (July 2022)

**INTERNATIONAL SEARCH REPORT**
**Information on patent family members**

International application No.
**PCT/KR2023/007948**

| Patent document cited in search report | Publication date (day/month/year) | Patent family member(s) | Publication date (day/month/year) |
|---|---|---|---|
| KR 10-2014-0016521 A | 10 February 2014 | KR 10-1480363 B1 | 09 January 2015 |
| | | US 2014-0031289 A1 | 30 January 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BUCHMAN A.L**. Side effects of corticosteroid therapy. *J. Clin. Gastroenterol.*, 2001, vol. 33 (4), 289-294
- **LARSEN C.** ; **OSTERGAARD J.** ; **LARSEN S.W.** ; **JENSEN H.** ; **JACOBSEN S.** ; **LINDEGAARD C.** ; **ANDERSEN P.H**. Intra-articular depot formulation principles: role in the management of postoperative pain and arthritic disorders. *J. Pharmacol. Sci.*, 2008, vol. 97 (11), 4622-4654
- **PRADAL J.** ; **ZULUAGA M.F.** ; **MAUDENS P.** ; **WALDBURGER J.M.** ; **SEEMAYER C.A.** ; **DOELKER E.** ; **GABAY C.** ; **JORDAN O.** ; **ALLEMANN E**. Intra-articular bioactivity of a p38 MAPK inhibitor and development of an extended-release system. EUR. *J. Pharm. Biopharm.*, 2015, vol. 93, 110-117
- **KOPECEK J**. Hydrogel biomaterials: a smart future?. *Biomaterials*, 2007, vol. 28 (34), 5185-5192
- **PARK C.W.** ; **MA K.W.** ; **JANG S.W.** ; **SON M.** ; **KANG M.J**. Comparison of piroxicam pharmacokinetics and anti-inflammatory effect in rats after intra-articular and intramuscular administration. *Biomol. Ther.*, 2014, vol. 22 (3), 260-266
- **HONG K.H.** ; **KIM Y.M.** ; **SONG S.C**. Fine-tunable and injectable 3D hydrogel for on-demand stem cell niche. *Adv. Sci.*, 2019, vol. 6 (17), 1900597
- **BROMBERG L.** ; **TEMCHENKO M**. Self-assembly in aqueous solutions of poly(ethylene oxide)-b-poly(propylene oxide)-b-poly(ethylene oxide)-b-poly(vinyl alcohol). *Langmuir*, 1999, vol. 15, 8633-8639